# EUROPEAN PATENT SPECIFICATION

(11) **EP 2 737 318 B1**
(45) Date of publication and mention of the grant of the patent: **01.03.2017**
(21) Application number: 11776842.4
(22) Date of filing: 27.07.2011
(51) Int. Cl.: G01N 33/576

(54) **A2M FRAGMENTS AND APPLICATIONS THEREOF**
A2M-FRAGMENTE UND IHRE VERWENDUNG
FRAGMENTS A2M ET LEURS APPLICATIONS

(43) Date of publication of application: 04.06.2014
(73) Proprietor: Bio-Rad Innovations, 92430 Marnes-La-Coquette (FR); Centre National de la Recherche Scientifique, 75794 Paris Cedex 16 (FR)
(72) Inventor: BATXELLI, Isabelle, Catherine, F-30670 Aigues-Vives (FR); WATELET, Bénédicte, F-34980 Saint Clement De Riviere (FR)
(74) Representative: Desaix, Anne
(86) International application number: PCT/IB2011/002066
(87) International publication number: WO 2013/014485

(56) References cited:
- WO-A2-2008/031051
- B. GANGADHARAN ET AL: "Novel Serum Biomarker Candidates for Liver Fibrosis in Hepatitis C Patients", CLINICAL CHEMISTRY, vol. 53, no. 10, 1 October 2007 (2007-10-01), pages 1792-1799, XP055018487, ISSN: 0009-9147, DOI: 10.1373/clinchem.2007.089144 cited in the application
- JAIN MOHIT RAJA ET AL: "Altered proteolytic events in experimental autoimmune encephalomyelitis discovered by iTRAQ shotgun proteomics analysis of spinal cord", PROTEOME SCIENCE, vol. 7, no. 1, 16 July 2009 (2009-07-16), page 25, XP021058840, BIOMED CENTRAL, LONDON, GB ISSN: 1477-5956, DOI: 10.1186/1477-5956-7-25
- Abcam Inc.: "Anti-alpha 2 Macroglobulin antibody (ab58703)", Product Datasheet , 4 February 2012 (2012-02-04), XP002668944, Retrieved from the Internet: URL:http://www.abcam.com/alpha-2-Macroglob ulin-antibody-ab58703.pdf [retrieved on 2012-02-07]
- HO AI-SHENG ET AL: "Novel biomarkers predict liver fibrosis in hepatitis C patients: alpha 2 macroglobulin, vitamin D binding protein and apolipoprotein AI", JOURNAL OF BIOMEDICAL SCIENCE, vol. 17, no. 1, 15 July 2010 (2010-07-15), page 58, XP021071241, KLUWER ACADEMIC PUBLISHERS, DO ISSN: 1423-0127, DOI: 10.1186/1423-0127-17-58
- PATEL K ET AL: "Evaluation of a panel of non-invasive serum markers to differentiate mild from moderate-to-advanced liver fibrosis in chronic hepatitis C patients", JOURNAL OF HEPATOLOGY, vol. 41, no. 6, 1 December 2004 (2004-12-01), pages 935-942, XP004776471, MUNKSGAARD INTERNATIONAL PUBLISHERS, COPENHAGEN, DK ISSN: 0168-8278, DOI: 10.1016/J.JHEP.2004.08.008 cited in the application
- SOTTRUP-JENSEN L ET AL: "PRIMARY STRUCTURE OF HUMAN ALPHA-2 MACRO GLOBULIN 5. THE COMPLETE STRUCTURE", JOURNAL OF BIOLOGICAL CHEMISTRY.(MICROFILMS), vol. 259, no. 13, 10 July 1984 (1984-07-10), pages 8318-8327, XP002413126, AMERICAN SOCIETY OF BIOLOGICAL CHEMISTS, BALTIMORE, MD, US

## Description

### FIELD

The application describes, and the invention relates to alpha-2-macroglobulin (A2M) polypeptides, the amino acid sequence of which is the sequence of A2M fragments, as well as to means deriving therefrom, such as ligands, which specifically bind to said A2M fragments, and peptides that can be used in the production of such fragments. The application also describes, and the invention also relates to medical and biological applications of said A2M fragments and of said means, more particularly in the field of hepatitis, still more particularly for the staging of hepatic fibrosis.

### BACKGROUND

Hepatitis C virus (HCV) is a major cause of chronic liver disease and consequently a significant cause of morbidity and mortality. Indeed, about 180 million people are infected worldwide and up to 70% of HCV-positive patients will develop chronic infection.

Chronically infected patients are at severe risk of developing hepatic fibrosis and cirrhosis.

Hepatic fibrosis is mediated by necro-inflammation and activation of liver stellate cells; it corresponds to an excess accumulation of extracellular matrix proteins including collagen.

Fibrosis progression in chronic hepatitis C determines the final prognosis and thus the need of treatment. In chronic hepatitis C, the transition from mild to moderate fibrosis seems to be a major prognostic step.

Currently, liver biopsy is considered the gold standard for the diagnosis/staging of hepatic fibrosis, but this invasive method is now being progressively replaced or combined with non-invasive methods, such as blood tests or hepatic elasticity measurement [Ahmad *et al*. 2011].

Various serum molecules have been assessed as potential markers of the fibrosis stage, and many different marker combinations include such molecules.

### For example:

- FIBROSPECT® is a combination of hyaluronic acid (HA), metalloproteinase inhibitor 1 (TIMP-1) and alpha-2-macroglobulin (A2M) [Zaman *et al.* 2007];
- FIBROTEST® is the combination of alpha-2-macroglobulin (A2M), haptoglobin (Hapto), apolipoprotein A1 (ApoA1), gamma glutamyl transpeptidase (GGT) and total bilirubin (BLT) [Imbert-Bismut *et al.* 2001; Poynard T *et al.* 2004] ;
- FIBROMETER™ is a combination of hyaluronic acid (HA), aspartate aminotransferase (AST), platelet count (PLQ), prothrombin index (TP), alpha-2-macroglobuline (A2M), urea and age [Cales *et al.* 2008 and 2010]
- HEPASCORE™ is a combination of bilirubin (BLT), gamma glutamyl transpeptidase (GGT), hyaluronic acid (HA), alpha-2-macroglobulin (A2M), gender and age [Guechot *et al.* 2010].

Many other markers and marker combinations have been described, such as:
- the PGAA index, which combines gamma glutamyl transpeptidase (GGT), apolipoprotein A1 (ApoA1), prothrombin index (TP) and alpha-2-macroglobulin (A2M) [Naveau *et al.* 1994];
- ACTITEST™, which is a modified form of the FIBROTEST® with the addition of ALT level [Imbert-Bismut *et al.* 2001];
- the marker combination, which has been described in the patent applications FR 1151022 and US 61/440,968 filed on 9 February 2011, which notably involves secreted phosphoprotein 1 (SPP1), alpha-2-macroglobulin (A2M) and/or vimentin (VIM), interleukin-8 (IL8) and/or C-X-C motif chemokine 10 (CXCL10) and/or endoglin (ENG).

The search for a marker combination with the required precision and accuracy, more particularly at the early stage of fibrosis, fosters the search for new serum markers having an improved prognostic value [Shaheen *et al.* 2007; Ahmad *et al.* 2011].

### SUMMARY

The invention relates to the subject-matter as defined in the claims as filed and as herein described.

The application provides polypeptides, the sequence of which is the sequence of fragments of alpha-2-macroglobulin (A2M). These A2M polypeptides have been isolated from the sera of hepatitis patients, and are differentially abundant depending on the stage of hepatic fibrosis. Remarkably, these A2M polypeptides are more abundant in those hepatitis patients, whose liver fibrosis scores at least F2, than in those patients, whose liver fibrosis scores at most F1 according to the Metavir scoring system.

The application describes these A2M polypeptides, as well as means deriving therefrom, such as:
- ligands, which specifically bind to said A2M polypeptides, more particularly antibodies and antibody fragments,
- peptides, which are fragments of said A2M polypeptides, and which can be used in the production of said A2M antibodies and antibody fragments,
- compounds, which comprise at least one A2M polypeptide or peptide of the application or invention,
- nucleic acids coding for said A2M polypeptides, vectors and host cells comprising at least one of said nucleic acids or at least one of said A2M polypeptides,
- primers and probes, which specifically amplify such nucleic acids.

The application also describes medical and biological applications of said A2M polypeptides and of said means, more particularly in the field of hepatitis, still more particularly for staging hepatic fibrosis, even still more particularly for determining whether a human liver is a fibrosis stage, which scores at most F1, or whether it scores at least F2 according to the Metavir scoring system.

The invention relates to:
- a polypeptide, the amino acid sequence of which is the sequence of a sub-fragment of the C-terminal thioester-cleaved fragment of human alpha-2-macroglobulin (A2M), wherein the molecular weight of said polypeptide is of 36 to 44 kDa, and wherein:
   - the first N-terminal amino acid of said polypeptide is an amino acid, which, in the full length sequence of said human A2M, is at a position 1,098 or 1,085 or 1,084 or 1,083, and
   - the last C-terminal amino acid of said polypeptide is an amino acid, which, in the full length sequence of said human A2M, is one of the last twenty C-terminal amino acids;
- a peptide, the sequence of which is one of SEQ ID NOs: 1-8, 17;
- a compound consisting of:
   - the polypeptide or peptide of the invention, and of
   - one or several other structural components, which are linked to said polypeptide or peptide, wherein said other structural components are selected from the group consisting of His tags, BSA, KLH;
- a coding nucleic acid, the sequence of which consists of a sequence coding for the polypeptide of any one of claims 1-7, or for the peptide of claim 8, or for the compound of claim 9.
- a vector, which comprises a coding nucleic acid inserted therein, wherein the expression product of said coding nucleic acid is the polypeptide or or compound of the invention;
- a genetically engineered host cell, which comprises a coding nucleic acid consisting of the nucleic acid of the invention and/or which comprises the vector of the invention;
- the *in vitro* use of an antibody or antibody fragment, or of a kit comprising said antibody or antibody fragment, for detecting hepatitis and/or liver fibrosis, wherein said antibody or antibody fragment specifically binds to the N-terminal extremity of the polypeptide or peptide of the invention, without binding to the full length human A2M protein, and wherein said antibody or antibody fragment does not bind to the C-terminal thioester-cleaved fragment of said human A2M, and does not bind to the C-terminal bait-cleaved fragment of said human A2M;
- the *in vitro* use of an antibody or antibody fragment, or of a kit comprising said antibody or antibody fragment, for scoring a liver fibrosis stage and/or for determining whether a liver fibrosis is at a stage, which scores at most F1 or at a stage, which scores at least F2, in accordance with the Metavir scoring system, wherein said antibody or antibody fragment specifically binds to the N-terminal extremity of the polypeptide or peptide of the invention, without binding to the full length human A2M protein, and wherein said antibody or antibody fragment does not bind to the C-terminal thioester-cleaved fragment of said human A2M, and does not bind to the C-terminal bait-cleaved fragment of said human A2M; and
- the *in vitro* use of a peptide of the invention for *in vitro* screening antibodies or antibody fragments.

### BRIEF DESCRIPTION OF THE FIGURES

In the figures, as well as throughout the text:
α2M is equal to A2M, and means alpha-2-macroglobulin, more particularly human alpha-2-macroglobulin;
2-DE means two dimensional gel electrophoresis;
1-DE means one dimensional gel electrophoresis.

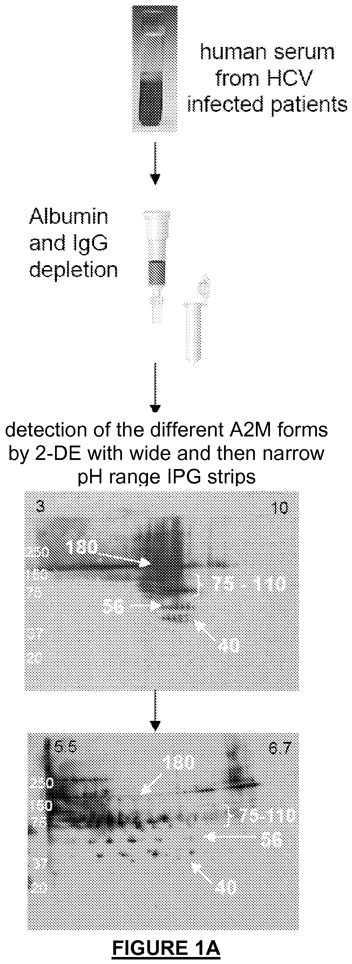

**Figure 1A****: flow chart of the experimental approach.**

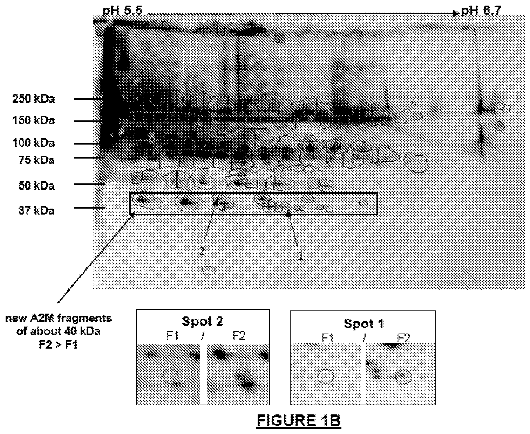

**Figure 1B****: comparative 2-DE analysis of F1 and F2 patients using a polyclonal anti-A2M antibody** (60 or 120 micrograms of total proteins from the hepatitis C patient's serum [Metavir score F1 or F2], said serum having been previously depleted from albumin and from immunoglobulins G).
   As expected, the full length monomeric A2M form (about 180 kDa), the thioester-cleaved N- and C-terminal A2M fragments (about 110 kDa and 56 kDa respectively) and the bait region-cleaved N- and C-terminal A2M fragments (about 75 kDa and about 85kDa respectively) were clearly detected.
   Furthermore, novel A2M fragments of about 40kDa were detected as several distinct spots ("40 kDa region"). These novel A2M fragments of about 40 kDa are present at a significantly higher level in the F2 patients compared to the F1 patients.

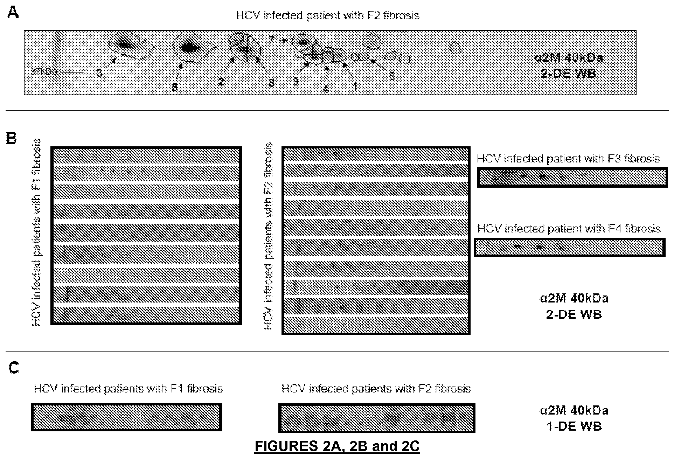

**Figure 2A, 2B and 2C****: analysis of the A2M 40kDa fragments.**
   Fig. 2A: nine 2-DE significant spots of A2M 40kDa fragments permitting to discriminate F1 and F2 stages of fibrosis.
   Fig. 2B: 2-DE spots of A2M 40kDa fragments in nine F1 patients, ten F2 patients, one F3 patient and one F4 patient (Metavir staging).
   Fig. 2C: 1-DE bands of A2M 40kDa fragments in nine F1 patients and ten F2 patients (Metavir staging).
**Figure 3****: box plots of spot intensity measurements *versus* Metavir fibrosis stage in the whole study population** (HCV-infected patients with F1 fibrosis, n=9 and HCV-infected patients with F2 fibrosis, n=10; A2M40 = A2M 40kDa fragment).
**Figures 4A, 4B** **and** **4C****: characterization of the A2M 40kDa fragments.**

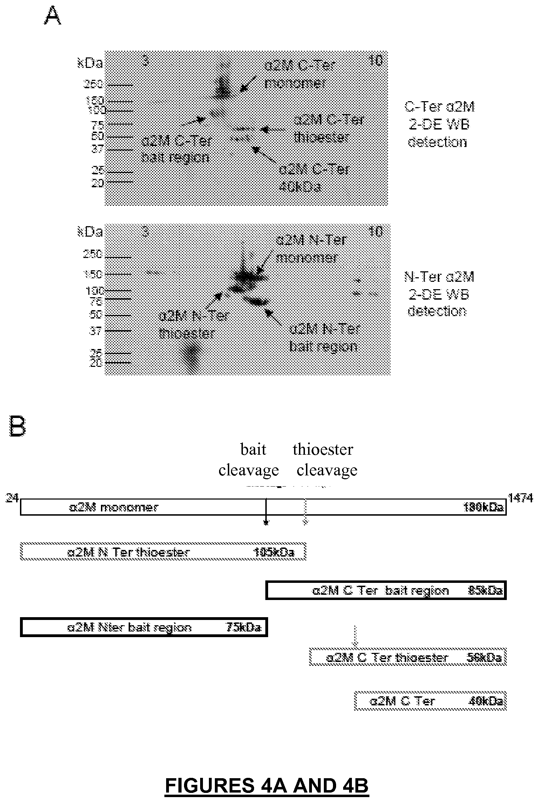

   Fig. 4A: detection of the different A2M fragments after 2-DE by western blot analysis using antibodies against the N-terminal (lower panel) and C-terminal (upper panel) regions of A2M.
   Fig. 4B: schematic representation of the A2M monomer, of the N-terminal and C-terminal bait-cleaved A2M fragments, of the N-terminal and C-terminal thioester-cleaved A2M fragments, and of the A2M C-terminal fragments of about 40kDa.

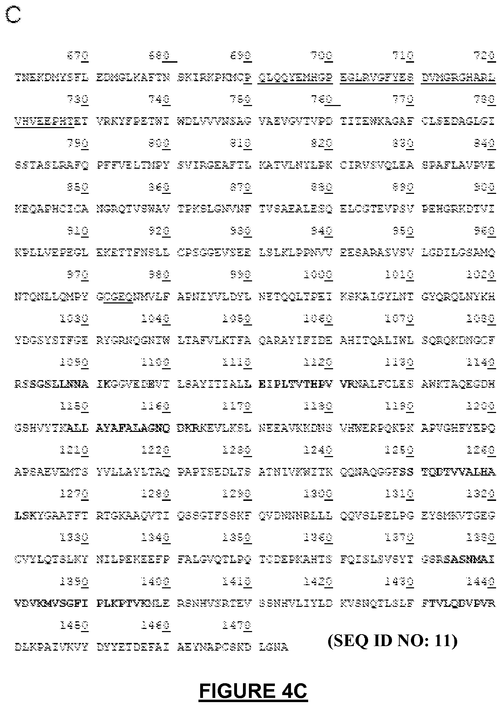

   Fig. 4C: A2M C-terminal sequence (fragment 661-1,474; SEQ ID NO: 11): underlined amino acids correspond to the sequence of the A2M bait cleavage site (690-728) and of the thioester bond cleavage site (972-975); bold amino acids correspond to the sequence of some of the most significant peptides identified by the LC-MS/MS approach (peptides 1,083-1,092; 1,110-1,122; 1,148-1,163; 1,249-1,263; 1,374-1,384; 1,385-1,397; 1,432-1,440); the amino acid starting positions of the (four) A2M 40kDa fragments are at positions 1,083 (Ser), 1,084 (Gly), 1,085 (Ser) and 1,098 (Glu), respectively.

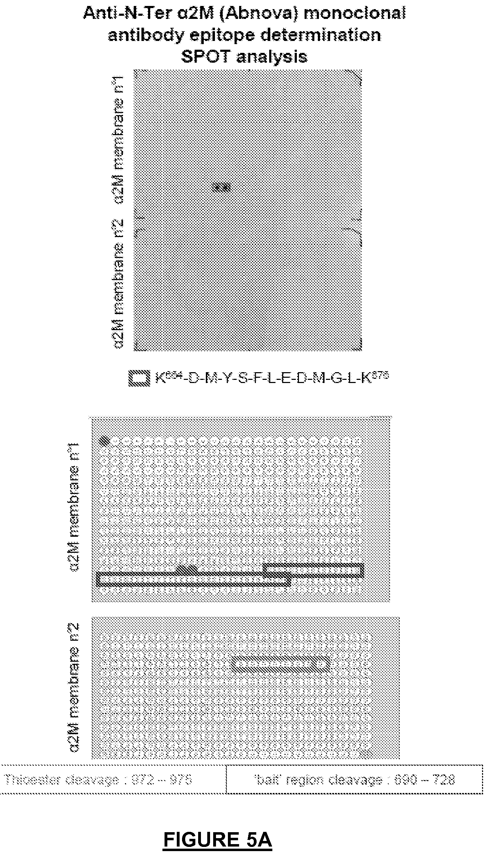

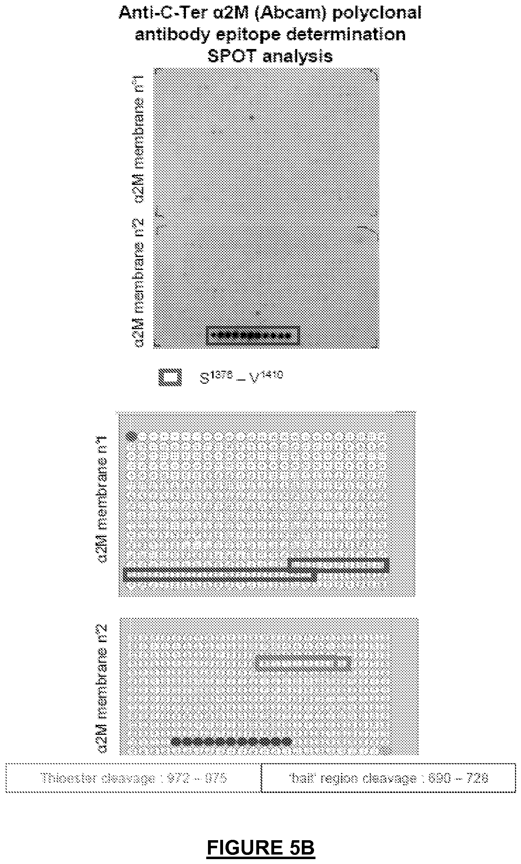

**Figures 5A** **and** **5B****: localization of the epitope of an anti-human C-terminal A2M rabbit polyclonal antibody (Abcam, ab58703) and of the epitope of an anti-human N-terminal A2M mouse monoclonal antibody (Abnova, H00000002-M03).**
   Overlapping synthetic peptides corresponding to the amino acid sequence of human A2M were prepared using the epitope mapping technique and probed with an anti-N-terminal A2M antibody (Fig. 5A) or an anti-C-terminal A2M antibody (Fig. 5B).

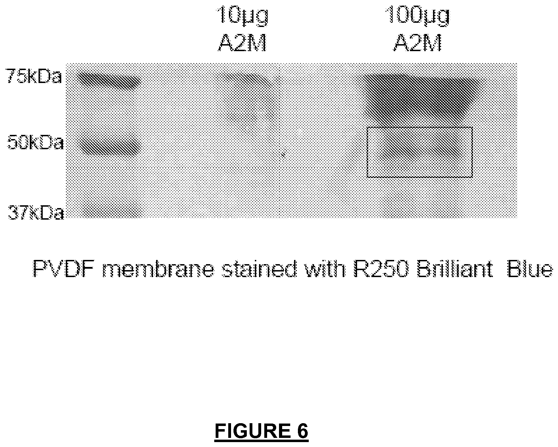

**Figure 6****: N-terminal sequencing of the 40kDa A2M fragments.**

Visualization of the three bands corresponding to the three 40kDa A2M fragments on a SDS-PAGE gel before excision for N-terminal sequencing (first lane: molecular weights; second and third lanes: 10 and 100 micrograms of A2M purified from human plasma, respectively). The huge quantity of A2M proteins analysed in this experience (100 micrograms) has influenced the protein migration in the gel and induced a localisation of the 40kDa A2M fragments upper than 40kDa. The three distinct bands were well-visualized after staining.

### DETAILED DESCRIPTION

The application provides, and the invention relates to, fragments of alpha-2-macroglobulin (A2M), which have been isolated from the sera of hepatitis patients. An A2M fragment of the application is a polypeptide, the amino acid sequence of which is the sequence of a C-terminal fragment of human alpha-2-macroglobulin (A2M), more particularly the sequence of a sub-fragment of the C-terminal thioester-cleaved fragment of human alpha-2-macroglobulin (A2M). The A2M fragment of the invention is a polypeptide, the amino acid sequence of which is the sequence of a sub-fragment of the C-terminal thioester-cleaved fragment of human alpha-2-macroglobulin (A2M), wherein the molecular weight of said polypeptide is of 36 to 44 kDa, and wherein:
- the first N-terminal amino acid of said polypeptide is an amino acid, which, in the full length sequence of said human A2M, is at a position 1,098 or 1,085 or 1,084 or 1,083, and
- the last C-terminal amino acid of said polypeptide is an amino acid, which, in the full length sequence of said human A2M, is one of the last twenty C-terminal amino acids.

The A2M fragments of the application or invention are differentially abundant depending on the stage of hepatic fibrosis. Either individually or in combination with other marker(s) and/or clinical feature(s), they enable to reliably score the fibrosis stage. More particularly, they enable to differentiate no/mild fibrosis (Metavir score of at most F1) from moderate/advanced/severe fibrosis (Metavir score of F2 or more). They can be recombinantly produced, and are highly useful tools in the diagnosis, prognosis and treatment of hepatitis.

The application further provides sub-fragments of the A2M fragments of the application or invention.

The A2M sub-fragments of the application are N-terminal sub-fragments of the A2M fragments of the application. The A2M sub-fragments of the invention are the peptides, the respective sequence of which is one of SEQ ID NOs: 1-8, 17. An A2M sub-fragment of the application or invention is notably useful as an antigen for the production of antibodies and antibody fragments, which specifically bind to an A2M fragment of the application or invention, and/or as an affinity ligand for screening for such antibodies or antibody fragments.

A polypeptide or peptide is a single linear chain of amino acids bonded together by peptide bonds. Said amino acid chain has an N-terminal extremity and a C-terminal extremity. In the application or invention, peptides consist of at most 50 amino acids, whereas polypeptides consist of more than 50 amino acids. The A2M fragments of the application or invention can be termed "polypeptides", whereas the A2M sub-fragments of the application or invention can be termed "peptides". The term "A2M polypeptides of the application or invention" (or the like) and the term "A2M fragment of the application or invention" (or the like) can be used interchangeably. Similarly, the term "A2M peptide of the application or invention" (or the like) and the term "A2M sub-fragment of the application or invention" (or the like) can be used interchangeably.

In the application and invention, unless specified otherwise and/or unless a context dictates otherwise, all the terms have their ordinary meaning in the relevant field(s).

The expression "fragment of human A2M" and the like is intended in accordance with its ordinary meaning in the field, and implies that the amino acid sequence of said fragment is identical to a continuous portion of the full length sequence of said human A2M from one amino acid to another, wherein at least one of said two amino acids is internal to said A2M full length sequence, and that said fragment is under an isolated form. The same applies to a sub-fragment of a fragment *mutatis mutandis.*

The application further provides and the invention relates to compounds, nucleic acids, vectors, host cells, antibodies, antibody fragments, which derive from the A2M polypeptides of the application or invention or from the A2M peptides of the application or invention.

The application also describes and the invention relates to applications of the means of the application or invention, notably in the field of hepatitis, still more particularly for the staging of hepatic fibrosis.

### A2M polypeptides of the application or invention:

The A2M polypeptides of the application or invention are A2M fragments, which have been identified and isolated by proteomic analysis of serum samples collected from HCV infected human patients. It is believed that, although human A2M is a well known protein, the A2M fragments of the application or invention have never been described before.

Human A2M is a serum glycoprotein, which has sequence similarity with other members of the A2M family including the complement components C3, C4 and C5. Human A2M is synthesized by the liver (primarily by hepatocytes) as a polypeptide of usually 1,474 amino acids with a signal peptide (usually 23 residues). The mature A2M protein is a tetramer (about 720 kDa) of four identical 180 kDa subunits, which form two disulfide bond-linked dimers. A2M is a general and irreversible protease inhibitor implicated in many processes and can inhibit all four classes of proteases by a unique trapping mechanism.

A human A2M sequence is available e.g., under accession number NM_000014, and is reproduced below:

In the application or invention, residue or amino acid positions are computed with respect to a full length human A2M sequence of 1,474 amino acids, more particularly with respect to sequence NM_000014 (SEQ ID NO: 9).

After cleavage of the 1-23 signal peptide, the A2M sequence of SEQ ID NO: 9 matures into:

Figure 4C shows the 661-1,474 C-terminal fragment **(SEQ ID NO: 11)** of said A2M sequence of SEQ ID NO: 9.

Naturally-occurring human A2M, the amino acid sequence of which differs from said sequence of SEQ ID NO: 9, have been described in the prior art. Examples of such naturally-occurring A2M variants include the proteins, the amino acid sequence of which differs from said sequence of SEQ ID NO: 9 by one or several of the following amino acid substitutions:
- N639D (Asp at position 639 instead of Asn);
- R704H (His at position 704 instead of Arg);
- L815Q (Gln at position 815 instead of Leu);
- C972Y (Tyr at position 972 instead of Cys);
- l1000V (Val at position 1,000 instead of Ile).

The bait region of human A2M (residues 690-728 within SEQ ID NO: 9) contains specific cleavage sites for different proteases. Cleavage of the bait region by a protease induces a conformation change that enables A2M to trap and form a covalent bond with the protease. Specifically, following cleavage of the bait region, a thioester bond (residues 972-975 within SEQ ID NO: 9) is hydrolyzed to mediate the covalent binding of A2M to the protease. The trapped protease remains active against small peptide substrates, but loses its ability to interact with large protein substrates or inhibitors. A2M family members are major liver products associated with inflammatory response.

An A2M fragment of the application or invention is a polypeptide, the amino acid sequence of which is the sequence of a C-terminal fragment of human alpha-2-macroglobulin (A2M), more particularly the sequence of a sub-fragment of the C-terminal bait-cleaved fragment of human A2M, still more particularly the sequence of a sub-fragment of the C-terminal thioester-cleaved fragment of human A2M. Please see Figure 4B.

The site of said bait cleavage of said human A2M is at positions 690-728 within the sequence of the full length human A2M (*e.g*., within SEQ ID NO: 9 or a variant sequence thereof) and/or said C-terminal bait-cleaved fragment of said human A2M is of about 85kDa.

The site of said thioester cleavage of said human A2M is at positions 972-975 within the sequence of the full length human A2M (*e.g*., within SEQ ID NO: 9 or a variant sequence thereof) and/or said C-terminal thioester-cleaved fragment of said human A2M is of about 56kDa.

An A2M fragment of the application can notably be defined:
- by feature(s) relating to the molecular weight of said A2M fragment, and/or
- by feature(s) relating to the start and/or end position(s) of said fragment within said full length A2M protein, and/or
- by feature(s) relating to the amino acid length of said fragment, and/or
- by feature(s) relating to the location of said fragment within a full length A2M sequence, and/or
- by feature(s) relating to the isoelectric point (pl) of said fragment.

The application explicitly includes each and every combination of such features.

Hence, according to an aspect of the application, said A2M fragment is of about 40 kDa (*cf. e.g.,* the examples below and the Figures). More precisely, according to said aspect of the application, said A2M fragment is of about 36 to about 44 kDa, more particularly of about 36 to 43.5 kDa, still more particularly of 36.5 to 43.5 kDa, still more particularly of about 38 to 43.5 kDa, still more particularly of 38.5 to 43.5 kDa, still more particularly of about 39 to 43.5 kDa, still more particularly of 39 to 43.5 kDa, still more particularly of about 40 to 43.5 kDa, still more particularly of 40.5 to 43.5 kDa, still more particularly of about 41 to 43.5 kDa, still more particularly of 41.5 to 43.5 kDa. A molecular weight (MW) can be determined by any conventional MW determination means that the person of ordinary skill in the art may find appropriate or convenient, e.g.,:
- by addition of the molecular weights of the amino acids of said A2M fragment, *e.g*., following the method described in Gasteiger *et al.,* 2005 and/or using a computer assisted tool, such as the ProtParam software (described in Gasteiger *et al.,* 2005, and available from http://www.expasy.org/tools/protparam.html), and/or
- by mass spectrometry, e.g., as described in the examples below, and/or
- by one-dimensional electrophoresis or two-dimensional electrophoresis.

Said one-dimensional or two-dimensional electrophoresis may *e.g.,* comprise a gel electrophoresis, more particularly a gel electrophoresis under denaturing conditions (*e.g.*, under SDS denaturing conditions), *e.g*., under denaturing and reducing conditions, *e.g*., under the denaturing and reducing conditions described in the examples below (*cf*. below, paragraph *"One dimensional gel electrophoresis (1-DE)"* and/or paragraph *"Two dimensional gel electrophoresis (2-DE)").*

For said two-dimensional gel electrophoresis, pH conditions of 3 to 10, more particularly of 5.5 to 6.7 can *e.g*., be used (*cf.* below, paragraph"*Two dimensional gel electrophoresis (2-DE)*" and/or Figure 1A and/or Figure 1 B).

Said two-dimensional electrophoresis may *e.g.*, be as shown in, or illustrated by, Figure 1A and/or in Figure 1B.

The detection of the protein bands of said one-dimensional or two-dimensional gel electrophoresis can be performed by any means, which the person of ordinary skill in the art may find appropriate *e.g.*,:
- by COOMASSIE® staining (*e.g*., COOMASSIE® Brilliant Blue R250) *e.g.*, as described in the examples below *(cf.* below, paragraph *"One dimensional gel electrophoresis (1-DE)"),* or
- by western blotting *e.g.*, as described in the examples below *(cf.* below, paragraph"*Two dimensional gel electrophoresis (2-DE)*")*.*

The protein(s) submitted to said one-dimensional or two-dimensional gel electrophoresis may *e.g*., be:
- purified human A2M (*e.g*., purified A2M that is commercially available from Sigma), or
- at least one sample of serum of a hepatitis C patient [*e.g*., an HCV-infected patient scoring F1 or F2 according to the Metavir scoring scale], wherein said serum preferably has previously been depleted from albumin and immunoglobulins G (*e.g*., using the AURUM® kit, Bio-Rad, Hercules, U.S.A., *cf*. the examples below).

The amount of proteins submitted to one-dimensional gel electrophoresis may *e.g*., be:
- when detection is performed by western blotting:
   ∘ 0.5 to 5 micrograms of purified human A2M, or
   ∘ 5 to 50 micrograms of the proteins of said albumin- and IgG-depleted serum;
- when detection is performed by COOMASSIE® staining, 10 to 100 micrograms of purified human A2M.

The quantity of proteins submitted to two-dimensional gel electrophoresis may *e.g.*, be:
- when detection is performed by western blotting:
   ∘ 1 to 10 micrograms of purified human A2M, or
   ∘ 50 to 150 micrograms of the proteins of said albumin- and IgG-depleted serum;
- when detection is performed by COOMASSIE® staining, 100 to 200 micrograms of purified human A2M.

According to an alternative and/or complementary aspect of the application:
- the first N-terminal amino acid of said A2M fragment is an amino acid, which, in the full length sequence of said A2M, is at a position selected from positions 1,081-1,123, and/or, preferably and,
- the last C-terminal amino acid of said A2M fragment is an amino acid, which, in the full length sequence of said A2M, is one of the last twenty C-terminal amino acids.

The A2M fragment of the invention is a polypeptide, the amino acid sequence of which is the sequence of a sub-fragment of the C-terminal thioester-cleaved fragment of human alpha-2-macroglobulin (A2M), wherein the molecular weight of said polypeptide is of 36 to 44 kDa, and wherein:
- the first N-terminal amino acid of said polypeptide is an amino acid, which, in the full length sequence of said human A2M, is at a position 1,098 or 1,085 or 1,084 or 1,083, and
- the last C-terminal amino acid of said polypeptide is an amino acid, which, in the full length sequence of said human A2M, is one of the last twenty C-terminal amino acids.

The expression "a position selected from positions X-Y" and the like explicitly encompasses position X and position Y as well as each and every position between X and Y. For example, "a position selected from positions 1,081-1,123" means position 1,081 or 1,082 or 1,083 or 1,084 or 1,085 or 1,086 or 1,087 or 1,088 or 1,089 or 1,090 or 1,091 or 1,092 or 1,093 or 1,094 or 1,095 or 1,096 or 1,097 or 1,098 or 1,099 or 1,100 or 1,101 or 1,102 or 1,103 or 1,104 or 1,105 or 1,106 or 1,107 or 1,108 or 1,109 or 1,110 or 1,111 or 1,112 or 1,113 or 1,114 or 1,115 or 1,116 or 1,117 or 1,118 or 1,119 or 1,120 or 1,121 or 1,122 or 1,123.

The expression "last twenty C-terminal amino acids" and the like means the stretch of twenty contiguous amino acids that are at the very C-terminal extremity of said A2M full length sequence. For example, if the full length sequence of said A2M consists of 1,474 amino acids, the last C-terminal amino acid of said fragment is an amino acid, which is at a position selected from positions 1,455-1,474 within said full length A2M sequence.

A human full length A2M sequence is considered to be consisting of 1,474 amino acids.

Examples of such human full length A2M sequences comprise the above-described sequence of SEQ ID NO: 9 (NM_000014) and the variant sequences thereof, which still are human A2M sequences. Such variants include the naturally-occurring human A2M variants, which are already known at the filing date of the application, as well as any human A2M variant that may be described after said filing date. Such variant sequences usually differ from said sequence of SEQ ID NO: 9 by one or more amino acid deletions (*e.g*., amino acid deletion(s) from the C-terminal extremity and/or N-terminal extremity of SEQ ID NO: 9) and/or by one or more amino acid substitutions. More particularly, such variant sequences differ from said sequence of SEQ ID NO: 9 only by amino acid substitution(s). The total number of amino acid substitution(s) and deletion(s) does usually not exceed ten, more particularly eight, still more particularly five. Hence, said one or more amino acid substitutions usually are at most ten, more particularly at most eight, still more particularly at most five. Said amino acid substitution(s) usually comprise one or several of the above-mentioned (naturally-occurring) N639D, R704H, L815Q, C972Y, I1000V substitutions.

When said full length A2M sequence is the sequence of SEQ ID NO: 9, said first N-terminal amino acid of said A2M fragment is one of the following amino acids:

**Table 4:**

| **Position within the full length A2M sequence of SEQ ID NO: 9** | **Amino acid** |
|---|---|
| 1,081 | R (Arg) |
| 1,082 | S (Ser) |
| **1,083** | **S (Ser)** |
| **1,084** | **G (Gly)** |
| **1,085** | **S (Ser)** |
| 1,086 | L (Leu) |
| 1,087 | L (Leu) |
| 1,088 | N (Asn) |
| 1,089 | N (Asn) |
| 1,090 | A (Ala) |
| 1,091 | I (Ile) |
| 1,092 | K (Lys) |
| 1,093 | G (Gly) |
| 1,094 | G (Gly) |
| 1,095 | V (Val) |
| 1,096 | E (Glu) |
| 1,097 | D (Asp) |
| **1,098** | **E (Glu)** |
| 1,099 | V (Val) |
| 1,100 | T (Thr) |
| 1,101 | L (Leu) |
| 1,102 | S (Ser) |
| 1,103 | A (Ala) |
| 1,104 | Y (Tyr) |
| 1,105 | I (Ile) |
| 1,106 | T (Thr) |
| 1,107 | I (Ile) |
| 1,108 | A (Ala) |
| 1,109 | L (Leu) |
| 1,110 | L (Leu) |
| 1,111 | E (Glu) |
| 1,112 | I (Ile) |
| 1,113 | P (Pro) |
| 1,114 | L (Leu) |
| 1,115 | T (Thr) |
| 1,116 | V (Val) |
| 1,117 | T (Thr) |
| 1,118 | H (His) |
| 1,119 | P (Pro) |
| 1,120 | V (Val) |
| 1,121 | V (Val) |
| 1,122 | R (Arg) |
| **1,123** | N (Asn) |

When said full length A2M sequence is the sequence of SEQ ID NO: 9, said last C-terminal amino acid of said A2M fragment is one of the following amino acids:

**Table 5:**

| **Position within the full length A2M sequence of SEQ ID NO: 9** | **Amino acid** |
|---|---|
| 1,455 | T (Thr) |
| 1,456 | D (Asp) |
| 1,457 | E (Glu) |
| 1,458 | F (Phe) |
| 1,459 | A (Ala) |
| 1,460 | I (Ile) |
| 1,461 | A (Ala) |
| 1,462 | E (Glu) |
| 1,463 | Y (Tyr) |
| 1,464 | N (Asn) |
| 1,465 | A (Ala) |
| 1,466 | P (Pro) |
| 1,467 | C(Cys) |
| 1,468 | S (Ser) |
| 1,469 | K (Lys) |
| 1,470 | D (Asp) |
| 1,471 | L (Leu) |
| 1,472 | G (Gly) |
| 1,473 | N (Asn) |
| **1,474** | **A (Ala)** |

More particularly:
- the first N-terminal amino acid of said A2M fragment can be an amino acid, which, in the full length sequence of said A2M, is:
   o at a position selected from positions 1,081-1,123,
   o still more particularly from positions 1,082-1,123,
   o even still more particularly from positions 1,083-1,123,
   o even still more particularly from positions 1,084-1,123,
   o even still more particularly from positions 1,085-1,123,
   o even still more particularly from positions 1,098-1,123,
   o even still more particularly from positions 1,081-1,098,
   o even still more particularly from positions 1,082-1,098,
   o even still more particularly from positions 1,083-1,098,
   o even still more particularly from positions 1,084-1,098,
   o even still more particularly from positions 1,085-1,098,
   and/or, preferably and,
- the last C-terminal amino acid of said A2M fragment is an amino acid, which, in the full length sequence of said A2M, is:
   ∘ one of the last twenty C-terminal amino acids (*i.e.,* if the full length A2M sequence consists of 1,474 amino acids: one of amino acids 1,455-1,474),
   o more particularly one of the last fifteen C-terminal amino acids (*i.e.,* if the full length A2M sequence consists of 1,474 amino acids: one of amino acids 1,460-1,474),
   o still more particularly one of the last ten C-terminal amino acids (*i.e.,* if the full length A2M sequence consists of 1,474 amino acids: one of amino acids 1,465-1,474),
   o still more particularly one of the last eight amino acids (*i.e.,* if the full length A2M sequence consists of 1,474 amino acids: one of amino acids 1,467-1,474),
   o still more particularly one of the last five amino acids (*i.e.,* if the full length A2M sequence consists of 1,474 amino acids: one of amino acids 1,470-1,474),
   o still more particularly one of the last four amino acids (*i.e.,* if the full length A2M sequence consists of 1,474 amino acids: one of amino acids 1,471-1,474),
   o even still more particularly the last C-terminal amino acid of said full length A2M sequence (*i.e.,* the amino acid at position 1,474 if the full length A2M sequence consists of 1,474 amino acids).

For example, the first N-terminal amino acid of said A2M fragment is an amino acid, which, in the full length sequence of said A2M, is at position 1,098 or 1,085 or 1,084 or 1,083 or 1,123. Advantageously, the first N-terminal amino acid of said A2M fragment is an amino acid, which, in the full length sequence of said A2M, is at position 1,098 or 1,085 or 1,084 or 1,083. Very advantageously, the first N-terminal amino acid of said A2M fragment is an amino acid, which, in the full length sequence of said A2M, is at position 1,098 or 1,085.

Advantageously, the last C-terminal amino acid of said A2M fragment is the last C-terminal amino acid of said full length A2M sequence.

Please see Tables 4 and 5 above for the identity of the amino acids at the corresponding positions within the full length A2M sequence of SEQ ID NO: 9.

The application explicitly discloses each and every combination of:
- a position feature of said first N-terminal amino acid, and of
- a position feature of said last C-terminal amino acid
that can be made with the above-mentioned features.

According to an alternative and/or complementary aspect, the amino acid sequence of said A2M fragment consists of 330 to 400 amino acids, more particularly of 350 to 400 amino acids, still more particularly of 334 to 392 amino acids, still more particularly of 353 to 392 amino acids, still more particularly of 358 to 392 amino acids, still more particularly of 353 to 391 amino acids, still more particularly of 353 to 390 amino acids, more particularly of 353 to 377 amino acids, even still more particularly of 377, 390, 391, 392 or 353 amino acids.

According to an alternative and/or complementary aspect, said A2M fragment is a C-terminal fragment of said human A2M, which does not comprise the thioester cleavage site of said A2M (in said A2M sequence of SEQ ID NO: 9, the thioester cleavage site consists of the amino acids 972-975; cf. Figures 4B and 4C). In other words, when considering the full length A2M sequence in a N-term to C-term orientation, the sequence of said A2M fragment is located in a region of said A2M sequence that is after the thioester cleavage site, and that does not include said thioester cleavage site.

According to an alternative and/or complementary aspect, said A2M fragment has an isoelectric point (pl) of 5.90 to 7.50, more particularly of 6.00 to 7.00, still more particularly of 6.02 to 6.18.

Illustrative and advantageous A2M fragments of the application or invention comprise the polypeptides, the amino acid sequence of which is:
- the sequence extending from position 1,098 to 1,474 of a full length human A2M sequence, *e.g.*, the sequence extending from position 1,098 to position 1,474 of sequence NM_000014 (SEQ ID NO: 9), *i.e.,* **SEQ ID NO: 12;**
- the sequence extending from position 1,085 to 1,474 of a full length human A2M sequence, *e.g*., the sequence extending from position 1,085 to position 1,474 of sequence NM_000014 (SEQ ID NO: 9), *i.e.,* **SEQ ID NO: 13;**
- the sequence extending from position 1,084 to 1,474 of a full length human A2M sequence, *e.g*., the sequence extending from position 1,084 to position 1,474 of sequence NM_000014 (SEQ ID NO: 9), *i.e.,* **SEQ ID NO: 14;**
- the sequence extending from position 1,083 to 1,474 of a full length human A2M sequence, *e.g*., the sequence extending from position 1,083 to position 1,474 of sequence NM_000014 (SEQ ID NO: 9), *i.e.,* **SEQ ID NO: 15.**

The A2M polypeptides of the application or invention can be recombinantly produced, or they can be purified and/or isolated from a native source, *e.g*., from the blood, plasma or serum of a hepatitis patient, more particularly a patient whose fibrosis score is of at least F2, still more particularly a patient whose fibrosis score is F2. The A2M polypeptides of the application or invention are highly useful tools in the diagnosis, prognosis and treatment of hepatitis.

### A2M peptides of the application or invention:

The application provides peptides, which are sub-fragments of the A2M polypeptides of the application or invention. The invention relates to peptides, the respective sequence of which is one of SEQ ID Nos: 1-8 and 17.

These peptides are termed the A2M peptides (or A2M sub-fragments) of the application or invention.

The A2M peptides of the application or invention are N-terminal sub-fragments of the A2M polypeptides of the application or invention. Said N-terminal sub-fragment starts at the first N-terminal amino acid of said A2M polypeptide of the application or invention, and extends (in N-term to C-term direction) for at most the next 49 contiguous amino acids.

In other words, an A2M peptide of the application or invention is a peptide, the sequence of which is the sequence of a fragment from an A2M polypeptide of the application or invention:
- wherein the first N-terminal amino acid of said fragment is the first N-terminal amino acid of said A2M polypeptide, and
- wherein said fragment consists of at most 50 amino acids.

Illustrative A2M peptides of the application comprise peptides, the sequences of which are the sequences of:
- (N-terminal) fragments of the A2M polypeptide of SEQ ID NO: 12 (the sequence of the A2M polypeptide of SEQ ID NO: 12 is the sequence of positions 1,098-1,474 of the full length human A2M of SEQ ID NO: 9);
- (N-terminal) fragments of the A2M polypeptide of SEQ ID NO: 13 (the sequence of the A2M polypeptide of SEQ ID NO: 13 is the sequence of positions 1,085-1,474 of the full length human A2M of SEQ ID NO: 9);
- (N-terminal) fragments of the A2M polypeptide of SEQ ID NO: 14 (the sequence of the A2M polypeptide of SEQ ID NO: 14 is the sequence of positions 1,084-1,474 of the full length human A2M of SEQ ID NO: 9);
- (N-terminal) fragments of the A2M polypeptide of SEQ ID NO: 15 (the sequence of the A2M polypeptide of SEQ ID NO: 15 is the sequence of positions 1,083-1,474 of the full length human A2M of SEQ ID NO: 9);
- wherein the first N-terminal amino acid of said fragments is the first N-terminal amino acid of said A2M polypeptide, and
- wherein said fragment consists of at most 50 amino acids.

In other words, illustrative A2M peptides of the application comprise peptides, the sequences of which are the sequences of:
- fragments of the full length human A2M of SEQ ID NO: 9, which start at position 1,098 of said full length human A2M sequence and extend (in N-term to C-direction) for at most the next 49 contiguous amino acids;
- fragments of the full length human A2M of SEQ ID NO: 9, which start at position 1,085 of said full length human A2M sequence and extend (in N-term to C-direction) for at most the next 49 contiguous amino acids;
- fragments of the full length human A2M of SEQ ID NO: 9, which start at position 1,084 of said full length human A2M sequence and extend (in N-term to C-direction) for at most the next 49 contiguous amino acids;
- fragments of the full length human A2M of SEQ ID NO: 9, which start at position 1,083 of said full length human A2M sequence and extend (in N-term to C-direction) for at most the next 49 contiguous amino acids.

According to an embodiment of the application, said fragment of at most 50 amino acids consists of at most 45 amino acids or at most 40 amino acids, or at most 35 amino acids, or at most 30 amino acids, or at most 25 amino acids, or of at most 20 amino acids, or at most 19 amino acids, or at most 18 amino acids, or at most 17 amino acids, or at most 16 amino acids, or at most 15 amino acids, or at most 14 amino acids, or at most 13 amino acids, or at most 12 amino acids, or at most 11 amino acids, or at most 10 amino acids, or at most 9 amino acids, or at most 8 amino acids, or at most 7 amino acids, or at most 6 amino acids, or at most 5 amino acids.

More particularly, said fragment of at most 50 amino acids may consist of at most 20 amino acids, or at most 19 amino acids, or at most 18 amino acids, or at most 17 amino acids, or at most 16 amino acids, or at most 15 amino acids, or at most 14 amino acids, or at most 13 amino acids, or at most 12 amino acids, or at most 11 amino acids, or at most 10 amino acids, or at most 9 amino acids, or at most 8 amino acids, or at most 7 amino acids.

Still more particularly, said fragment of at most 50 amino acids may consist of at most 16 amino acids, or at most 15 amino acids, or at most 14 amino acids, or at most 13 amino acids, or at most 12 amino acids, or at most 11 amino acids, or at most 10 amino acids, or at most 9 amino acids, or at most 8 amino acids, or at most 7 amino acids.

Illustrative A2M peptides of the application notably comprise the peptides of the invention, *i.e.,*:
- the peptide of **SEQ ID NO:** 1 (EVTLSAYITI), which is the N-terminal 10aa fragment from the A2M polypeptide of SEQ ID NO: 12 *(i.e.,* positions 1,098-1,107 within the full length human A2M sequence of SEQ ID NO: 9);
- the peptide of **SEQ ID NO: 2** (SLLNNAIK), which is the N-terminal 8aa fragment from the A2M polypeptide of SEQ ID NO: 13 *(i.e.,* positions 1,085-1,092 within the full length human A2M sequence of SEQ ID NO: 9);
- the peptide of **SEQ ID NO: 3** (SLLNNAIKGGVEDE), which is the N-terminal 14aa fragment from the A2M polypeptide of SEQ ID NO: 13 *(i.e.,* positions 1,085-1,098 within the full length human A2M sequence of SEQ ID NO: 9);
- the peptide of **SEQ ID NO: 4** (SLLNNAIKGG), which is the N-terminal 10aa fragment from the A2M polypeptide of SEQ ID NO: 13 *(i.e.,* positions 1,085-1,094 within the full length human A2M sequence of SEQ ID NO: 9);
- the peptide of **SEQ ID NO: 5** (GSLLNNAIK), which is the N-terminal 9aa fragment from the A2M polypeptide of SEQ ID NO: 13 (*i.e.,* positions 1,084-1,092 within the full length human A2M sequence of SEQ ID NO: 9);
- the peptide of **SEQ ID NO: 6** (GSLLNNAIKGGVEDE), which is the N-terminal 15aa fragment from the A2M polypeptide of SEQ ID NO: 14 (*i.e.,* positions 1,084-1,098 within the full length human A2M sequence of SEQ ID NO: 9);
- the peptide of **SEQ ID NO: 7** (SGSLLNNAIK), which is the N-terminal 10aa fragment from the A2M polypeptide of SEQ ID NO: 15 (*i.e.,* positions 1,083-1,092 within the full length human A2M sequence of SEQ ID NO: 9);
- the peptide of **SEQ ID NO: 8** (SGSLLNNAIKGGVEDE), which is the N-terminal 16aa fragment from the A2M polypeptide of SEQ ID NO: 15 (*i.e.,* positions 1,083-1,098 within the full length human A2M sequence of SEQ ID NO: 9);
- the peptide of **SEQ ID NO: 17** (EVTLSAY), which is the N-terminal 7aa fragment from the A2M polypeptide of SEQ ID NO: 12 *(i.e.,* positions 1,098-1,104 within the full length human A2M sequence of SEQ ID NO: 9).

The A2M peptides of the application or invention can be produced by peptide synthesis (*e.g.*, by solid-phase synthesis) or by recombinant expression. They are notably useful as antigens for the production of antibodies and antibody fragments, which specifically bind to an A2M polypeptide of the application or invention. For such an application, an A2M peptide of the application or invention may advantageously be linked to (*e.g.*, by covalent linkage) or conjugated to, or bound to at least one immunogenicity carrier, *i.e.,* to at least one immunogenic molecule, which contains antigenic determinants recognized by T cells.

Immunogenicity carriers may render immunogenic a polypeptide or peptide that is linked or conjugated to it, or may enhance the immunogencity of said polypeptide or peptide.

Illustrative immunogenicity carriers comprise lipids, proteins, polypeptides and peptides, such as Keyhole Limpet Hemocyanin (KLH), Horseshoe Crab Hemocyanin (HCH), and Bovine Serum Albumin (BSA). Preferably, said immunogenicity carrier(s) is(are) linked to the C-terminal extremity of said A2M peptide of the application or invention, whereby the N-terminal amino group of said peptide remains free.

The application also discloses any composition, which comprises:
- at least one A2M peptide of the application or invention (optionally linked to (e.g., by covalent linkage) or conjugated to or bound to at least one immunogenicity carrier, more particularly to at least one protein, polypeptide or peptide, which is an immunogenicity carrier, such as KLH or BSA, more particularly KLH); and
- at least one immunologic adjuvant, such as Freund's complete adjuvant, Freund's incomplete adjuvant, TITERMAX® gold adjuvant, alum, bacterial lipopolysaccharide (LPS).

The A2M peptides of the application or invention are also useful as affinity ligands for screening for such antibodies or antibody fragments. For such an application, an A2M peptide of the application or invention may advantageously be linked to a protein, polypeptide or peptide, which can bind solid support (such as a glass support or a plastic support e.g., a polystyrene solid support), for example to Bovine Serum Albumin (BSA).

### A2M compounds of the application or invention (A2M polypeptides of the application or invention, or A2M peptides of the application or invention, linked to other structural components):

The application also discloses any compound, which consists of:
- at least one A2M polypeptide of the application or invention, as above-described, or at least one A2M peptide of the application or invention, as above-described,
linked to (*e.g*., by covalent linkage), or otherwise conjugated, bound or coupled to
- one or several other structural components *(i.e.,* one or several structural components other than A2M fragments of the application or invention),
provided that said A2M compound:
- is not human A2M, and
- is not a human A2M fragment, which would comprise an A2M polypeptide of the application or invention and be a bigger A2M fragment than said A2M polypeptide of the application or invention.

Such a compound is herein termed an A2M compound of the application.

Said other structural components are molecules, atoms, ions or complexes. A structural component can *e.g*., be a polypeptide, but in such a case, this polypeptide is not an A2M fragment of the application or invention. For example, an A2M compound of the application can comprise:
- at least one A2M fragment of the application or invention (as above-described), and
- another A2M fragment, which is not an A2M fragment of the application or invention but which, in the full length A2M sequence, is not a fragment that is immediately contiguous to said A2M fragment of the application or invention.

Nevertheless, a particular embodiment of the application is that none of said other structural components are fragments of human A2M.

Such other structural components are for example structural components that are useful:
- for polypeptide detection,
- for the recombinant production of a polypeptide,
- for the use of a polypeptide as a screening tool, more particularly as an antibody screening tool,
- for the use of a polypeptide as an antigen,
more particularly at least one structural component that is useful for the recombinant production of a polypeptide, *e.g.*, for purification and/or isolation of a recombinantly produced polypeptide.

For example, said one or several other structural components can be:
- at least one label that can be useful for polypeptide detection (*e.g*., biotin, a radioactive element, a fluorophore), and/or
- at least one linker (such as an antibody and antibody fragment) for binding or linking an A2M polypeptide or peptide of the application or invention onto a solid support, such a glass support or a plastic support (*e.g*., a plaque, a microplaque or a chip), and/or
- at least one tag for purification or isolation of recombinantly produced polypeptide or peptide (*e.g*., at least one peptide tag such as at least one His tag), and/or
- at least one protein, polypeptide or peptide, which is useful for ELISA_{S}, immunoblot or immunocytochemistry, more particularly at least one protein, polypeptide or peptide, which can bind a solid support such as a glass support or a plastic support (*e.g*., a polystyrene solid support), for example Bovine Serum Albumin (BSA), and/or
- at least one immunogenicity carrier, *i.e.,* to at least one immunogenic molecule (*e.g.*, a lipid, protein, polypeptide or peptide), which contains antigenic determinants recognized by T cells, and which may thereby render a polypeptide or peptide immunogenic, or enhance its immunogenicity, such as Keyhole Limpet Hemocyanin (KLH), Horseshoe Crab Hemocyanin (HCH), serum albumin, Bovine Serum Albumin (BSA), bovine thyroglobulin, soybean trypsin inhibitor [*e.g*., by using a bifunctional or derivatizing agent, for example, maleimidobenzoyl sulfosuccinimide ester (conjugation through cysteine residues), N-hydroxysuccinimide (through lysine residues), glutaraldehyde, succinic anhydrid or SOCl₂].

Hence, said other structural components can *e.g*., be selected from the group consisting of:
- peptide tags for purification or isolation of recombinantly produced polypeptides (such as at least one His tag),
- antibodies, antibody fragments,
- biotin,
- fluorophores,
- radioactive elements,
- at least one protein, polypeptide or peptide, which can bind a solid support such as a glass support or a plastic support (*e.g*., a polystyrene solid support), such as BSA,
- proteins, polypeptides or peptides, which are immunogenicity carriers, such as KLH, HCH, BSA;
more particularly
- peptide tags for purification or isolation of recombinantly produced polypeptides (such as at least one His tag),
- radioactive elements,
- at least one protein, polypeptide or peptide, which can bind a solid support such as a glass support or a plastic support (*e.g*., a polystyrene solid support), such as BSA,
- proteins, polypeptides or peptides, which are immunogenicity carriers, such as KLH, HCH, serum albumin, BSA;
still more particularly peptide tags for purification or isolation of recombinantly produced polypeptide (such as at least one His tag), serum albumin, BSA and KLH.

According to an embodiment of the application, said A2M compound of the application consists of:
- at least one A2M polypeptide of the application or invention, as above-described, linked to (*e.g.*, by covalent linkage), or otherwise bound or coupled to
- one or several other structural components, which comprise or consist of peptide tags for purification or isolation of recombinantly produced polypeptide (such as His tags),
provided that said A2M compound:
- is not human A2M, and
- is not a human A2M fragment, which would comprise an A2M polypeptide of the application or invention and be a bigger A2M fragment than said A2M polypeptide of the application or invention.

According to an embodiment of the application, said A2M compound of the application consists of:
- at least one A2M peptide of the application or invention, as above-described, linked to (*e.g*., by covalent linkage), or otherwise conjugated, bound or coupled to
- one or several other structural components, which comprise or consist of:
   o proteins, polypeptides or peptides, which can bind a solid support such as a glass support or a plastic support (*e.g*., a polystyrene solid support), such as BSA,
   o proteins, polypeptides, peptides, which are immunogenicity carriers, such as KLH, HCH, serum albumin, BSA, more particularly KLH or BSA, still more particularly KLH;
provided that said A2M compound:
- is not human A2M, and
- is not a human A2M fragment, which would comprise an A2M polypeptide of the application or invention and be a bigger A2M fragment than said A2M polypeptide of the application or invention.

The invention relates to any compound consisting of the polypeptide or peptide of the invention, and of one or several other structural components, which are linked to said polypeptide or peptide, wherein said other structural components are selected from the group consisting of His tags, BSA, KLH.

According to an embodiment, said other structural components are linked to (*e.g*., by covalent linkage), or otherwise conjugated, bound or coupled to the C-terminal extremity of said A2M polypeptide or peptide of the application or invention (*e.g*., to the last C-terminal amino acid of said C-terminal extremity).

According to a particular embodiment, none of said other structural components are linked to (*e.g*., by covalent linkage), or otherwise conjugated, bound or coupled to the first N-terminal amino acid of the N-terminal extremity of said A2M polypeptide or peptide of the application or invention. The amino group of said first N-terminal amino acid remains thereby free.

The application also discloses any A2M polypeptide of the application or invention, or A2M peptide of the application or invention, or A2M compound of the application or invention, which is linked or otherwise bound to a solid support, such as a glass support or a plastic support (*e.g*., a polystyrene solid support). Said solid support can *e.g*., a chip, or in the form of a plaque or microplate, *e.g*., a plaque or microplate comprising wells, such as an ELISA plaque or microplate.

The application also discloses any non-human animal, *e.g*., any non-human mammal, *e.g.,* any non-human primate or any rodent (*e.g*., any rabbit), the body of which contains at least one A2M peptide of the application or invention and/or at least one A2M compound of the application or invention, wherein said at least one A2M compound comprises at least one A2M peptide of the application or invention (*e.g*., at least one A2M compound comprising at least one A2M peptide of the application or invention and at least one immunogenicity carrier).

### Nucleic acids, vectors, host cells of the application or invention:

The application also discloses any nucleic acid, the sequence of which codes for an A2M polypeptide of the application, or for an A2M peptide of the application, or for an A2M compound of the application, in accordance with the universal genetic code and taking due account of its redundancy. The invention relates to any coding nucleic acid, the sequence of which consists of a sequence coding for the polypeptide of the invention or for the peptide of the invention or for the compound of the invention.

When the one or several of said other structural components that are contained in the A2M compound of the application or invention are not all made of amino acids, the nucleic acid of the application or invention does of course code for the portion of said A2M compound, which is made of amino acids, *i.e.,* for at least one A2M polypeptide or peptide of the application or invention and for those of said other structural components, which comprise or are made of amino acids. In an embodiment, the coding sequence of said nucleic acid consists of a sequence coding for an A2M polypeptide of the application or invention, or for an A2M peptide of the application or invention, or for an A2M compound of the application or invention.

The application further provides vectors, more particularly nucleic acid vectors, still more particularly recombinant vectors. Said vectors are vehicles, in which a nucleic acid has been inserted. The vector itself usually is a nucleic acid molecule comprising a nucleic acid backbone into which the nucleic acid to be transferred is inserted. Said vectors are notably useful for transferring a coding nucleic acid into a cell.

The invention relates to any vector, which comprises a coding nucleic acid inserted therein, wherein the expression product of said coding nucleic acid is the polypeptide of the invention, or the peptide of the invention, or the compound of the invention.

Vectors notably comprise transformation vectors (to transform bacterial cells), transfection vectors (to transfect eukaryotic cells), and expression vectors (to express the transferred nucleic acid). Illustrative vectors notably comprise plasmids and viral vectors (such as retroviruses). An expression vector usually comprises an expression cassette, wherein a coding nucleic acid is operably linked to control sequence(s). The terms "expression cassette", "control sequence(s)", "operably linked" are intended in accordance with their ordinary meaning in the field. For example, the term "expression cassette" means a nucleic acid construct, which contains a coding nucleic acid and the control sequence(s) required for expression of said coding nucleic acid. Such control(s) sequence(s) notably comprise at least one promoter. They usually further include transcriptional and translational stop signals. They may further include a leader sequence and/or a polyadenylation sequence and/or a signal peptide sequence and/or a transcription terminator. The expression "operably linked" implies that the control sequence is a *cis*-acting regulatory element. The person of ordinary skill in the art understands that a regulatory element is appropriately placed with respect to said coding nucleic acid such that said control sequence can direct or modulate the expression of the polypeptide encoded by said nucleic acid. For example, a promoter is placed in 5' of the coding nucleic acid so that it can drive the expression of said coding nucleic acid.

The application more particularly discloses any vector, more particularly any expression and/or recombinant vector:
- which comprises a nucleic acid, the sequence of which codes for
   and/or
- which comprises a coding nucleic acid inserted therein, wherein the expression product of said coding nucleic acid is
   and/or
- which comprises a nucleic acid coding for the expression of
   one or several A2M polypeptide(s) of the application or invention, and/or one or several A2M peptide(s) of the application or invention, and/or one or several A2M compound(s) of the application or invention, *e.g.*, an A2M polypeptide of the application or invention that is linked to a tag that can be useful in the purification or isolation of a recombinantly produced polypeptide, *e.g*., a His tag.

A coding nucleic acid is a nucleic acid, the sequence of which codes for a polypeptide or peptide in accordance with the universal genetic code and taking due account of its redundancy.

Said coding nucleic acid does not code a full length human A2M, and does not code any of the thioester-cleaved and bait-cleaved fragments of said human A2M. Hence, the sequence of the coding nucleic acid may consist of a sequence coding for one or several A2M polypeptide(s) of the application or invention, and/or one or several A2M peptide(s) of the application or invention, and/or one or several A2M compound(s) of the application or invention, *e.g.*, for an A2M polypeptide of the application or invention that is linked to a tag that can be useful in the purification or isolation of a recombinantly produced polypeptide, *e.g.,* a His tag.

The A2M polypeptide(s), peptide(s) or compound(s) coded by said coding nucleic acid is(are) expressed in isolated form. Nevertheless, once said polypeptide or peptide or compound is expressed by said vector, it may bind or link to other structural elements, such as *e.g.*, structural components and/or organelles of the host cell, into which said vector has been transferred.

The application more particularly discloses any expression vector, which, when transferred into a host cell (such as *E. coli* or *S*. *cerevisae*), expresses an A2M polypeptide of the application or invention (in isolated form), or an A2M peptide of the application or invention (in isolated form), or an A2M compound of the application or invention (in isolated form), *e.g*., an A2M polypeptide of the application or invention that is linked to a tag that can be useful in the purification or isolation of a recombinantly produced polypeptide, *e.g*., a His tag.

The expression "in isolated form" means that the sequence of the coding nucleic acid that is inserted into said vector consists of a sequence coding for an A2M polypeptide of the application or invention, or for an A2M peptide of the application or invention, or for an A2M compound of the application or invention. Nevertheless, once said polypeptide or peptide or compound is expressed by said vector in a host cell, it may of course link or bind to other structural elements, such as e.g., structural components and/or organelles of the host cell.

The application further discloses any host cell:
- which comprises or expresses an A2M polypeptide of the application or invention and/or an A2M peptide of the application or invention and/or an A2M compound of the application or invention, and/or
- which comprises a nucleic acid of the application or invention and/or a vector of the application or invention.

The invention relates to any genetically engineered host cell, which comprises a coding nucleic acid consisting of the nucleic acid of the invention and/or which comprises the vector of the invention.

Such host cells may notably be produced by genetic engineering. The host cell may be a prokaryote or a eukaryote. Illustrative prokaryotes notably comprise bacterial cells, such as *E. coli.* Illustrative eukaryotes comprise a mammalian cell, a non-human mammalian cell, a non-human animal cell, a plant cell, a yeast cell (such as *S*. *cerevisiae*)*.* Means for genetically engineering a host cell are known in the art. They for example include the electroporation of the nucleic acid to be incorporated into said cell or the growth of host cells in the presence of vector(s) comprising said nucleic acid.

The nucleic acids, vectors and host cells of the application or invention are notably useful for producing an A2M polypeptide, or an A2M peptide, or an A2M compound of the application or invention, *e.g.,* for *in vitro* producing and/or for recombinantly producing an A2M polypeptide, or an A2M peptide, or an A2M compound of the application or invention.

The application therefore provides a method of production of an A2M polypeptide or an A2M peptide or an A2M compound of the application or invention, which comprises:
- growing a genetically engineered host cell of the application or invention in a cell culture, and
- recovering said A2M polypeptide or A2M peptide or A2M compound from the supernatant of said culture and/or from said grown cells.

The application also provides primers and probes. The notion of primer is known to the person of ordinary skill in the art. For example, it includes any oligonucleotide able to anneal to a target nucleic acid under suitable stringency conditions (such as conditions of high stringency), and allowing for polymerase strand elongation. The typical length of said primer is 15-30 nucleotides (nt), preferably 18, 19, 20, 21, 22, 23, 24 or 25 nt. The nucleotide sequence of the primer may *e.g.,* be at least 85%, more particularly at least 90%, still more particularly at least 95%, still more particularly 100% identical to:
- the sequence of the same length that is the 5' end extremity of said target nucleic acid (forward primer), or
- the sequence that is the complementary sequence of the sequence of the same length that is the 3' end extremity of said target nucleic acid (reverse primer),
whereby the sequence of the amplified nucleic acid by said primer pair is a sequence consisting of the sequence of a nucleic acid of the application or invention. The notion of probe is also known to the person of ordinary skill in the art. For example, it includes any oligonucleotide able to anneal to a target nucleic acid under the desired hybridization conditions (such as conditions of high stringency). The typical length of said probe is 20-55 nucleotides (nt), preferably 15-60 nt, more preferably 20-55 nt, more preferably 30-50 nt, more preferably 35-45 nt. Preferably, said probe is fluorescently labelled and/or is in a TAOMAN ™ probe format (hydrolysis probes) and/or in a molecular BEACON™ format (beacon probes or molecular beacon probes) and/or in a SCORPION™ probe format.

The portion of the probe that anneals to said target nucleic acid may *e.g.,* be a nucleotide sequence that is at least 85%, more particularly at least 90%, still more particularly at least 95%, still more particularly 100% identical to a sequence of the same length that is contained in said target nucleic acid or to the complementary sequence thereof.

Sequence identity percentages are to be understood in accordance with their ordinary meaning in the field (schematically: the percentage of identical matches between the two sequences over the optimum alignment of the two sequences; see e.g., Needleman and Wunsch 1970). In the application or invention, said target nucleic acid is a nucleic acid coding for an A2M polypeptide of the application or invention, such as a nucleic acid coding for the polypeptide of SEQ ID NO: 12, 13, 14 or 15. According to an embodiment, the primers and/or the probes specifically hybridize to a nucleic acid of the application or invention (or to the complementary sequence thereof) under conditions of high stringency.

An example of conditions of high stringency is hybridization to filter-bound DNA in 5xSaline-Sodium Citrate (SSC), 2% Sodium Dodecyl Sulfate (SDS), 100 micrograms/mL single stranded DNA at 55-65°C for 8 hours, and washing in 0.2xSSC and 0.2% SDS at 60-65°C for thirty minutes. The application provides a set of oligonucleotides, comprising at least one primer pair of the application, *i.e.,* a forward primer and a reverse primer of the application. Said set may further comprise at least one probe of the application. The primers and/or probes of the application are notably useful for amplifying, *e.g.*, by PCR, a nucleic acid of the application or invention, *e.g.,* from a biological sample (*e.g*., from blood, plasma or serum) of a hepatitis patient.

### Binding reactants of the application (antibodies and antibody fragments):

The application also discloses any antibody or antibody fragment, which specifically binds to an A2M polypeptide or peptide of the application or invention.

According to a particular embodiment, the antibody or antibody fragment of the application binds to said A2M polypeptide or peptide of the application or invention, without binding to the full length human A2M protein. More particularly, the antibody or antibody fragment of the application binds to the N-terminal extremity of said A2M polypeptide or peptide of the application or invention, without binding to the full length human A2M protein. Still more particularly, the antibody or antibody fragment of the application binds to the N-terminal extremity of said A2M polypeptide or peptide of the application or invention at a site or epitope, which comprises the N-terminal amino group of said A2M polypeptide or peptide. By convention, when the person of ordinary skill in the art is drawing an amino acid chain:
- the amino acid bearing the free amino group (*i.e.,* the free alpha-amino group) is drawn to the left-hand side and is the first amino acid (first N-terminal amino acid), and
- the amino acid bearing the free carboxyl group (*i.e.,* a free alpha-carboxyl group) is drawn to the right-hand side and is the last amino acid (last C-terminal amino acid).

The N-terminal amino group of said A2M polypeptide or peptide is the amino group, which the person of ordinary skill in the art considers to be the free amino group of the first N-terminal amino acid of said A2M polypeptide or peptide, when drawing the amino acid chain of said A2M polypeptide or peptide in an N-term to C-term orientation, *i.e.,* the amino group which the person of ordinary skill in the art considers to be the N-terminal alpha-amino group. The antibody or antibody fragment of the application specifically binds to said A2M polypeptide or peptide of the application or invention, when the N-terminal amino group of said A2M polypeptide or peptide is free. The term "free" is intended in accordance with its ordinary meaning in the field. A free N-terminal amino group is not engaged in a peptide bond (*i.e.,* it is not forming a peptide bond). More generally, a free N-terminal amino group is not engaged in any covalent bond. Still more generally, a free N-terminal amino group is not engaged in any chemical bond, at least not in a chemical bond, which may prevent an antibody from binding thereto. In other words, a free N-terminal bond is unbound such as to be available for antibody binding thereto. When said N-terminal amino group is not free, *e.g.*, when it is forming a peptide bound with another amino acid, the antibody or antibody fragment of the application does not bind to the amino acid chain of the A2M polypeptide or peptide of the application or invention. For example, when the amino acid chain of said A2M polypeptide or peptide of the application or invention is inserted within another amino acid sequence, *e.g*., when it is contained within the full length sequence of human A2M, the N-terminal amino group of said A2M polypeptide or peptide sequence is not free: it forms a peptide bond with the amino acid, which immediately precedes it within the full length human A2M sequence.

The antibody or antibody fragment of the application does not bind to such an inserted A2M polypeptide or peptide amino acid chain. For the same reason:
- the antibody or antibody fragment of the application does not bind to the thioester-cleaved fragments of the human A2M protein, more particularly to the C-terminal thioester-cleaved fragment;
- the antibody or antibody fragment of the application does not bind to the bait-cleaved fragments of the human A2M protein, more particularly to the C-terminal bait-cleaved fragment.

More particularly, the application discloses any antibody or antibody fragment, which binds to the A2M polypeptide of SEQ ID NO: 12, 13, 14 or 15, without binding to the full length human A2M protein of SEQ ID NO: 9.

An antibody of the application can be a polyclonal antibody or a monoclonal antibody, more particularly a monoclonal antibody.

An antibody fragment of the application can be any antibody fragment, which has retained the binding properties of an antibody of the application, more particularly its binding specificity. Illustrative antibody fragments of the application comprise Fab fragments, F(ab')2, Fv, Fab/c (consisting of one Fab and a complete Fc). Antibody fragments of the application also encompass constructs made of antibody fragments, such as a single chain Fv (scFv), *i.e.,* a construct wherein the H-chain V-region is linked to the L-chain V-region, *e.g.,* through an an appropriate linker.

The application discloses any hybridoma, which produces (or secretes) a monoclonal antibody of the application, as well as any composition comprising such an hybridoma.

The application further provides a method of producing an antibody of the application, which comprises:
- immunizing an animal, more particularly a non-human animal, with at least one A2M polypeptide of the application or invention, or with at least one A2M peptide of the application or invention or at least one A2M compound of the application or invention or with an antigenic functional derivative thereof, more particularly with at least one A2M peptide of the application or invention or with at least one A2M compound of the application or invention that comprises at least one A2M peptide of the application or invention,
- recovering the antibodies from said immunized animal.

To immunize said animal, the person may use any A2M polypeptide, peptide or compound of the application or invention, which he/she finds appropriate.

Advantageously, the person of ordinary skill in the art may use one or several of the A2M peptides of the application or invention, such as one or several of the peptides of SEQ ID NOs: 1-8. The A2M peptide(s) may be linked, preferably at its/their C-terminal end, to an immunogenicity carrier, such as KLH, serum albumin, BSA, bovine thyroglobulin, soybean trypsin inhibitor, e.g., by using a bifunctional or derivatizing agent, for example, maleimidobenzoyl sulfosuccinimide ester (conjugation through cysteine residues), N-hydroxysuccinimide (through lysine residues), glutaraldehyde, succinic anhydrid or SOCl₂.

Means to produce antibodies are known to the person or ordinary skill in the art. Animals can be immunized with an A2M polypeptide of the application or invention or with an A2M peptide of the application or invention or with an A2M compound of the application or invention or with an antigenic functional derivative thereof, more particularly with an A2M peptide of the application or invention or with an A2M compound of the application or invention that comprises at least one A2M peptide of the application or invention.

Appropriate animals notably comprise mammals, more particularly non-human mammals, such as rabbits. For example, the animal is injected intraperitoneally or subcutaneously with said A2M polypeptide, peptide or compound or an antigenic functional derivative thereof. Said A2M polypeptide, peptide or compound or antigenic functional derivative thereof, may be diluted with, or suspended in an appropriate volume of PBS (Phosphate-Buffered Saline), physiological saline or the like. An appropriate volume of a standard adjuvant can be mixed with the product, if necessary or desired. Illustrative standard adjuvants notably comprise Freund's (complete or incomplete) adjuvant, mineral gels such as aluminium hydroxide, surface active substances such as lysolecithin, pluronic polyols, polyanions, peptides, oil emulsions, KLH, dinitrophenol, and potentially useful human adjuvants such as BCG (bacille Calmette-Guerin) and *Corynebacterium parvum.*

Emulsification can be performed and the solution is administered to mammals, e.g., several times every 4 to 21 days. Polyclonal antibodies are heterogeneous populations of antibody molecules, which can be derived from the sera of immunized animals.

Monoclonal antibodies, which are homogeneous populations of antibodies to a particular antigen, may be obtained by any technique which provides for the production of antibody molecules by continuous cell lines in culture. These include, but are not limited to the hybridoma technique of Köhler and Milstein (Köhler and Milstein 1975; U.S. Pat. No. 4,376,110), the human B-cell hybridoma technique (Kosbor *et al.* 1983; Cole *et al.* 1983) and the EBV-hybridoma technique (Cole *et al.* 1985). Such antibodies may be of any immunoglobulin class including IgG, IgM, IgE, IgA, IgD and any subclass thereof. The hybridoma producing a monoclonal antibody of the application may be cultivated *in vitro* or *in vivo.*

The method of producing an antibody the application may further comprise the step of purifying or isolating those antibodies, which specifically binds to said A2M polypeptide, peptide or compound of the application or invention. More particularly, the method of the application may comprise the step of purifying or isolating those antibodies:
- which specifically binds to said A2M polypeptide, peptide or compound of the application or invention, when the N-terminal amino group of said A2M polypeptide, peptide or compound is free, and
- which does not bind to the amino acid chain of said A2M polypeptide, peptide or compound, when the N-terminal amino group of said A2M polypeptide, peptide or compound is not free.

For example, the method of the application may comprise the step of purifying or isolating those antibodies:
- which specifically binds to said A2M polypeptide, peptide or compound of the application or invention, when the N-terminal amino group of said A2M polypeptide, peptide or compound is free, and
- which does not bind to the full length human A2M protein (*e.g.*, the A2M protein of SEQ ID NO: 9 or 10) and/or to the C-terminal thioester-cleaved fragment thereof and/or to the C-terminal bait-cleaved fragment thereof.

Isolation and/or purification of the antibodies can be performed using affinity column(s), for example a protein A column (*e.g*., for purification and/or isolation of polyclonal antibodies) or a protein B column (*e.g*., for purification and/or isolation of a monoclonal antibody). Other standard methods for isolation and/or purification of antibodies may be used. For example, a chromatography column other than the above affinity column, a filter, ultrafiltration, a method of salting out, dialyses and the like may be appropriately selected and combined for use, so that antibodies can be isolated and purified (Harlow and Lane 1988 "Antibodies A Laboratory Manuaf*"*)*.*

The application also encompasses a kit comprising at least one antibody or antibody fragment of the application. Such a kit may further comprise one or several of the following means:
- means suitable for collecting or obtaining a biological sample from a human patient, *e.g*., means for the collection of blood, and/or means for separating plasma or serum from blood, such as syringe(s) and/or tube(s) (*e.g*., centrifugation tube(s)), and/or
- means for purifying said biological sample, more particularly means for depleting albumin and/or immunoglobulin G from said biological sample (*e.g.*, from blood, plasma or serum), such as a resin (*e.g*., agarose beads) allowing a selective binding of albumin and/or immunoglobulin G from a biological sample, *e.g*., cross-linked agarose beads with covalently attached CIBACRON BLUE F3GA™ dye and protein A (such cross-linked agarose beads are *e.g*., available in the AURUM® kit, Bio-Rad, Hercules, CA), and/or
- means suitable for denaturing proteins, e.g., heating means or a surfactant that denatures proteins, such as Sodium Dodecyl Sulfate (SDS), TRITON®, urea, thiourea.

Said kit may further comprise any means that are useful for the performance of antibody-antigen reactions and/or for their detection.

### Illustrative applications of the means of the application or invention:

The means of the application or invention are notably useful in the field of hepatitis, more particularly of viral hepatitis, such as Hepatitis C Virus (HCV) infection and/or Hepatitis B Virus (HBV) infection and/or Hepatitis D Virus (HDV) infection, more particularly of HCV infection.

The means of the application or invention are more particularly useful for determining the extent of liver fibrosis, *i.e.,* for staging fibrosis. The extent of liver fibrosis is staged according to a scoring system, known as the Metavir score (F score), as follows:
- no fibrosis (Metavir F score = F0);
- mild fibrosis (Metavir F score = F1);
- moderate fibrosis (Metavir F score = F2);
- advanced fibrosis (Metavir F score = F3);
- severe fibrosis or cirrhosis (Metavir F score = F4).

Determining the fibrosis stage is of upmost clinical importance. Indeed, due to the highly noxious secondary effects of the current treatments, the fibrosis stage is the main element on which is based the decision to administer a treatment. The F2 Metavir score is generally chosen as a threshold for treatment of HCV infection. The fibrosis score thereby is a prognostic tool. Determining the fibrosis stage of a patient before and/or during and/or after a treatment also enables to determine whether this treatment is useful in reducing the extent of fibrosis. Various markers can be used either individually on their own, or in combination with other marker(s) and/or with clinical feature(s) [such as age, sex, etc.], to determine the fibrosis score from a non-invasive biological sample, such as blood, serum, plasma. Human A2M is one of such markers. Please see the background section above. Please also see the French patent application FR 1151022 filed on 9 February 2011, and its foreign counter-parts, notably its US counter-part (US 61/440,986 filed on 9 February 2011), the respective contents of which being herein incorporated by reference. Said French patent application FR 1151022 or foreign counterpart thereof describe a combination of markers, which enables to score the fibrosis stage, more particularly to determine whether the fibrosis score of a hepatic patient is of at most F1 or of at least F2. This combination is the combination of the level of transcription or translation of the following genes:
- SPP1, and
- at least one gene among A2M and VIM, and
- at least one gene among IL8, CXCL10 and ENG, and
- optionally, at least one gene among the following list of sixteen genes : IL6ST, p14ARF, MMP9, ANGPT2, CXCL11, MMP2, MMP7, S100A4, TIMP1, CHI3L1, COL1A1, CXCL1, CXCL6, IHH, IRF9, MMP1,
wherein SPP1 is secreted phosphoprotein 1; VIM is vimentin; IL8 is interleukin-8; CXCL10 is C-X-C motif chemokine 10; ENG is endoglin; IL6ST is interleukin-6 signal transducer; p14ARF is cyclin-dependent kinase 2A inhibitor; MMP9 is matrix metalloproteinase 9; ANGPT2 is angiopoietin-2; CXCL11 is C-X-C motif chemokine 11; MMP2 is matrix metalloproteinase 2; MMP7 is matrix metalloproteinase 7; S100A4 is protein S100-A4; TIMP1 is metalloproteinase inhibitor 1; CHI3L1 is chitinase-3-like protein; COL1A1 is collagen alpha-1(I) chain; CXCL1 is growth-regulated alpha protein C-X-C motif chemokine 1; CXCL6 is C-X-C motif chemokine 6; IHH is Indian hedgehog protein; IRF9 is interferon regulatory factor 9; MMP1 is matrix metalloproteinase-1.

The A2M polypeptides of the application or invention are differentially abundant depending on the stage of hepatic fibrosis. More particularly, the content or concentration of an A2M polypeptide of the application or invention is higher in those patients, whose fibrosis score is of at least F2, compared to those patients, whose fibrosis score is of at most F1 (Metavir scoring system). This difference is notably observed in a biological fluid from said patients (*e.g*., blood, serum, plasma, urine or cerebrospinal fluid (CSF), more particularly blood, serum or plasma). Please see the examples below. Therefore, the A2M polypeptides of the application or invention are markers, which can be used either individually or in combination with each other and/or with other marker(s) and/or clinical feature(s), such as in the above-mentioned marker combination. Detecting an A2M polypeptide of the application or invention, and/or measuring its content or concentration, enable to, or contribute to enable:
- to detect liver fibrosis and/or
- to determine a fibrosis score and/or
- to determine whether the fibrosis score is of at most F1 or of at least F2.

For said A2M polypeptide detection and/or measure, antibody(ies) or antibody fragment(s) of the application can be used. To produce such antibodies, A2M peptide(s) of the application or invention can be used.

Therefore, the application describes the *in vitro* use of at least one antibody or antibody fragment of the application or of at least one kit of the application:
- for detecting at least one polypeptide or peptide of the application or invention (or a polypeptide or peptide having the same starting position), and/or
- for detecting hepatitis and/or liver fibrosis, and/or
- for measuring the content of concentration at least one polypeptide of the application or invention (or a polypeptide having the same starting position), and/or
- for scoring a liver fibrosis stage, and/or
- for determining whether a liver fibrosis is at a stage, which scores at most F1 or at a stage, which scores at least F2, in accordance with the Metavir scoring system.

The invention relates to the *in vitro* use of an antibody or antibody fragment or of a kit comprising said antibody or antibody fragment, for detecting hepatitis and/or liver fibrosis, wherein said antibody or antibody fragment specifically binds to the N-terminal extremity of a polypeptide or peptide of the invention, without binding to the full length human A2M protein, and wherein said antibody or antibody fragment does not bind to the C-terminal thioester-cleaved fragment of said human A2M, and does not bind to the C-terminal bait-cleaved fragment of said human A2M.

The invention relates to the *in vitro* use of an antibody or antibody fragment or of a kit comprising said antibody or antibody fragment, for scoring a liver fibrosis stage and/or for determining whether a liver fibrosis is at a stage, which scores at most F1 or at a stage, which scores at least F2, in accordance with the Metavir scoring system, wherein said antibody or antibody fragment specifically binds to the N-terminal extremity of the polypeptide of any one of claims 1-7 or of the peptide of claim 8, without binding to the full length human A2M protein, and wherein said antibody or antibody fragment does not bind to the C-terminal thioester-cleaved fragment of said human A2M, and does not bind to the C-terminal bait-cleaved fragment of said human A2M.

Said antibody or antibody fragment may specifically bind to the N-terminal extremity of the polypeptide of the invention or of the peptide of the invention at a site, which comprises the non-covalently linked N-terminal alpha-amino group of said polypeptide.

Said kit may further comprise at least one means selected from:
- at least one syringe, and/or
- at least one tube, and/or
- at least one resin having a selective binding affinity for albumin and/or immunoglobulin G, and/or
- at least one protein denaturant.

The invention also relates to the *in vitro* use of the peptide of the invention for *in vitro* screening antibodies or antibody fragments.

The application also describes a method:
- for detecting at least one polypeptide of the application or invention (or a polypeptide having the same starting position), and/or
- for detecting hepatitis and/or liver fibrosis, and/or
- for measuring the content of concentration at least one polypeptide of the application or invention (or a polypeptide having the same starting position),
which comprises:
- placing at least one antibody or antibody fragment of the application in contact with a biological sample of a biological fluid of a human being under conditions enabling reactions of the antibody-antigen type, more particularly under conditions enabling the binding of said at least one antibody to its epitope,
- detecting the antibody or antibody fragment thereby bound, and/or measuring the concentration of antibody or antibody fragment thereby bound.

The presence of bound antibody or antibody fragment is indicative of the fact that said human being has at least one A2M polypeptide of the application or invention in his/her biological fluid. The presence of bound antibody or antibody fragment is also indicative of the fact that said human being has hepatitis and/or liver fibrosis. The concentration of bound antibody or antibody fragment is the concentration of the A2M polypeptide of the application or invention, onto which said antibody or antibody fragment binds.

Said biological sample of a biological fluid of a human being can more particularly be a biological sample of blood, serum, plasma, urine or cerebrospinal fluid (CSF), still more particularly of blood, serum, plasma. Said human being preferably is a human being infected by at least one hepatitis virus, still more particularly by at least HCV, even still more particularly by HCV. Said human being may e.g., be afflicted by a chronic hepatitis.

The detected presence or absence of, or the measured concentration of, bound antibody or antibody fragment can be compared to the presence/absence, or to the concentration of, bound antibody or antibody fragment, respectively, which is observed:
- in a cohort of hepatitis patients, more particularly of HCV patients, who score at most F1 in accordance with the Metavir scoring system, and in
- in a cohort of hepatitis patients, more particularly of HCV patients, who score at least F2 in accordance with the Metavir scoring system.

This comparison can be made by any statistical analysis tool that the person of ordinary skill in the art may find appropriate to classify said detected presence or absence, or said measured concentration, respectively, in the cohort of the cohort of patients, who are at most F1, or in the cohort of the patients, who are at least F2, thereby attributing a score of "at most F1" or "of at least F2" to said human being.

A similar methodology but performed with a range of cohorts, which corresponds to the full range of Metavir scores, enables to score said human being as being F0, F1, F2, F3, F4. Of course, partial scoring range may equally be performing (such as e.g., at most F1, F2, at least F3).

Hence, the method of the application also is a method:
- for scoring a liver fibrosis stage, and/or
- for determining whether a liver fibrosis is at a stage, which scores at most F1 or at a stage, which scores at least F2, in accordance with the Metavir scoring system.

Since the decision to administer an anti-hepatitis and/or anti-HCV and/or anti-fibrosis treatment is usually directly linked to the fact that the patient has a liver fibrosis that scores at least F2 according to the Metavir scoring system, the method of the application to determine whether a liver fibrosis is at a stage, which scores at most F1 or at a stage, which scores at least F2, in accordance with the Metavir scoring system, also is a method to administer an anti-hepatitis and/or anti-HCV and/or anti-fibrosis treatment in a patient in need thereof, which comprises:
- determining by a means of the application or invention (e.g., using at least one antibody or antibody fragment of the application), whether the patient's liver is at a fibrosis stage, which scores at most F1 or at a stage, which scores at least F2 (in accordance with the Metavir scoring system), and
- administering said treatment to said patient if his/her liver scores at least F2.

The application also describes the use of at least one A2M peptide of the application or invention to *in vivo* produce an antibody or antibody fragment of the application. Said A2M peptide is then used as antigen, and may be linked to an immunogenicity carrier such as KLH or BSA. The application therefore describes the use of at least one A2M peptide of the application or invention, for its *in vivo* injection in an immunocompetent non-human animal and/or for the *in vivo* production of antibodies or antibody fragments.

The application also discloses any non-human immunocompetent animal (*e.g.*, a rabbit), the body of which comprises at least one polypeptide of the application or invention and/or at least one peptide of the application or invention.

The application also describes the use of at least one A2M peptide of the application or invention to *in vitro* produce an antibody or antibody fragment of the application, *e.g*., by recombinant production.

The application also describes the use of at least one A2M peptide of the application or invention to screen antibodies or antibody fragments, more particularly to identify and/or isolate an antibody or antibody fragment of the application. Said peptide is then used as an affinity ligand, and may then *e.g*., be linked to a protein, polypeptide or peptide, which can bind solid support (such as a glass support or a plastic support *e.g*., a polystyrene solid support), for example to BSA.

The term "comprising", which is synonymous with "including" or "containing", is openended, and does not exclude additional, unrecited element(s), ingredient(s) or method step(s), whereas the term "consisting of" is a closed term, which excludes any additional element, step, or ingredient which is not explicitly recited.

The term "essentially consisting of" is a partially open term, which does not exclude additional, unrecited element(s), step(s), or ingredient(s), as long as these additional element(s), step(s) or ingredient(s) do not materially affect the basic and novel properties of the invention.

The term "comprising" (or "comprise(s)") hence includes the term "consisting of" ("consist(s) of"), as well as the term "essentially consisting of" ("essentially consist(s) of").

Accordingly, the term "comprising" (or "comprise(s)") is, in the application or invention, meant as more particularly encompassing the term "consisting of" ("consist(s) of"), and the term "essentially consisting of" ("essentially consist(s) of").

In an attempt to help the reader of the application, the description has been separated in various paragraphs or sections. These separations should not be considered as disconnecting the substance of a paragraph or section from the substance of another paragraph or section. To the contrary, the description encompasses all the combinations of the various sections, paragraphs and sentences that can be contemplated.

Each of the relevant disclosures of all references cited herein is specifically incorporated by reference. The following examples are offered by way of illustration, and not by way of limitation.

### EXAMPLES

### EXAMPLE 1:

### Materials and Methods

### Serum samples

Serum samples were collected at Beaujon Hospital (Paris, France) from 21 HCV-infected patients who were not undergoing treatment. The study was approved by the local ethics committee and was conformed to the 1975 Declaration of Helsinki. All patients gave written informed consent.

The degree of hepatic fibrosis in each HCV-infected patient was determined using the Metavir scoring scale. Nine patients had mild fibrosis (F1), ten patients had moderate fibrosis (F2), one patient had severe fibrosis (F3) and one patient had cirrhosis (F4). Only the serum samples from patients with F1 and F2 liver fibrosis (total n=19) were used for the differential study so as to focus on the identification of markers that might differentiate between F1 and F2 fibrosis; their clinical characteristics are listed in Table 1.

**Table 1. Clinical parameters and total A2M concentration in serum of the subjects under study. Results are expressed as medians (range).**

| | Total | F1 HCV | F2 HCV |
|---|---|---|---|
| Patients (n) | 19 | 9 | 10 |
| Gender (M/F) | 10/9 | 2/7 | 8/2 |
| Age (years) | 53 (29-72) | 51 (29-66) | 53 (38-72) |
| BMI (kg/m²) | 24.4 (19.8-30.9) | 23.8 (19.8-29.7) | 24.7 (21.9-30.9) |
| total A2M (mg/mL) | 2.97 (1.44-4.24) | 2.20 (1.50-3.49) | 3.71 (1.44-4.24) |

### Serum fractionation

The AURUM® system (Bio-Rad, Hercules, CA) allows the depletion of the two most abundant proteins in serum (*i.e.,* albumin and IgG) by using cross-linked agarose beads with covalently attached CIBACRON BLUE F3GA™ dye and protein A. Sixty microlitres of each crude serum sample were added onto the mini-column and depleted according to the manufacturer's instructions. Eluted, depleted samples were stored at -80°C until analysis.

### Protein assay

The total protein content of each depleted serum sample was quantified using the QUICK START BRADFORD PROTEIN ASSAY KIT 2® (Bio-Rad) according to the manufacturer's instructions with bovine serum albumin (BSA) as standard (Bio-Rad).

### One dimensional gel electrophoresis (1-DE)

Ten micrograms of proteins from each depleted serum sample were diluted in Laemmli sample buffer (Bio-Rad) in which 5% 2-ß-mercaptoethanol had been added. Samples were incubated at 95°C for 10 min and ran through 12% Tris-HCI/SDS-PAGE gels. Gels were stained with COOMASSIE® Brilliant Blue R250. The signal intensity of each band was quantified with a GS-800 Densitometer (Bio-Rad) and the QUANTITY ONE 1D-ANALYSIS® software (Bio-Rad).

### Two dimensional gel electrophoresis (2-DE)

All depleted serum samples were treated with the ReadyPrep™ 2-DE Clean-up kit (Bio-Rad) before 2-DE analysis to remove impurities such as nucleic acids, lipids and salts. One hundred twenty or sixty micrograms of proteins from each sample were solubilised in 200 µL rehydration buffer (7 M Urea, 2 M Thiourea, 4% CHAPS, 15 mM DTT, 2 mM TBP, 0.2% or 0.4% Ampholytes, Bromophenol Blue) and loaded onto respectively 11 cm, pH 3-10 or pH 5.5-6.7 IPG strips (Bio-Rad). Strips were passively re-hydrated at 20°C for 12 h.

Proteins were isoelectrically focused at 250 V for 20 min (pH 3-10) or 15 min (pH 5.5-6.7), then at 8,000 V for 150 min (pH 3-10) or 60 min (pH 5.5-6.7) and finally maintained at 8,000 V for a total of 25,000 Vh/gel (pH 3-10) or 35,000 Vh/gel (pH 5.5-6.7). Strips from the first dimension separation were equilibrated in 2 mL reduction buffer (50 mM Tris pH 6.8, 6M Urea, 30% Glycerol, 2% SDS, 65 mM DTT) for 15 min and then in 2 mL alkylation buffer (50 mM Tris pH 8.8, 6M Urea, 30% Glycerol, 2% SDS, 81 mM lodoacetamide) for 15 min. Strips were rinsed in 1X TGS running buffer (10X Tris / Glycine / SDS, Bio-Rad) and then loaded onto 4-20% Tris-HCI Precast Criterion linear gradient gels (Bio-Rad) that were run at 80 V for 20 min, then at 150 V for 1 h and at 200 V for 15 min in the CRITERION™ DODECA™ Cell system (Bio-Rad). Finally, proteins were blotted onto nitrocellulose membranes at 25 V for 45 min using the Trans-Blot SD Semi-Dry Transfer Cell system (Bio-Rad).

### Western blot analysis

After 2-DE separation, serum proteins were blotted onto nitrocellulose membranes that were blocked in 5% non-fat dried milk/Phosphate Saline Buffer (Dulbecco) overnight.

They were probed with 1ug/mL of anti-human A2M goat polyclonal antibody (R&D Systems, AF1938) and then with anti-goat IgG HRP conjugated secondary antibody (1/80 000 dilution) (Sigma, A5420). Antibody binding was detected with ECL Western Blotting Detection Reagents (GE Healthcare) and Kodak BioMax Light-2 Films (VWR) were used.

For detection of the N-terminal and C-terminal regions of A2M , nitrocellulose membranes were probed respectively with 1ug/mL of anti-human N-Ter A2M mouse monoclonal antibody (Abnova, H00000002-M03) and 1ug/mL of anti-human C-Ter A2M rabbit polyclonal antibody (Abcam, ab58703). Then, membranes were probed respectively with an anti-mouse (1/80,000 dilution) (Sigma, A9044) and an anti-rabbit IgG HRP conjugated secondary antibody (1/80,000 dilution, Sigma, A9169).

### Differential image analysis

The PROGENESIS SAMESPOT® software (Non Linear Dynamics, Quayside, UK) was used for spot detection, quantification and comparison. The 2-DE gels of the 19 serum samples (patients with F1 and F2 liver fibrosis) fractionated with AURUM® were aligned.

After automatic matching of the spots, gels were manually inspected to detect matching errors. The intensity of each spot was normalized to the total intensity of all the valid spots in the whole gel. Spots in gels from patients with F1 and F2 liver fibrosis were considered to have a significantly different intensity based on the following fixed parameters: ANOVA < 0.05 and median fold change > 1.3.

### Determination of the N-Terminal or C-terminal localization of the A2M 40kDa fragments

To determine whether the A2M 40kDa fragments are N-terminal or C-terminal A2M fragments, an anti-human N-terminal A2M mouse monoclonal antibody (Abnova, H00000002-M03) and an anti-human C-terminal A2M rabbit polyclonal antibody (Abcam, ab58703) have been used. The sequence of the respective epitopes of these antibodies has been determined by the SPOT method [Franck 1992].

Overlapping synthetic peptides corresponding to the amino acid sequence of human A2M (Expasy accession number P01023) were prepared using the epitope mapping technique [Franck 1992]. Two series of membrane-bound peptides (overlapping 15-mer peptides with a frameshift of two residues) were synthesized according to the procedure described by Molina *et al.* 1996.

After synthesis, non-specific binding to the membranes was blocked by incubation with TBS-TWEEN® 20% saccharose, blocking buffer diluted 1/50 (Roche, Germany) at room temperature overnight, and then membranes were probed with anti-N-terminal or anti-C-terminal antibodies (1ug/mL in TBS-Tween 20% Saccharose, blocking buffer diluted 1/50).

Antibody binding (anti-mouse HRP conjugated, Sigma, A9044; or anti-rabbit HRP conjugated, Sigma, A9169; 1/80 000 dilution) was revealed by incubation (at room temperature for 60 min) with ECL (GE Healthcare, RPN2106).

### In-Gel Digestion

The 40kDa A2M region from a 2-DE gel stained with COOMASSIE® Brilliant Blue R250 (150ug of commercially available total A2M purified from human plasma; Sigma, M6159) was manually cut into pieces of about 2 mm² and transferred to 2 mL EPPENDORF® tubes. Pieces of gel were first washed with 1 mL of water and then 1 mL of 25 mM NH₄HCO₃. De-staining was performed twice in the presence of 1 mL of 50% acetonitrile in 25 mM NH₄HCO₃. Pieces of gel were dehydrated twice by 1 mL of 100 % CH₃CN, and finally dried at room temperature.

### Tryptic digestion:

Thirty microliters of a trypsin solution (Sequencing Grade Modified Trypsin, Promega, Madison, USA), at a concentration of 0.0125ug/uL in 25 mM NH₄HCO₃, were added to every gel pieces and were kept for 15 min on ice. Forty microlitres of 25 mM NH₄HCO₃ were added, and the samples were kept another 15 min at room temperature. Digestion was performed overnight at 37°C.

### Endoproteinase Glu-C digestion:

Ten microliters of a Endoproteinase Glu-C solution (Sequencing Grade from Staphylococcus aureus V8, Roche, Germany), at a concentration of 0.1ug/uL in 25 mM NH₄HCO₃, were added to every gel pieces that were kept for 15 min on ice. Sixty microlitres of 25 mM NH₄HCO₃ were added, and the samples were kept another 15 min at room temperature. Digestion was performed overnight at 25°C.

After digestion, peptides were extracted by addition of 200 uL of 75 % acetonitrile in water 2% formic acid. Digests were sonicated in an ultrasonic bath for 10 min and supernatants were transferred into 0.5 mL EPPENDORF® tubes then dried in a vacuum centrifuge. The peptides were suspended in 10 µL 2 % formic acid, and moved in HPLC polypropylene tubes.

### Mass Spectrometry

The protein digests were analyzed using 2 different liquid chromatography - tandem mass spectrometry (LC-MS/MS) configurations: a High Capacity ion trap mass spectrometer (Esquire HCT; Bruker Daltonik GmbH, Bremen, Germany) interfaced with a nano-HPLC Chip-Cube system (Agilent Technologies, Santa Clara, USA) and a QTOF mass spectrometer (Maxis; Bruker Daltonik GmbH, Bremen, Germany) interfaced with a nano-HPLC Ultimate 3000 (Dionex,Sunnyvale, USA).

### Chip-Cube - HCT configuration:

The chips contained both the pre-column and the column (Zorbax 300SB-C18; Agilent Technologies, Santa Clara, USA). Samples were first loaded onto the 4 mm enrichment cartridge at a flow rate of 4 µL/min using solvent A (0.1 % formic acid in water, v/v). After pre-concentration, peptides were separated on the column (75 µm diameter, 43 mm length) at a flow rate of 0.3 µL/min using a 30 min linear gradient from 3 % to 45 % solvent B (0.1 % formic acid, 90 % acetonitrile in water, v/v/v), and eluted into the mass spectrometer. A capillary voltage of 1.8-2.1 kV in the positive ion mode was used together with a dry gas flow rate of 4.5 L/min at 250°C. A first full-scan mass spectrum was measured in the 310 m/z to 1,800 m/z range, followed by a second scan at higher resolution to measure precisely the mass of the three major ions in the previous scan.

Finally, a third scan was performed to acquire the collision-induced MS/MS spectra of the selected ions.

### Ultimate 3000 - Maxis configuration:

The protein digests were analyzed using a QTOF mass spectrometer (Maxis; Bruker Daltonik GmbH, Bremen, Germany), interfaced with a nano-HPLC Ultimate 3000 (Dionex,Sunnyvale, USA). Samples were first loaded onto the pre-column (C18 PepMap100, 300 µm x 5 mm, 5 µm, 100 A, Dionex) at a flow rate of 20 µL/min for 3 min with solvent A (0.1 % formic acid, 2 % acetonitrile in water, v/v/v). After pre-concentration, peptides were separated on the reversed-phase column (C18 PepMap100, 75 µm x 250 mm, 3 µm, 100 A, Dionex) at a flow rate of 0.3 µL/min using a 69 min linear gradient from 8 % to 27 % solvent B (0.1 % formic acid, 90 % acetonitrile in water, v/v/v), and eluted into the mass spectrometer. The instrument was operated in the positive ion mode and the nano-ESI source parameters are: a capillary voltage of 5000 V, a nebulization gas pressure of 0.4 bars and a dry gas flow rate of 4 L/min at 140°C. After an initial MS scan at 2 Hz over the mass range of 50-2,200 Th, the five most intense precursors were fragmented by collision-induced dissociation.

### Mass spectrometry data analysis

The tandem mass spectrometry (MS/MS) raw data obtained were analyzed using Data Analysis software (Bruker Daltonik GmbH, Bremen, Germany) to generate the peak lists.

The UniProtKB/Swiss-Prot (release 20100121) was queried locally using the Mascot search engine (v. 2.2.04; Matrix Science, London, U.K.) and with the following parameters: Human for the taxonomy, semi-trypsin or semi-endoGluV8 as enzyme, 2 missed cleavages allowed, carbamidomethylation of Cystein as fixed modification, oxidation of Methionine as variable modification, 0.1 Da mass accuracy in MS and 0.3 Da in MS/MS for HCT experiment vs. 10 pmm in MS and 0.05 Da in MS/MS for Maxis experiments. Under these conditions, peptides with a *p-value* < 0.05 were automatically validated. Peptides with lower scores were manually validated when fragmentation displayed at least 4 y-ions with 3 of them consecutive within the 10 most intense peaks.

### N-terminal sequencing of the 40kDa A2M fragments

10 and 100 µg of commercially available human A2M (Sigma) were separated on Tris-HCI 12% SDS-PAGE gels. Proteins were blotted by liquid transfer (10 mM CAPS pH 11, 10% methanol) onto a PVDF membrane and stained with a solution made of 0.1% Brilliant Blue R250, 40% methanol, 30% acetic acid. The three distinct bands were well-visualized after staining.

The bands corresponding to the 40kDa A2M fragments were excised and stored at -20°C until analysis at the IBS facility (Grenoble, France).

Amino acid sequencing was carried out following the Edman degradation method and using a gas-phase sequencer (Applied Biosystems, model 492). The phenylthiohydantoin amino acid derivatives generated during each sequence cycle were identified on-line with an Applied Biosystems Model 140C HPLC system using the Applied Biosystems 610A protein sequencer data analyzer (software version 2.1). Procedures and reagents were as recommended by the manufacturer.

### Statistical analysis

Variables were summarized as median values (± standard errors) and compared using the Wilcoxon test or one-way analysis of variance (ANOVA). The *p values* were calculated using the Progenesis Samespot software and the KaleidaGraph 4.0 software.

### Results

### 2-DE determination of A2M fragments in serum from HCV infected patients

Serum samples from 19 HCV-infected patients with F1 and F2 liver fibrosis were pre-fractionated to specifically deplete two high-abundance proteins (albumin and IgG) (see Fig. 1A for a schematic description of the experimental procedure).

A sample from a patient with F2 fibrosis was used to optimize the 2-DE separation of serum protein. Briefly, the depleted sample was first separated using wide range IPG strips (pH 3-10) and the A2M fragments were visualized by western blot analysis using a polyclonal anti-A2M antibody. The full length monomeric A2M form (180kDa), the thioester-cleaved N- and C-terminal A2M fragments (about 110 and 56kDa respectively) and the bait region-cleaved N- and C-terminal A2M fragments (about 75 and 85kDa respectively) were clearly detected (Fig. 1 B). Furthermore, new A2M fragments of about 40kDa were detected as several distinct spots.

Then, the 2-DE separation of human serum A2M was optimized by using narrow range IPG strips (pH 5.5-6.7) to be closer to the isoelectric point (pl) of A2M (5.98) in order to better investigate these new 40kDa A2M fragments. More resolved protein spots corresponding to the A2M monomer and to all the previously described fragments could be observed (Fig. 1 B). More particularly, 18 spots in the region of the 40kDa A2M fragments were detected (Fig. 1 B; Fig. 2A).

To the best of our knowledge, the A2M fragments of about 40 kDa are novel A2M fragments.

They are differently abundant in F1 and F2 patients, *i.e.,* they are present at a significantly higher level in the F2 patients compared to the F1 patients.

Please note that the thioester-cleaved C-terminal A2M fragment of about 56 kDa, from which said new 40 kDa fragments may derive by cleavage, is not differently abundant in the F1 and F2 patients: at such early fibrosis stages, the 56kDa fragment is not discriminative, contrary to the 40 kDa fragments.

### 2-DE and 1-DE differential analysis of the 40kDa A2M fragments in serum samples from HCV-infected patients with F1 and F2 liver fibrosis

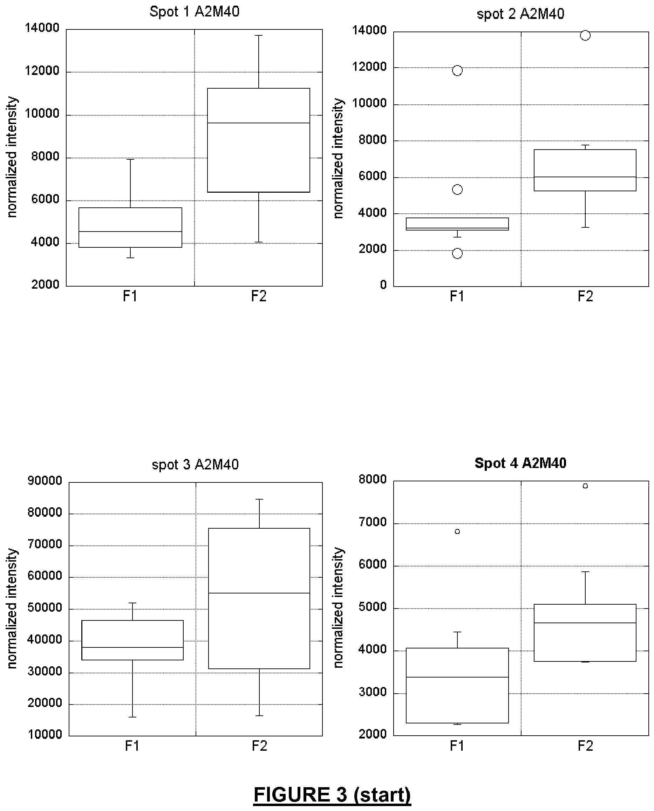

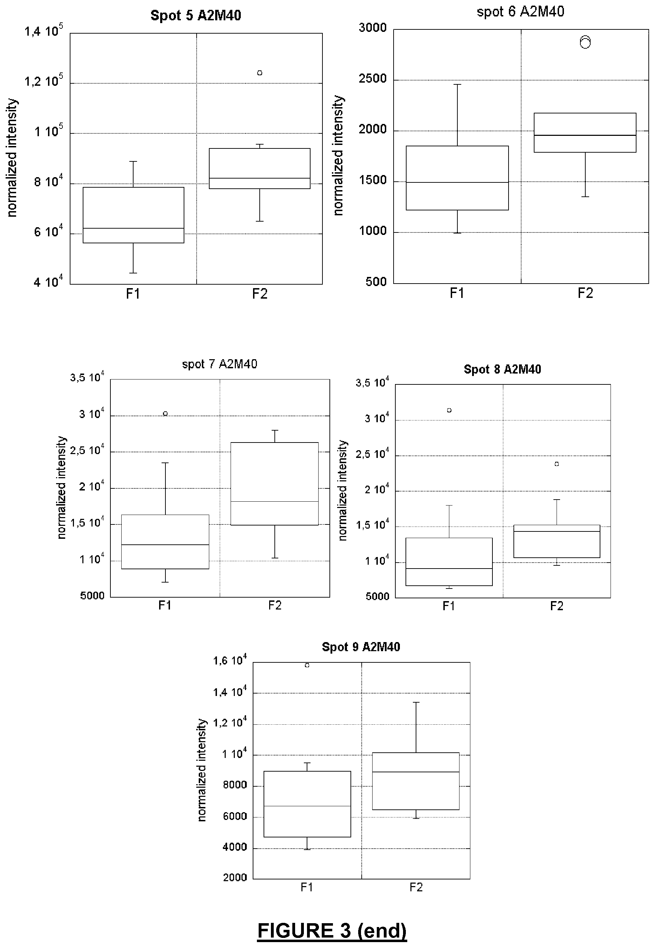

To analyze the diagnostic potential of the new A2M fragments found in the 40kDa region to differentiate HCV-infected patients with mild (F1 patients) from those with moderate fibrosis (F2 patients), a 2-DE differential analysis was performed using the 19 depleted serum samples from F1 (n=9) and F2 (n=10) patients and narrow range IPG strips (pH range 5.5-6.7) (one gel for each patient). The different A2M fragments were immunodetected with the polyclonal anti-A2M antibody. Nine of the 18 A2M spots in the 40kDa region were found to be significantly more intense in the samples from F2 patients (Fig. 2A and 2B). The intensity of the nine spots showed a fold change > 1.30 between F1 and F2 samples and five of them had a *p-value <* 0.05 (Table 2 and Fig. 3).

**Table 2. 2-DE spot and 1-DE band intensity in samples from HCV-positive patients with F1 and F2 liver fibrosis (Metavir fibrosis stages). Fold changes are expressed as medians (range).**

| A2M 40kDa | F1 (n=9) vs F2 (n=10) | |
|---|---|---|
| | *p-value* | Fold Change |
| 2-DE spot 1 | 0.003 | 2.11 |
| 2-DE spot 2 | 0.022 | 1.87 |
| 2-DE spot 3 | 0.198 | 1.44 |
| 2-DE spot 4 | 0.038 | 1.38 |
| 2-DE spot 5 | 0.015 | 1.28 |
| 2-DE spot 6 | 0.038 | 1.31 |
| 2-DE spot 7 | 0.108 | 1.48 |
| 2-DE spot 8 | 0.159 | 1.57 |
| 2-DE spot 9 | 0.173 | 1.33 |
| 2-DE 9-spots | < 0.05 | 1.55 |
| 1-DE 3 bands | < 0.05 | 1.89 |

The region in which the nine spots were localized presented a fold change of 1.55 and a *p-value <* 0.05. The other nine A2M spots identified in the 40kDa region (Fig. 2A) were not differentially abundant in serum samples from F1 and F2 patients and they were also not the strongest spots of this region. Overall, the region including all the 18 spots corresponding to the A2M 40kDa fragments presented a fold change of 1.26 between gels from F1 and F2 patients. By analyzing two more sera from HCV infected patients with F3 and F4 stages of fibrosis, we found that the A2M fragments in the 40kDa region were more concentrated than in sera from F2 and F1 patients (Fig. 2B).

The depleted serum samples from F1 and F2 patients were also used to perform a 1-DE differential analysis. Immuno-detection of the different A2M fragments by the polyclonal anti-A2M antibody visualized two major and one minor distinct 40kDa bands that were differentially abundant in F1 and F2 patients with a fold change of 1.89 and a *p-value <* 0.05 (Fig. 2C and Table 2).

Finally, the possible correlations between gender, age and total A2M concentration (Table 1) and the signal intensity of the A2M 40kDa fragments were investigated. Gender, age > or < 45 years did not show any significant correlation with the signal intensity of the nine significant spots corresponding to A2M 40kDa fragments (all *p-values* > 0.05).

However, total A2M concentration (> or < 2.97mg/mL, median concentration of total A2M in F1 and F2 patients) was correlated to the signal intensity for the spots 1, 7, 8 and 9 with respectively, *p-values* of 0.013, < 0.001, 0.035 and 0.017. The spots 7, 8 and 9 didn't show significant *p-values* in the A2M 40kDa fragment analysis but only significant fold changes (Table 2). The strongest spots in the 40kDa A2M region (spots 1 to 6) were differentially abundant in HCV-positive patients with F1 and F2 liver fibrosis with high fold change and significant *p-value* (< 0.05).

### Determination of the N-Terminal or C-terminal localization of the A2M 40kDa fragments

In order to determine the localization of the A2M 40kDa fragments in the A2M protein sequence, two antibodies directed against A2M epitopes localized respectively in the C-and N-terminal regions of the protein were used. First, the epitopes of these antibodies were determined using the epitope mapping technique. The epitope of the anti-human C-terminal A2M rabbit polyclonal antibody (Abcam) corresponded to amino acids 1,376 - 1,410 and the epitope of the anti-human N-terminal A2M mouse monoclonal antibody (Abnova) to amino acids 662 - 679 (*cf*. Figures 5A and 5B). Then, the two anti-A2M antibodies were used to assess the amount of the different A2M fragments by western blotting in a 2-DE gel of a serum sample from a HCV-infected patient with F2 liver fibrosis (Fig. 4A). The C-terminal antibody detected the A2M 40kDa fragments, whereas the N-terminal antibody did not. These results show that 40kDa fragments are located in the C-terminal domain of human A2M. As expected, the already characterized A2M N-terminal and C-terminal fragments (*i.e.,* the 105kDa N-ter thioester-cleaved and the 75kDa N-ter bait-cleaved fragments; the 56kDa C-ter thioester-cleaved and the 85kDa C-ter bait-cleaved fragments) were detected by antibodies in accordance with the epitope exposed (Fig.4A and 4B).

### Determination of the cleavage sites generating the A2M 40kDa fragments

To characterize the A2M 40kDa fragments and determine the cleavage sites, which may generate the corresponding fragments, a LC-MS/MS approach was first employed. After trypsin digestion (cleavage after R or L) of the 2-DE gel section corresponding to the region containing the A2M 40kDa fragments, the digested peptides were identified and their amino acid sequences obtained. The identified peptides selected based on their high frequency of appearance confirm a C-terminal localization of the A2M 40kDa fragments (Fig. 4C). Three different peptides were identified (Table 3) that had different starting positions and that were likely to correspond to the N-terminus of the A2M 40kDa fragments. These peptides [1,083-SGSLLNNAIK (SEQ ID NO: 7); 1,084-GSLLNNAIK (SEQ ID NO: 5); and 1,085-SLLNNAIK (SEQ ID NO: 3)] were localized in the C-terminal region of human A2M, after the bait region and the thioester bond cleavage sites (Fig. 4B and 4C). They were selected based on their high frequency of appearance in HCT1, HCT2 and Maxis analyses (n=3). A second digestion of the peptides using the Glu protease (cleavage after D or E) confirmed the biologic origin of the three previously identified peptides (Table 3).

Finally, the N-terminal sequence of the A2M 40kDa fragments was obtained by running 100 µg of A2M purified from human plasma on a 1-DE gel and then transferring it onto a PVDF membrane (*cf*. Figure 6). The well-visualized bands corresponding to the A2M 40kDa fragments were excised and their N-terminal sequences were determined. N-terminal sequencing confirmed the presence of the 1,085-SLLNNAIK peptide and also identified a new one [1,098-EVTLSAYITI (SEQ ID NO: 1)] (Table 3).

The 1,085 and 1,098 N-termini were confirmed by N-terminal sequencing. It was observed that the fragments starting at amino acid 1,085 or 1,098 are the most abundant of the four fragments. The fact that the 1,098 N-terminus was not found in the LC-MSMS experience certainly is likely due to the fact that this fragment is not well ionized, and therefore not detected using the mass spectrometry approach.

Moreover, the A2M Expasy tool (Gasteiger *et al.,* 2005; software available from http://www.expasy.org/tools/protparam.html) calculated:
- the theoretical length (390 amino acids for the 1,085-A2M 40kDa fragment and 377 amino acids for the 1,098-A2M 40kDa fragment),
- the molecular weight (42.9 kDa and 41.5 kDa), and
- the isoelectric point (6.02 and 6.18)
of the two A2M 40kDa fragments starting at positions 1,085 and 1,098.

These values were in agreement with the results obtained by 2-DE and 1-DE analysis.

**Table 3. Identification of the A2M-40 kDa fragment cleavage sites by LC-MS/MS and N-terminal sequencing. The cleavage site sequences were identified using the data gathered from three independent experiments and using different apparatuses (HCT, High Capacity Trap ion mass spectrometer; ND, non determined; X, Mascot identification in UniProtKB/Swiss-Prot with p < 0.05; x, manual validation)**

| N Terminus A2M-40kDa | LC - MS/MS | | | | | N-terminal sequencing |
|---|---|---|---|---|---|---|
| | Peptides identified after enzymatic digestion | Trypsin | | EndoGlu-V8 | | |
| | | HCT | Maxis | HCT | Maxis | |
| 1,085-SLLNNAIK (SEQ ID NO: 2) | SLLNNAIK / SLLNNAIKGGVEDE (SEQ ID NO: 2) / (SEQ ID NO: 3) | X | X | X | X | 1,085 - SLLNNAIKXG **(SEQ ID NO: 16)** |
| 1,084 - GSLLNNAIK (SEQ ID NO: 5) | GSLLNNAIK / GSLLNNAIKGGVEDE (SEQ ID NO: 5) / (SEQ ID NO: 6) | X | X | X | X | ND |
| 1,083 - SGSLLNNAIK (SEQ ID NO: 7) | SGSLLNNAIK / SGSLLNNAIKGGVEDE (SEQ ID NO: 7) / (SEQ ID NO: 8) | X | X | ND | X | ND |
| 1,098 - EVTLSAYITI (SEQ ID NO: 1) | ND | ND | ND | ND | ND | 1,098 - EVTLSAYITI (SEQ ID NO: 1) |

### Discussion

We have identified fragments of human A2M of about 40kDa.

We demonstrate that these fragments are biomarkers of hepatic fibrosis, more particularly biomarkers of the early stages of hepatic fibrosis. A particularly advantageous feature is that they are biomarkers, which differentiate no/mild fibrosis (Metavir score of at most F1) from moderate/advanced/severe fibrosis (Metavir score of F2 or more). We found that the strongest spots in the 40kDa A2M region were differentially abundant in HCV-positive patients with F1 and F2 liver fibrosis with high fold change and significant p-*value* (< 0.05).

We found that, within the full length A2M protein, these new A2M fragments are localized in the C-terminal part of the protein sequence. We determined that the N-termini of these fragments correspond to amino acids 1,083, 1,084, 1,085 and 1,098 of full length A2M, respectively, the most abundant fragments being the 1,085- and 1,098-starting fragments.

Although human A2M is a well-known protein, it is believed that these new 40kDa fragments localized in the C-terminal region of A2M have never been described before.

Human A2M monomer or thioester fragments have been reported in several proteomics studies, but, to the best of our knowledge, neither the 40kDa fragments we have identified, nor any biological sub-fragment from the 56kDa thioester-cleaved A2M fragment (*i.e.,* any sub-fragment from said 56kDa thioester-cleaved A2M fragment, which would have been observed to be naturally-occurring in the blood of a HCV-infected patient) have never been described before.

For example, when a *posteriori* analyzing the previously available data, it can be observed that, in a study on the primary structure of human A2M, Kristensen and coworkers described a 40kDa fragment resulting from chemical cleavages with CNBr [Kristensen *et al.* 1984]. This 40kDa fragment consisted of two CNBr fragments (CB21 and CB22) connected by an uncleaved Met-Thr bond and was located in the C-terminal part of A2M (CB21, residues 955-1,185 and CB22, residues 1,186-1,291). The calculated theoretical isoelectric point of the 40kDa CNBr fragment was 7.85. Therefore it is too basic to correspond to the new 40kDa biological fragments we identified. Furthermore, the N-terminal sequences do not correspond.

Furthermore, to the best of our knowledge, it has never been described before that the 40kDa A2M fragments are markers of hepatic fibrosis, more particularly markers of the early stages of hepatic fibrosis, still more particularly markers, which differentiate no/mild fibrosis (Metavir score of at most F1) from moderate/advanced/severe fibrosis (Metavir score of F2 or more).

A2M serum concentrations were found to increase during hepatic fibrosis development [Naveau *et al.* 1994; Donnelly *et al.* 1983; Hiramatsu *et al.* 1976] and many non-invasive diagnostic tests have already included A2M as an independent predictor of the extent of fibrosis [Imbert-Bismut *et al.* 2001; Poynard *et al.* 2002; Patel *et al.* 2004; Cales *et al.* 2005].

For example, A2M total serum concentration has already been described to be fibrosis biomarker, but:
- the A2M total concentration is the measure of several of the different A2M forms that are present in the patient's serum, without discriminating between these different forms: the A2M total concentration is the concentration of at least two elements from the group comprising the full length A2M monomer, the N-terminal and C-terminal thioester-cleaved A2M fragments, the N-terminal and C-terminal bait-cleaved A2M fragments; for example, when an anti-N-terminal A2M antibody has been used, the total A2M concentration is in fact the concentration of the A2M monomer + the N-terminal bait-cleaved A2M fragment of about 75 kDa + the N-terminal thioester-cleaved A2M fragment of about 105 kDa, and
- the total A2M concentration has been described to be relevant for discriminating mild/moderate fibrosis (Metavir score of F1 or F2) from advanced/severe fibrosis (Metavir score of F3 or F4) [Imbert-Bismut *et al.* 2001; Poynard *et al.* 2002; Patel *et al.* 2004; Cales *et al.* 2005].

By comparison, it can be observed that:
- our data do not relate to the A2M monomer, the bait-cleaved A2M fragments, the thioester-cleaved A2M fragments, but specifically relate to a particular group of A2M forms, *i.e.,* the A2M fragments of about 40 kDa, and
- our data demonstrate that these particular A2M fragments of about 40 kDa are present at a significantly higher level in the F2 patients compared to the F1 patients (*i.e.,* our data's threshold is F2 *versus* F1, whereas, with the total A2M concentration, the threshold is F3 *versus* F2).

Some proteomic studies have confirmed the diagnostic potential of the A2M monomer (about 180kDa) [Cheung *et al.* 2009; Ho *et al.* 2010].

Gangadharan and coworkers have shown that A2M thioester cleavage products are increased in cirrhotic patients *(i.e.,* Metavir score of F4) and may give a more reliable indication of hepatic lesions [Gangadharan *et al.* 2007].

However, the thioester-cleaved C-terminal A2M fragment of about 56 kDa, from which said 40 kDa fragments could be considered to derive by cleavage, have not described to be a marker of hepatic fibrosis, and our data demonstrate that the F2 *versus* F1 differential abundance, which is observed for the A2M fragments of about 40 kDa, is not observed with the thioester-cleaved C-terminal A2M fragment of about 56 kDa.

Since our data indicate that the A2M 40kDa fragments can be used to differentiate between F1 and F2 fibrosis, monoclonal antibodies against these A2M 40kDa fragments can be developed. Such antibodies can be used to measure the concentrations of these new fragments in the serum of HCV-infected patients. Such antibodies can be used in a larger cohort of HCV-infected patients and/or with a cohort comprising more F3 and F4 patients and/or comprising F0 patients, to further illustrate the diagnostic potential of our 40kDa A2M fragments.

Monomeric, homodimeric and homotetrameric members of the A2M family are widely distributed throughout the human species and share the ability to inhibit proteases, displaying different specificities and catalytic mechanisms. Several cell types including fibroblasts, macrophages, hepatocytes and dermal dendritic cells express a receptor for the A2M - protease complex for rapid internalization and intracellular degradation of the complex, whereas the receptor is recycled.

The new A2M 40kDa biological fragments we identified may correspond to the A2M-proteinase complex binding domain that is generated by autolytic cleavage or nucleophilic reaction before complex internalization into the cell. Alternatively, the A2M 40kDa fragments could be a degradation product of the 56kDa C-terminal thioester fragment (Fig. 4B).

### EXAMPLE 2: examples of values of MW and of isoelectric point (pl)

**Table 6:**

| **A2M40 peptides** | | number of amino acids | MW (kDa) |
|---|---|---|---|
| **start** | **stop** | | |
| **1081** | **1474** | 394 | 43.25 |
| **1083** | **1474** | 392 | 43.01 |
| **1084** | **1474** | 391 | 42.92 |
| **1085** | **1474** | 390 | 42.86 |
| **1098** | **1474** | 377 | 41.55 |
| **1123** | **1474** | 352 | 38.82 |
| **1081** | **1455** | 375 | 41.24 |
| **1083** | **1455** | 373 | 41,00 |
| **1084** | **1455** | 372 | 40.91 |
| **1085** | **1455** | 371 | 40.85 |
| **1098** | **1455** | 358 | 39.54 |
| **1123** | **1455** | 333 | 36.81 |

**Table 7:**

| A2M40 peptides | | number of amino acids | pl |
|---|---|---|---|
| start | stop | | |
| 1081 | 1474 | 394 | 6.18 |
| 1083 | 1474 | 392 | 6.02 |
| 1084 | 1474 | 391 | 6.03 |
| 1085 | 1474 | 390 | 6.02 |
| 1098 | 1474 | 377 | 6.18 |
| 1123 | 1474 | 352 | 6.28 |
| 1081 | 1455 | 375 | 6.83 |
| 1083 | 1455 | 373 | 6.51 |
| 1084 | 1455 | 372 | 6.55 |
| 1085 | 1455 | 371 | 6.51 |
| 1098 | 1455 | 358 | 6.85 |
| 1123 | 1455 | 333 | 7.23 |

Start and stop positions are computed with respect to the full length human A2M sequence of SEQ ID NO: 9 (NM_000014).

### EXAMPLE 3: example of antibody production

CD-1 mice were immunized at 30µg/injection with peptide 1,085-1,092 (SLLNNAIK; SEQ ID NO: 2) or peptide 1,098-1,104 (EVTLSAY; **SEQ ID NO: 17**) linked to a carrier protein (-Hx-GSGC-CONH2 for peptide 1,085-1,092; RSGRS-Diox-C-CONH2 for peptide 1,098-1,104) emulsified in adjuvant until antibody titer in sera wass strong enough to perform a fusion. For each immunogen peptide, fusion was done between SP2/0 myeloma and splenocytes from immunized mouse. Hybridoma supernatants were screened through a funnel-shaped screening for antibodies capable of binding to recombinant or purified A2M-40Kd and without binding to others A2M fragments. Screening format were ELISA, sandwich ELISA, western blot (or any other relevant formats). Primary screening post-fusion corresponding to 20 seeded 96 wells-plates wass done by ELISA on A2M-40Kd and counter-screening wass done on full length A2M (FL-A2M). Up to 96 clones positive for A2M-40Kd and negative on FL-A2M were selected. Confirmation of the 96 selected hybridomas was performed by ELISA on A2M-40Kd and on immunogen peptide; counter-screening was done on FL-A2M and irrelevant peptides with Nter different from immunogen peptide. Up to 48 hybridoma positive on A2M-40Kd and immunogen peptide, and negative on FL-A2M were selected. These 48 hybridomas were frozen and constituted the mother clones of the fusion. These clones were positive for the N-ter part of the A2M-40Kd. Next step was the secondary screenings to choose up to 20 clones to be sub-cloned starting from the 48 mother hybridomas; screening formats were the following:
- sandwich ELISA to identify antibodies that recognize free protein: anti-Cter A2M in capture/ A2M-40Kd/ hybridoma/ anti-mouse IgG-POD,
- western-blot with native purified A2M (source Sigma that contains all forms of A2M including A2M-40Kd) to identify antibodies that recognize native form,
- epitope mapping to select only clones targeting Nter sequence of A2M-40Kd.

For the epitope mapping of the antibodies produced with peptide 1,085-1,092:

| | |
|---|---|
| SLLNNAIK | (immunogen; SEQ ID NO: 2) |
| SSGSLLNNAIKGG | (control other S in terminal position; **SEQ ID NO: 18**) |
| SGSLLNNAIKGG | (control other S in terminal position; **SEQ ID NO: 19**) |
| GSLLNNAIKGG | (control S85 non terminal; **SEQ ID NO: 20**) |
| SLLNNAIKGG | (targeted sequence; **SEQ ID NO: 21**) |
| LLNNAIKGG | (control without terminal S; **SEQ ID NO: 22**) |
| ALLNNAIKGG | (control without terminal S; **SEQ ID NO: 23**) |
| SALNNAIKGG | (Alascan to identify other potential essential aa; **SEQ ID NO: 24**) |
| SLANNAIKGG | (Alascan; **SEQ ID NO: 25**) |
| SLLANAIKGG | (Alascan; **SEQ ID NO: 26**) |

For the epitope mapping of the antibodies produced with peptide 1,098-1,104:

| | |
|---|---|
| EVTLSAY | (immunogen; SEQ ID NO: 17) |
| EDEVTLSAYITI | (control other E in terminal position; **SEQ ID NO: 27**) |
| DEVTLSAYITI | (control E98 non terminal; **SEQ ID NO: 28**) |
| EVTLSAYITI | (targeted sequence; **SEQ ID NO: 29**) |
| VTLSAYITI | (control without terminal E; **SEQ ID NO: 30**) |
| AVTLSAYITI | (control without terminal E; **SEQ ID NO: 31**) |
| EATLSAYITI | (Alascan to identify other potential essential aa; **SEQ ID NO: 32** |
| EVALSAYITI | (Alascan; **SEQ ID NO: 33**) |
| EVTASAYITI | (Alascan; **SEQ ID NO: 34**) |
| EVTLAAYITI | (Alascan; **SEQ ID NO: 35**) |

Up to the 20 best clones were then sub-cloned and screened in ELISA, sELISA and WB.

Then the 10 best sub-clones 1 were then sub-cloned again to ensure monoclonality. Screening of sub-clones 2 was done by ELISA and sELISA; the ten final best sub-clones-2 were then produced and purified.

### BIBLIOGRAPHIC REFERENCES

Ahmad W, Ijaz B, Gull S, Asad S, Khaliq S, Jahan S, Sarwar MT, Kausar H, Sumrin A, Shahid I, Hassan S, 2011. A brief review on molecular, genetic and imaging techniques for HCV fibrosis evaluation. Virol J 8: 53.
Harlow E and Lane E, 1988. Antibodies A Laboratory Manual. Cold Spring Harbor Laboratory press, Cold Spring Harbor, New York, U.S.A.
Cales P, Oberti F, Michalak S, Hubert-Fouchard I, Rousselet MC, Konate A, et al. A novel panel of blood markers to assess the degree of liver fibrosis. Hepatology. 2005;42:1373-81.
Cales P, Boursier J, Oberti F, Hubert I, Gallois Y, Pousselet MC, Dib N, Moal V, Macchi L, Chevailler A, Michalak S, Hunault G, Chaigneau J, Sawadogo A and Lunel F, 2008. FibroMeters: a family of blood tests for liver fibrosis. Gastroentérol Clinique et Biologique 32: 40-51.
Cales P, Boursier J, Bertrais S, Oberti F, Gallois Y, Fouchard-Hubert I, Dib N, Zarski JP and Rousselet MC, 2010. Optimization and robustness of blood tests for liver fibrosis and cirrhosis. Clin Biochem 43: 1315-1322.
Cheung KJ, Tilleman K, Deforce D, Colle I, Van Vlierberghe H. The HCV serum proteome: a search for fibrosis protein markers. J Viral Hepat. 2009;16:418-29.
Cole et al. 1983. Proc. Natl. Acad. Sci. USA 80:2026-2030
Cole et al. 1985. Monoclonal Antibodies And Cancer Therapy, Alan R. Liss, Inc., pp. 77-96
Donnelly PK, Shenton BK, Alomran A, Francis DM, Proud G, Taylor RM. The clinical relevance of alpha 2-macroglobulin. Ann N Y Acad Sci. 1983;421:382-7.
Frank R. Spot-Synthesis: an easy technique for the positionally addressable, parallel chemical synthesis on a membrane support. Tetrahedron. 1992;48:9217-32.
Friedman SL. Evaluation of fibrosis and hepatitis C. Am J Med. 1999;107:27S-30S.
Gangadharan B, Antrobus R, Dwek RA, Zitzmann N. Novel serum biomarker candidates for liver fibrosis in hepatitis C patients. Clin Chem. 2007;53:1792-9.
Gasteiger E., Hoogland C., Gattiker A., Duvaud S., Wilkins M.R., Appel R.D., Bairoch A. Protein Identification and Analysis Tools on the ExPASy Server; (In) John M. Walker (ed): The Proteomics Protocols Handbook, Humana Press, 2005; pp. 571-607.
Guechot J, Lasnier E, Sturm N, Paris A, Zarski JP, 2010. Automation of the Hepascore and validation as a biochemical index of liver fibrosis in patients with chronic hepatitis C from the ANRS HC EP 23 Fibrostar cohort. Clin Chim Acta 411: 86-91.
Hiramatsu S, Kojima J, Okada TT, Inai S, Ohmori K. The serum protein profile in chronic hepatitis, cirrhosis and liver cancer. Acta Hepatogastroenterol (Stuttg). 1976;23:177-82.
Ho AS, Cheng CC, Lee SC, Liu ML, Lee JY, Wang WM, et al. Novel biomarkers predict liver fibrosis in hepatitis C patients: alpha 2 macroglobulin, vitamin D binding protein and apolipoprotein Al. J Biomed Sci. 2010;17:58.
Imbert-Bismut F, Ratziu V, Pieroni L, Charlotte F, Benhamou Y, Poynard T., 2001. Biochemical markers of liver fibrosis in patients with hepatitis C virus infection: a prospective study. Lancet 357: 1069-75.
Köhler and Milstein 1975. Nature 256: 495-497.
Kosbor et al. 1983. Immunology Today 4: 72.
Kristensen T, Wierzbicki DM, Sottrup-Jensen L. Primary structure of human alpha 2-macroglobulin. IV. Primary structure of two large CNBr fragments, located in the COOH-terminal part and accounting for 337 residues. J Biol Chem. 1984;259:8313-7.
Molina F, Laune D, Gougat C, Pau B, Granier C. Improved performances of spot multiple peptide synthesis. Pept Res. 1996;9:151-5.
Naveau S, Poynard T, Benatar C, Bedossa P, Chaput JC, 1994. Alpha-2-macroglobulin and hepatic fibrosis. Diagnostic interest. Dig Dis Sci 39(11): 2426-2432.
Needleman and Wunsch 1970. Journal of Molecular Biology 48: 443-453.
Patel K, Gordon SC, Jacobson I, Hezode C, Oh E, Smith KM, et al. Evaluation of a panel of non-invasive serum markers to differentiate mild from moderate-to-advanced liver fibrosis in chronic hepatitis C patients. J Hepatol. 2004;41:935-42.
Poynard T, Imbert-Bismut F, Ratziu V, Chevret S, Jardel C, Moussalli J, et al. Biochemical markers of liver fibrosis in patients infected by hepatitis C virus: longitudinal validation in a randomized trial. J Viral Hepat. 2002;9:128-33.
Poynard T et al., 2004. Overview of diagnostic value of biochemical markers of liver fibrosis (FibroTest, HCV FibroSure) and necrosis (ActiTest) in patients with chronic hepatitis C. Comparative Hepatology 3: 8.
Shaheen AA, Wan AF, Myers RP, 2007. FibroTest and FibroScan for the prediction of hepatitis C-related fibrosis: a systematic review of diagnostic test accuracy. Am J Gastroenterol 102(11): 2589-2600.
U.S. Pat. No. 4,376,110
Zaman A, Rosen HR, Ingram K, Corless CL, Oh E and Smith K, 2007. Assessment of FIBROSpect II to detect hepatic fibrosis in chronic hepatitic C patients. Am J Med 120: 280, e289-214.

## Claims

1. A polypeptide, the amino acid sequence of which is the sequence of a sub-fragment of the C-terminal thioester-cleaved fragment of human alpha-2-macroglobulin (A2M), wherein the molecular weight of said polypeptide is of 36 to 44 kDa, and wherein:
- the first N-terminal amino acid of said polypeptide is an amino acid, which, in the full length sequence of said human A2M, is at a position 1,098 or 1,085 or 1,084 or 1,083, and
- the last C-terminal amino acid of said polypeptide is an amino acid, which, in the full length sequence of said human A2M, is one of the last twenty C-terminal amino acids.

2. The polypeptide of claim 1, wherein said full length A2M amino acid sequence consists of 1,474 amino acids.

3. The polypeptide of any one of claims 1-2, wherein the site of said thioester cleavage of said human A2M is at positions 972-975 and/or wherein said C-terminal thioester-cleaved fragment of said human A2M is of 56 kDa.

4. The polypeptide of any one of claims 1-3, wherein said full length sequence of said human A2M is the sequence of SEQ ID NO: 9, or a sequence which differs from the sequence of SEQ ID NO: 9 by at most five amino acid substitutions.

5. The polypeptide of any one of claims 1-4, wherein said first N-terminal amino acid of said fragment is an amino acid, which, in the full length sequence of said A2M, is at position 1,098 or 1,085.

6. The polypeptide of any one of claims 1-5, the amino acid sequence of which is SEQ ID NO: 12, SEQ ID NO: 13, SEQ ID NO: 14 or SEQ ID NO: 15.

7. The polypeptide of any one of claims 1-6, which is of 39 to 43.5 kDa.

8. A peptide, the sequence of which is one of SEQ ID NOs: 1-8, 17.

9. A compound consisting of:
- the polypeptide of any one of claims 1-7 or the peptide of claim 8, and of
- one or several other structural components, which are linked to said polypeptide or peptide, wherein said other structural components are selected from the group consisting of His tags, BSA, KLH.

10. A coding nucleic acid, the sequence of which consists of a sequence coding for the polypeptide of any one of claims 1-7, or for the peptide of claim 8, or for the compound of claim 9.

11. A vector, which comprises a coding nucleic acid inserted therein, wherein the expression product of said coding nucleic acid is the polypeptide of any one of claims 1-7, or the peptide of claim 8, or the compound of claim 9.

12. A genetically engineered host cell, which comprises a coding nucleic acid consisting of the nucleic acid of claim 10 and/or which comprises the vector of claim 11.

13. *In vitro* use of an antibody or antibody fragment or of a kit comprising said antibody or antibody fragment, for detecting hepatitis and/or liver fibrosis, wherein said antibody or antibody fragment specifically binds to the N-terminal extremity of the polypeptide of any one of claims 1-7 or of the peptide of claim 8, without binding to the full length human A2M protein, and wherein said antibody or antibody fragment does not bind to the C-terminal thioester-cleaved fragment of said human A2M, and does not bind to the C-terminal bait-cleaved fragment of said human A2M.

14. The *in vitro* use of claim 13, wherein said antibody or antibody fragment specifically binds to the N-terminal extremity of the polypeptide of any one of claims 1-7 or of the peptide of claim 8 at a site, which comprises the non-covalently linked N-terminal alpha-amino group of said polypeptide or peptide.

15. The *in vitro* use of claim 13 or 14, wherein said kit further comprises at least one means selected from:
- at least one syringe, and/or
- at least one tube, and/or
- at least one resin having a selective binding affinity for albumin and/or immunoglobulin G, and/or
- at least one protein denaturant.

16. *In vitro* use of an antibody or antibody fragment or of a kit comprising said antibody or antibody fragment, for scoring a liver fibrosis stage and/or for determining whether a liver fibrosis is at a stage, which scores at most F1 or at a stage, which scores at least F2, in accordance with the Metavir scoring system, wherein said antibody or antibody fragment specifically binds to the N-terminal extremity of the polypeptide of any one of claims 1-7 or of the peptide of claim 8, without binding to the full length human A2M protein, and wherein said antibody or antibody fragment does not bind to the C-terminal thioester-cleaved fragment of said human A2M, and does not bind to the C-terminal bait-cleaved fragment of said human A2M.

17. The *in vitro* use of claim 16, wherein said antibody or antibody fragment specifically binds to the N-terminal extremity of the polypeptide of any one of claims 1-7 or of the peptide of claim 8 at a site, which comprises the non-covalently linked N-terminal alpha-amino group of said polypeptide or peptide.

18. The *in vitro* use of claim 16 or 17, wherein said kit further comprises at least one means selected from:
- at least one syringe, and/or
- at least one tube, and/or
- at least one resin having a selective binding affinity for albumin and/or immunoglobulin G, and/or
- at least one protein denaturant.

19. The *in vitro* use of the peptide of claim 8 for *in vitro* screening antibodies or antibody fragments.

## Patentansprüche

1. Polypeptid, dessen Aminosäuresequenz die Sequenz eines Unterfragments des C-terminalen Thioester-gespaltenen Fragments von humanem alpha-2-Makroglobulin (A2M) ist, wobei das Molekulargewicht des Polypeptids von 36 bis 44 kDa beträgt, und wobei:
- die erste N-terminale Aminosäure des Polypeptids eine Aminosäure ist, die sich in der Sequenz voller Länge des humanen A2M an einer Position 1098 oder 1085 oder 1084 oder 1083 befindet, und
- die letzte C-terminale Aminosäure des Polypeptids eine Aminosäure ist, die in der Sequenz voller Länge des humanen A2M eine der letzten zwanzig C-terminalen Aminosäuren ist.

2. Polypeptid nach Anspruch 1, wobei die A2M-Aminosäuresequenz voller Länge aus 1474 Aminosäuren besteht.

3. Polypeptid nach einem beliebigen der Ansprüche 1-2, wobei die Stelle der Thioesterspaltung des humanen A2M sich an Positionen 972-975 befindet, und/oder wobei das C-terminale Thioester-gespaltene Fragment des humanen A2M von 56 kDa ist.

4. Polypeptid nach einem beliebigen der Ansprüche 1-3, wobei die Sequenz voller Länge des humanen A2M die Sequenz von SEQ ID NO: 9 ist, oder eine Sequenz, die sich von der Sequenz von SEQ ID NO: 9 durch höchstens fünf Aminosäuresubstitutionen unterscheidet.

5. Polypeptid nach einem beliebigen der Ansprüche 1-4, wobei die erste N-terminale Aminosäure des Fragments eine Aminosäure ist, die sich in der Sequenz voller Länge des A2M an Position 1098 oder 1085 befindet.

6. Polypeptid nach einem beliebigen der Ansprüche 1-5, dessen Aminosäuresequenz SEQ ID NO: 12, SEQ ID NO: 13, SEQ ID NO: 14 oder SEQ ID NO: 15 ist.

7. Polypeptid nach einem beliebigen der Ansprüche 1-6, das von 39 bis 43,5 kDa ist.

8. Peptid, dessen Sequenz eine von SEQ ID NOs: 1-8, 17 ist.

9. Verbindung, bestehend aus:
- dem Polypeptid gemäß einem beliebigen der Ansprüche 1-7 oder dem Peptid gemäß Anspruch 8, und aus
- einem oder mehreren anderen strukturellen Bestandteilen, die mit dem Polypeptid oder Peptid verknüpft sind, wobei die anderen strukturellen Bestandteile ausgewählt sind aus der Gruppe bestehend aus His-Tags, BSA, KLH.

10. Kodierende Nukleinsäure, deren Sequenz aus einer Sequenz besteht, die für das Polypeptid gemäß einem beliebigen der Ansprüche 1-7, oder für das Peptid gemäß Anspruch 8, oder für die Verbindung gemäß Anspruch 9 kodiert.

11. Vektor, der eine kodierende Nukleinsäure darin insertiert umfasst, wobei das Expressionprodukt der kodierenden Nukleinsäure das Polypeptid gemäß einem beliebigen der Ansprüche 1-7 oder das Peptid gemäß Anspruch 8 oder die Verbindung gemäß Anspruch 9 ist.

12. Gentechnisch veränderte Wirtszelle, die eine kodierende Nukleinsäure umfasst, die aus der Nukleinsäure gemäß Anspruch 10 besteht und/oder die den Vektor gemäß Anspruch 11 umfasst.

13. *In vitro*-Verwendung eines Antikörpers oder Antikörperfragments oder eines Kits, das den Antikörper oder das Antikörperfragment umfasst, zum Nachweisen von Hepatitis und/oder Leberfibrose, wobei der Antikörper oder das Antikörperfragment an das N-terminale Ende des Polypeptids gemäß einem beliebigen der Ansprüche 1-7 oder des Peptids gemäß Anspruch 8 bindet, ohne an das humane A2M-Protein voller Länge zu binden, und wobei der Antikörper oder das Antikörperfragment nicht an das C-terminale Thioestergespaltene Fragment des humanen A2M bindet, und nicht an das C-terminale "Bait"-gespaltene Fragment des humanen A2M bindet.

14. *In-vitro*-Verwendung nach Anspruch 13, wobei der Antikörper oder das Antikörperfragment spezifisch an das N-terminale Ende des Polypeptids gemäß einem beliebigen der Ansprüche 1-7 oder des Peptids gemäß Anspruch 8 an einer Stelle bindet, die die nicht kovalent verbundene N-terminale alpha-Aminogruppe des Polypeptids oder Peptids umfasst.

15. *In-vitro*-Verwendung nach Anspruch 13 oder 14, wobei das Kit weiterhin mindestens ein Mittel umfasst ausgewählt aus:
- mindestens einer Spritze, und/oder
- mindestens einer Röhre, und/oder
- mindestens einem Harz mit selektiver Bindungsaffinität für Albumin und/oder Immunglobulin G, und/oder
- mindestens einem Proteindenaturierungsmittel.

16. *In-vitro*-Verwendung eines Antikörpers oder Antikörperfragments oder eines Kits umfassend den Antikörper oder das Antikörperfragment, zum Bewerten eines Leberfibrosestadiums und/oder zum Bestimmen, ob eine Leberfibrose sich in einem Stadium befindet, das mit höchstens F1 bewertet wird, oder in einem Stadium, das mit mindestens F2 bewertet wird, in Übereinstimmung mit dem Metavir-Bewertungssystem, wobei der Antikörper oder das Antikörperfragment spezifisch an das N-terminale Ende des Polypeptids gemäß einem beliebigen der Ansprüche 1-7 oder des Peptids gemäß Anspruch 8 bindet, ohne an das humane A2M-Protein voller Länge zu binden, und wobei der Antikörper oder das Antikörperfragment nicht an das C-terminale Thioester-gespaltene Fragment des humanen A2M bindet, und nicht an das C-terminale "Bait"-gespaltene Fragment des humanen A2M bindet.

17. *In vitro*-Verwendung nach Anspruch 16, wobei der Antikörper oder das Antikörperfragment spezifisch an das N-terminale Ende des Polypeptids gemäß einem beliebigen der Ansprüche 1-7 oder des Peptids gemäß Anspruch 8 an einer Stelle bindet, die die nicht kovalent verbundene N-terminale alpha-Aminogruppe des Polypeptids oder Peptids umfasst.

18. *In-vitro*-Verwendung nach Anspruch 16 oder 17, wobei das Kit weiterhin mindestens ein Mittel umfasst ausgewählt aus:
- mindestens einer Spritze, und/oder
- mindestens einer Röhre, und/oder
- mindestens einem Harz mit selektiver Bindungsaffinität für Albumin und/oder Immunglobulin G, und/oder
- mindestens einem Proteindenaturierungsmittel.

19. *In-vitro*-Verwendung des Peptids gemäß Anspruch 8 zum *in vitro*-Screenen von Antikörpern oder Antikörperfragmenten.

## Revendications

1. Polypeptide, dont la séquence d'acides aminés est la séquence d'un sous-fragment du fragment à thioester C-terminal clivé de l'alpha-2-macroglobuline humaine (A2M), dans lequel le poids moléculaire dudit polypeptide est de 36 à 44 kDa, et dans lequel :
- le premier acide aminé N-terminal dudit polypeptide est un acide aminé qui, dans la séquence pleine longueur de ladite A2M humaine, se trouve à une position 1 098 ou 1 085 ou 1 084 ou 1 083, et
- le dernier acide aminé C-terminal dudit polypeptide est un acide aminé qui, dans la séquence pleine longueur de ladite A2M humaine, est l'un des vingt derniers acides aminés C-terminaux.

2. Polypeptide selon la revendication 1, dans lequel ladite séquence d'acides aminés A2M pleine longueur est constituée de 1 474 acides aminés.

3. Polypeptide selon l'une quelconque des revendications 1 à 2, dans lequel le site dudit clivage de thioester de ladite A2M humaine se trouve aux positions 972 à 975 et/ou dans lequel ledit fragment à thioester C-terminal clivé de ladite A2M humaine est de 56 kDa.

4. Polypeptide selon l'une quelconque des revendications 1 à 3, dans lequel ladite séquence pleine longueur de ladite A2M humaine est la séquence de SEQ ID NO: 9, ou une séquence qui diffère de la séquence de SEQ ID NO: 9 d'au plus cinq substitutions d'acides aminés.

5. Polypeptide selon l'une quelconque des revendications 1 à 4, dans lequel ledit premier acide aminé N-terminal dudit fragment est un acide aminé, qui, dans la séquence pleine longueur de ladite A2M, se trouve à la position 1 098 ou 1 085.

6. Polypeptide selon l'une quelconque des revendications 1 à 5, dont la séquence d'acides aminés est SEQ ID NO: 12, SEQ ID NO: 13, SEQ ID NO: 14 ou SEQ ID NO: 15.

7. Polypeptide selon l'une quelconque des revendications 1 à 6, qui est de 39 à 43,5 kDa.

8. Peptide, dont la séquence est l'une des SEQ ID NOs: 1 à 8, 17.

9. Composé constitué par :
- le polypeptide selon l'une quelconque des revendications 1 à 7, ou le peptide selon la revendication 8, et par
- un ou plusieurs autres composants structurels, qui sont liés audit polypeptide ou peptide, dans lesquels lesdits autres composants structurels sont choisis dans le groupe constitué par des étiquettes His, la BSA, la KLH.

10. Acide nucléique codant, dont la séquence est constituée d'une séquence codant pour le polypeptide selon l'une quelconque des revendications 1 à 7, ou pour le peptide selon la revendication 8, ou pour le composé selon la revendication 9.

11. Vecteur, qui comprend un acide nucléique codant inséré dans celui-ci, dans lequel le produit d'expression dudit acide nucléique codant est le polypeptide selon l'une quelconque des revendications 1 à 7, ou le peptide selon la revendication 8, ou le composé selon la revendication 9.

12. Cellule hôte génétiquement modifiée, qui comprend un acide nucléique codant constitué par l'acide nucléique selon la revendication 10 et/ou qui comprend le vecteur selon la revendication 11.

13. Utilisation *in vitro* d'un anticorps ou d'un fragment d'anticorps ou d'un kit comprenant ledit anticorps ou fragment d'anticorps, pour détecter une hépatite et/ou une fibrose hépatique, dans laquelle ledit anticorps ou fragment d'anticorps se lie de manière spécifique à l'extrémité N-terminale du polypeptide selon l'une quelconque des revendications 1 à 7 ou du peptide selon la revendication 8, sans se lier à la protéine A2M humaine pleine longueur, et dans laquelle ledit anticorps ou fragment d'anticorps ne se lie pas au fragment à thioester C-terminal clivé de ladite A2M humaine, et ne se lie pas au fragment à appât C-terminal clivé de ladite A2M humaine.

14. Utilisation *in vitro* selon la revendication 13, dans laquelle ledit anticorps ou fragment d'anticorps se lie de manière spécifique à l'extrémité N-terminale du polypeptide selon l'une quelconque des revendications 1 à 7 ou du peptide selon la revendication 8 au niveau d'un site, qui comprend le groupe alpha-amino N-terminal lié de manière non covalente dudit polypeptide ou peptide.

15. Utilisation *in vitro* selon la revendication 13 ou 14, dans laquelle ledit kit comprend en outre au moins un moyen choisi parmi :
- au moins une seringue, et/ou
- au moins un tube, et/ou
- au moins une résine présentant une affinité de liaison sélective pour l'albumine et/ou une immunoglobuline G, et/ou
- au moins un dénaturant de protéine.

16. Utilisation *in vitro* d'un anticorps ou d'un fragment d'anticorps ou d'un kit comprenant ledit anticorps ou fragment d'anticorps, pour noter un stade de fibrose hépatique et/ou pour déterminer si une fibrose hépatique est à un certain stade, qui note au plus F1 ou à un stade, qui note au moins F2, conformément au système de notation de Métavir, dans laquelle ledit anticorps ou fragment d'anticorps se lie de manière spécifique à l'extrémité N-terminale du polypeptide selon l'une quelconque des revendications 1 à 7 ou du peptide de la revendication 8, sans se lier à la protéine A2M humaine pleine longueur, et dans laquelle ledit anticorps ou fragment d'anticorps ne se lie pas au fragment à thioester C-terminal clivé de ladite A2M humaine, et ne se lie pas au fragment à appât C-terminal clivé de ladite A2M humaine.

17. Utilisation *in vitro* selon la revendication 16, dans laquelle ledit anticorps ou fragment d'anticorps se lie de manière spécifique à l'extrémité N-terminale du polypeptide selon l'une quelconque des revendications 1 à 7 ou du peptide selon la revendication 8 au niveau d'un site, qui comprend le groupe alpha-amino N-terminal lié de manière non covalente dudit polypeptide ou peptide.

18. Utilisation *in vitro* selon la revendication 16 ou 17, dans laquelle ledit kit comprend en outre au moins un moyen choisi parmi :
- au moins une seringue, et/ou
- au moins un tube, et/ou
- au moins une résine présentant une affinité de liaison sélective pour l'albumine et/ou une immunoglobuline G, et/ou
- au moins un dénaturant de protéine.

19. Utilisation *in vitro* du peptide selon la revendication 8 pour le criblage *in vitro* d'anticorps ou de fragments d'anticorps.


### SEQUENCE LISTING

<110> BIO-RAD INNOVATIONS
<120> Novel A2M fragments and applications thereof
<130> B09452 - FP/BA
<160> 35
<170> PatentIn version 3.3
<210> 1
   <211> 10
   <212> PRT
   <213> Homo sapiens
<400> 1
   Glu Val Thr Leu Ser Ala Tyr Ile Thr Ile
   1               5                   10
<210> 2
   <211> 8
   <212> PRT
   <213> Homo sapiens
<400> 2
   Ser Leu Leu Asn Asn Ala Ile Lys
   1               5
<210> 3
   <211> 14
   <212> PRT
   <213> Homo sapiens
<400> 3
   Ser Leu Leu Asn Asn Ala Ile Lys Gly Gly Val Glu Asp Glu
   1               5                   10
<210> 4
   <211> 10
   <212> PRT
   <213> Homo sapiens
<400> 4
   Ser Leu Leu Asn Asn Ala Ile Lys Gly Gly
   1               5                   10
<210> 5
   <211> 9
   <212> PRT
   <213> Homo sapiens
<400> 5
   Gly Ser Leu Leu Asn Asn Ala Ile Lys
   1               5
<210> 6
   <211> 15
   <212> PRT
   <213> Homo sapiens
<400> 6
   Gly Ser Leu Leu Asn Asn Ala Ile Lys Gly Gly Val Glu Asp Glu
   1               5                   10                  15
<210> 7
   <211> 10
   <212> PRT
   <213> Homo sapiens
<400> 7
   Ser Gly Ser Leu Leu Asn Asn Ala Ile Lys
   1               5                   10
<210> 8
   <211> 16
   <212> PRT
   <213> Homo sapiens
<400> 8
   Ser Gly Ser Leu Leu Asn Asn Ala Ile Lys Gly Gly Val Glu Asp Glu
   1               5                   10                  15
<210> 9
   <211> 1474
   <212> PRT
   <213> Homo sapiens
<400> 9
   Met Gly Lys Asn Lys Leu Leu His Pro Ser Leu Val Leu Leu Leu Leu
   1               5                   10                  15
   Val Leu Leu Pro Thr Asp Ala Ser Val Ser Gly Lys Pro Gln Tyr Met
               20                  25                  30
   Val Leu Val Pro Ser Leu Leu His Thr Glu Thr Thr Glu Lys Gly Cys
           35                  40                  45
   Val Leu Leu Ser Tyr Leu Asn Glu Thr Val Thr Val Ser Ala Ser Leu
       50                  55                  60
   Glu Ser Val Arg Gly Asn Arg Ser Leu Phe Thr Asp Leu Glu Ala Glu
   65                  70                  75                  80
   Asn Asp Val Leu His Cys Val Ala Phe Ala Val Pro Lys Ser Ser Ser
                   85                  90                  95
   Asn Glu Glu Val Met Phe Leu Thr Val Gln Val Lys Gly Pro Thr Gln
               100                 105                 110
   Glu Phe Lys Lys Arg Thr Thr Val Met Val Lys Asn Glu Asp Ser Leu
           115                 120                 125
   Val Phe Val Gln Thr Asp Lys Ser Ile Tyr Lys Pro Gly Gln Thr Val
       130                 135                 140
   Lys Phe Arg Val Val Ser Met Asp Glu Asn Phe His Pro Leu Asn Glu
   145                 150                 155                 160
   Leu Ile Pro Leu Val Tyr Ile Gln Asp Pro Lys Gly Asn Arg Ile Ala
                   165                 170                 175
   Gln Trp Gln Ser Phe Gln Leu Glu Gly Gly Leu Lys Gln Phe Ser Phe
               180                 185                 190
   Pro Leu Ser Ser Glu Pro Phe Gln Gly Ser Tyr Lys Val Val Val Gln
           195                 200                 205
   Lys Lys Ser Gly Gly Arg Thr Glu His Pro Phe Thr Val Glu Glu Phe
       210                 215                 220
   Val Leu Pro Lys Phe Glu Val Gln Val Thr Val Pro Lys Ile Ile Thr
   225                 230                 235                 240
   Ile Leu Glu Glu Glu Met Asn Val Ser Val Cys Gly Leu Tyr Thr Tyr
                   245                 250                 255
   Gly Lys Pro Val Pro Gly His Val Thr Val Ser Ile Cys Arg Lys Tyr
               260                 265                 270
   Ser Asp Ala Ser Asp Cys His Gly Glu Asp Ser Gln Ala Phe Cys Glu
           275                 280                 285
   Lys Phe Ser Gly Gln Leu Asn Ser His Gly Cys Phe Tyr Gln Gln Val
       290                 295                 300
   Lys Thr Lys Val Phe Gln Leu Lys Arg Lys Glu Tyr Glu Met Lys Leu
   305                 310                 315                 320
   His Thr Glu Ala Gln Ile Gln Glu Glu Gly Thr Val Val Glu Leu Thr
                   325                 330                 335
   Gly Arg Gln Ser Ser Glu Ile Thr Arg Thr Ile Thr Lys Leu Ser Phe
               340                 345                 350
   Val Lys Val Asp Ser His Phe Arg Gln Gly Ile Pro Phe Phe Gly Gln
           355                 360                 365
   Val Arg Leu Val Asp Gly Lys Gly Val Pro Ile Pro Asn Lys Val Ile
       370                 375                 380
   Phe Ile Arg Gly Asn Glu Ala Asn Tyr Tyr Ser Asn Ala Thr Thr Asp
   385                 390                 395                 400
   Glu His Gly Leu Val Gln Phe Ser Ile Asn Thr Thr Asn Val Met Gly
                   405                 410                 415
   Thr Ser Leu Thr Val Arg Val Asn Tyr Lys Asp Arg Ser Pro Cys Tyr
               420                 425                 430
   Gly Tyr Gln Trp Val Ser Glu Glu His Glu Glu Ala His His Thr Ala
           435                 440                 445
   Tyr Leu Val Phe Ser Pro Ser Lys Ser Phe Val His Leu Glu Pro Met
       450                 455                 460
   Ser His Glu Leu Pro Cys Gly His Thr Gln Thr Val Gln Ala His Tyr
   465                 470                 475                 480
   Ile Leu Asn Gly Gly Thr Leu Leu Gly Leu Lys Lys Leu Ser Phe Tyr
                   485                 490                 495
   Tyr Leu Ile Met Ala Lys Gly Gly Ile Val Arg Thr Gly Thr His Gly
               500                 505                 510
   Leu Leu Val Lys Gln Glu Asp Met Lys Gly His Phe Ser Ile Ser Ile
           515                 520                 525
   Pro Val Lys Ser Asp Ile Ala Pro Val Ala Arg Leu Leu Ile Tyr Ala
       530                 535                 540
   Val Leu Pro Thr Gly Asp Val Ile Gly Asp Ser Ala Lys Tyr Asp Val
   545                 550                 555                 560
   Glu Asn Cys Leu Ala Asn Lys Val Asp Leu Ser Phe Ser Pro Ser Gln
                   565                 570                 575
   Ser Leu Pro Ala Ser His Ala His Leu Arg Val Thr Ala Ala Pro Gln
               580                 585                 590
   Ser Val Cys Ala Leu Arg Ala Val Asp Gln Ser Val Leu Leu Met Lys
           595                 600                 605
   Pro Asp Ala Glu Leu Ser Ala Ser Ser Val Tyr Asn Leu Leu Pro Glu
       610                 615                 620
   Lys Asp Leu Thr Gly Phe Pro Gly Pro Leu Asn Asp Gln Asp Asn Glu
   625                 630                 635                 640
   Asp Cys Ile Asn Arg His Asn Val Tyr Ile Asn Gly Ile Thr Tyr Thr
                   645                 650                 655
   Pro Val Ser Ser Thr Asn Glu Lys Asp Met Tyr Ser Phe Leu Glu Asp
               660                 665                 670
   Met Gly Leu Lys Ala Phe Thr Asn Ser Lys Ile Arg Lys Pro Lys Met
           675                 680                 685
   Cys Pro Gln Leu Gln Gln Tyr Glu Met His Gly Pro Glu Gly Leu Arg
       690                 695                 700
   Val Gly Phe Tyr Glu Ser Asp Val Met Gly Arg Gly His Ala Arg Leu
   705                 710                 715                 720
   Val His Val Glu Glu Pro His Thr Glu Thr Val Arg Lys Tyr Phe Pro
                   725                 730                 735
   Glu Thr Trp Ile Trp Asp Leu Val Val Val Asn Ser Ala Gly Val Ala
               740                 745                 750
   Glu Val Gly Val Thr Val Pro Asp Thr Ile Thr Glu Trp Lys Ala Gly
           755                 760                 765
   Ala Phe Cys Leu Ser Glu Asp Ala Gly Leu Gly Ile Ser Ser Thr Ala
       770                 775                 780
   Ser Leu Arg Ala Phe Gln Pro Phe Phe Val Glu Leu Thr Met Pro Tyr
   785                 790                 795                 800
   Ser Val Ile Arg Gly Glu Ala Phe Thr Leu Lys Ala Thr Val Leu Asn
                   805                 810                 815
   Tyr Leu Pro Lys Cys Ile Arg Val Ser Val Gln Leu Glu Ala Ser Pro
               820                 825                 830
   Ala Phe Leu Ala Val Pro Val Glu Lys Glu Gln Ala Pro His Cys Ile
           835                 840                 845
   Cys Ala Asn Gly Arg Gln Thr Val Ser Trp Ala Val Thr Pro Lys Ser
       850                 855                 860
   Leu Gly Asn Val Asn Phe Thr Val Ser Ala Glu Ala Leu Glu Ser Gln
   865                 870                 875                 880
   Glu Leu Cys Gly Thr Glu Val Pro Ser Val Pro Glu His Gly Arg Lys
   885 890 895
   Asp Thr Val Ile Lys Pro Leu Leu Val Glu Pro Glu Gly Leu Glu Lys
   900 905 910
   Glu Thr Thr Phe Asn Ser Leu Leu Cys Pro Ser Gly Gly Glu Val Ser
   915 920 925
   Glu Glu Leu Ser Leu Lys Leu Pro Pro Asn Val Val Glu Glu Ser Ala
   930 935 940
   Arg Ala Ser Val Ser Val Leu Gly Asp Ile Leu Gly Ser Ala Met Gln
   945 950 955 960
   Asn Thr Gln Asn Leu Leu Gln Met Pro Tyr Gly Cys Gly Glu Gln Asn
   965 970 975
   Met Val Leu Phe Ala Pro Asn Ile Tyr Val Leu Asp Tyr Leu Asn Glu
   980 985 990
   Thr Gln Gln Leu Thr Pro Glu Ile Lys Ser Lys Ala Ile Gly Tyr Leu
   995 1000 1005
   Asn Thr Gly Tyr Gln Arg Gln Leu Asn Tyr Lys His Tyr Asp Gly
   1010 1015 1020
   Ser Tyr Ser Thr Phe Gly Glu Arg Tyr Gly Arg Asn Gln Gly Asn
   1025 1030 1035
   Thr Trp Leu Thr Ala Phe Val Leu Lys Thr Phe Ala Gln Ala Arg
   1040 1045 1050
   Ala Tyr Ile Phe Ile Asp Glu Ala His Ile Thr Gln Ala Leu Ile
   1055 1060 1065
   Trp Leu Ser Gln Arg Gln Lys Asp Asn Gly Cys Phe Arg Ser Ser
   1070 1075 1080
   Gly Ser Leu Leu Asn Asn Ala Ile Lys Gly Gly Val Glu Asp Glu
   1085 1090 1095
   Val Thr Leu Ser Ala Tyr Ile Thr Ile Ala Leu Leu Glu Ile Pro
   1100 1105 1110
   Leu Thr Val Thr His Pro Val Val Arg Asn Ala Leu Phe Cys Leu
   1115 1120 1125
   Glu Ser  Ala Trp Lys Thr Ala  Gln Glu Gly Asp His  Gly Ser His
       1130                 1135                 1140
   Val Tyr  Thr Lys Ala Leu Leu  Ala Tyr Ala Phe Ala  Leu Ala Gly
       1145                 1150                 1155
   Asn Gln  Asp Lys Arg Lys Glu  Val Leu Lys Ser Leu  Asn Glu Glu
       1160                 1165                 1170
   Ala Val  Lys Lys Asp Asn Ser  Val His Trp Glu Arg  Pro Gln Lys
       1175                 1180                 1185
   Pro Lys  Ala Pro Val Gly His  Phe Tyr Glu Pro Gln  Ala Pro Ser
       1190                 1195                 1200
   Ala Glu  Val Glu Met Thr Ser  Tyr Val Leu Leu Ala  Tyr Leu Thr
       1205                 1210                 1215
   Ala Gln  Pro Ala Pro Thr Ser  Glu Asp Leu Thr Ser  Ala Thr Asn
       1220                 1225                 1230
   Ile Val  Lys Trp Ile Thr Lys  Gln Gln Asn Ala Gln  Gly Gly Phe
       1235                 1240                 1245
   Ser Ser  Thr Gln Asp Thr Val  Val Ala Leu His Ala  Leu Ser Lys
       1250                 1255                 1260
   Tyr Gly  Ala Ala Thr Phe Thr  Arg Thr Gly Lys Ala  Ala Gln Val
       1265                 1270                 1275
   Thr Ile  Gln Ser Ser Gly Thr  Phe Ser Ser Lys Phe  Gln Val Asp
       1280                 1285                 1290
   Asn Asn  Asn Arg Leu Leu Leu  Gln Gln Val Ser Leu  Pro Glu Leu
       1295                 1300                 1305
   Pro Gly  Glu Tyr Ser Met Lys  Val Thr Gly Glu Gly  Cys Val Tyr
       1310                 1315                 1320
   Leu Gln  Thr Ser Leu Lys Tyr  Asn Ile Leu Pro Glu  Lys Glu Glu
       1325                 1330                 1335
   Phe Pro  Phe Ala Leu Gly Val  Gln Thr Leu Pro Gln  Thr Cys Asp
       1340                 1345                 1350
   Glu Pro  Lys Ala His Thr Ser  Phe Gln Ile Ser Leu  Ser Val Ser
       1355                 1360                 1365
   Tyr Thr Gly Ser Arg Ser Ala Ser Asn Met Ala Ile Val Asp Val
   1370 1375 1380
   Lys Met Val Ser Gly Phe Ile Pro Leu Lys Pro Thr Val Lys Met
   1385 1390 1395
   Leu Glu Arg Ser Asn His Val Ser Arg Thr Glu Val Ser Ser Asn
   1400 1405 1410
   His Val Leu Ile Tyr Leu Asp Lys Val Ser Asn Gln Thr Leu Ser
   1415 1420 1425
   Leu Phe Phe Thr Val Leu Gln Asp Val Pro Val Arg Asp Leu Lys
   1430 1435 1440
   Pro Ala Ile Val Lys Val Tyr Asp Tyr Tyr Glu Thr Asp Glu Phe
   1445 1450 1455
   Ala Ile Ala Glu Tyr Asn Ala Pro Cys Ser Lys Asp Leu Gly Asn
   1460 1465 1470
   Ala
<210> 10
   <211> 1451
   <212> PRT
   <213> Homo sapiens
<400> 10
   Ser Val Ser Gly Lys Pro Gln Tyr Met Val Leu Val Pro Ser Leu Leu
   1               5                   10                  15
   His Thr Glu Thr Thr Glu Lys Gly Cys Val Leu Leu Ser Tyr Leu Asn
               20                  25                  30
   Glu Thr Val Thr Val Ser Ala Ser Leu Glu Ser Val Arg Gly Asn Arg
           35                  40                  45
   Ser Leu Phe Thr Asp Leu Glu Ala Glu Asn Asp Val Leu His Cys Val
       50                  55                  60
   Ala Phe Ala Val Pro Lys Ser Ser Ser Asn Glu Glu Val Met Phe Leu
   65                  70                  75                  80
   Thr Val Gln Val Lys Gly Pro Thr Gln Glu Phe Lys Lys Arg Thr Thr
                   85                  90                  95
   Val Met Val Lys Asn Glu Asp Ser Leu Val Phe Val Gln Thr Asp Lys
               100                 105                 110
   Ser Ile Tyr Lys Pro Gly Gln Thr Val Lys Phe Arg Val Val Ser Met
           115                 120                 125
   Asp Glu Asn Phe His Pro Leu Asn Glu Leu Ile Pro Leu Val Tyr Ile
       130                 135                 140
   Gln Asp Pro Lys Gly Asn Arg Ile Ala Gln Trp Gln Ser Phe Gln Leu
   145                 150                 155                 160
   Glu Gly Gly Leu Lys Gln Phe Ser Phe Pro Leu Ser Ser Glu Pro Phe
                   165                 170                 175
   Gln Gly Ser Tyr Lys Val Val Val Gln Lys Lys Ser Gly Gly Arg Thr
               180                 185                 190
   Glu His Pro Phe Thr Val Glu Glu Phe Val Leu Pro Lys Phe Glu Val
           195                 200                 205
   Gln Val Thr Val Pro Lys Ile Ile Thr Ile Leu Glu Glu Glu Met Asn
       210                 215                 220
   Val Ser Val Cys Gly Leu Tyr Thr Tyr Gly Lys Pro Val Pro Gly His
   225                 230                 235                 240
   Val Thr Val Ser Ile Cys Arg Lys Tyr Ser Asp Ala Ser Asp Cys His
                   245                 250                 255
   Gly Glu Asp Ser Gln Ala Phe Cys Glu Lys Phe Ser Gly Gln Leu Asn
               260                 265                 270
   Ser His Gly Cys Phe Tyr Gln Gln Val Lys Thr Lys Val Phe Gln Leu
           275                 280                 285
   Lys Arg Lys Glu Tyr Glu Met Lys Leu His Thr Glu Ala Gln Ile Gln
       290                 295                 300
   Glu Glu Gly Thr Val Val Glu Leu Thr Gly Arg Gln Ser Ser Glu Ile
   305                 310                 315                 320
   Thr Arg Thr Ile Thr Lys Leu Ser Phe Val Lys Val Asp Ser His Phe
                   325                 330                 335
   Arg Gln Gly Ile Pro Phe Phe Gly Gln Val Arg Leu Val Asp Gly Lys
               340                 345                 350
   Gly Val Pro Ile Pro Asn Lys Val Ile Phe Ile Arg Gly Asn Glu Ala
           355                 360                 365
   Asn Tyr Tyr Ser Asn Ala Thr Thr Asp Glu His Gly Leu Val Gln Phe
       370                 375                 380
   Ser Ile Asn Thr Thr Asn Val Met Gly Thr Ser Leu Thr Val Arg Val
   385                 390                 395                 400
   Asn Tyr Lys Asp Arg Ser Pro Cys Tyr Gly Tyr Gln Trp Val Ser Glu
                   405                 410                 415
   Glu His Glu Glu Ala His His Thr Ala Tyr Leu Val Phe Ser Pro Ser
               420                 425                 430
   Lys Ser Phe Val His Leu Glu Pro Met Ser His Glu Leu Pro Cys Gly
           435                 440                 445
   His Thr Gln Thr Val Gln Ala His Tyr Ile Leu Asn Gly Gly Thr Leu
       450                 455                 460
   Leu Gly Leu Lys Lys Leu Ser Phe Tyr Tyr Leu Ile Met Ala Lys Gly
   465                 470                 475                 480
   Gly Ile Val Arg Thr Gly Thr His Gly Leu Leu Val Lys Gln Glu Asp
                   485                 490                 495
   Met Lys Gly His Phe Ser Ile Ser Ile Pro Val Lys Ser Asp Ile Ala
               500                 505                 510
   Pro Val Ala Arg Leu Leu Ile Tyr Ala Val Leu Pro Thr Gly Asp Val
           515                 520                 525
   Ile Gly Asp Ser Ala Lys Tyr Asp Val Glu Asn Cys Leu Ala Asn Lys
       530                 535                 540
   Val Asp Leu Ser Phe Ser Pro Ser Gln Ser Leu Pro Ala Ser His Ala
   545                 550                 555                 560
   His Leu Arg Val Thr Ala Ala Pro Gln Ser Val Cys Ala Leu Arg Ala
                   565                 570                 575
   Val Asp Gln Ser Val Leu Leu Met Lys Pro Asp Ala Glu Leu Ser Ala
               580                 585                 590
   Ser Ser Val Tyr Asn Leu Leu Pro Glu Lys Asp Leu Thr Gly Phe Pro
           595                 600                 605
   Gly Pro Leu Asn Asp Gln Asp Asn Glu Asp Cys Ile Asn Arg His Asn
       610                 615                 620
   Val Tyr Ile Asn Gly Ile Thr Tyr Thr Pro Val Ser Ser Thr Asn Glu
   625 630 635 640
   Lys Asp Met Tyr Ser Phe Leu Glu Asp Met Gly Leu Lys Ala Phe Thr
   645 650 655
   Asn Ser Lys Ile Arg Lys Pro Lys Met Cys Pro Gln Leu Gln Gln Tyr
   660 665 670
   Glu Met His Gly Pro Glu Gly Leu Arg Val Gly Phe Tyr Glu Ser Asp
   675 680 685
   Val Met Gly Arg Gly His Ala Arg Leu Val His Val Glu Glu Pro His
   690 695 700
   Thr Glu Thr Val Arg Lys Tyr Phe Pro Glu Thr Trp Ile Trp Asp Leu
   705 710 715 720
   Val Val Val Asn Ser Ala Gly Val Ala Glu Val Gly Val Thr Val Pro
   725 730 735
   Asp Thr Ile Thr Glu Trp Lys Ala Gly Ala Phe Cys Leu Ser Glu Asp
   740 745 750
   Ala Gly Leu Gly Ile Ser Ser Thr Ala Ser Leu Arg Ala Phe Gln Pro
   755 760 765
   Phe Phe Val Glu Leu Thr Met Pro Tyr Ser Val Ile Arg Gly Glu Ala
   770 775 780
   Phe Thr Leu Lys Ala Thr Val Leu Asn Tyr Leu Pro Lys Cys Ile Arg
   785 790 795 800
   Val Ser Val Gln Leu Glu Ala Ser Pro Ala Phe Leu Ala Val Pro Val
   805 810 815
   Glu Lys Glu Gln Ala Pro His Cys Ile Cys Ala Asn Gly Arg Gln Thr
   820 825 830
   Val Ser Trp Ala Val Thr Pro Lys Ser Leu Gly Asn Val Asn Phe Thr
   835 840 845
   Val Ser Ala Glu Ala Leu Glu Ser Gln Glu Leu Cys Gly Thr Glu Val
   850 855 860
   Pro Ser Val Pro Glu His Gly Arg Lys Asp Thr Val Ile Lys Pro Leu
   865 870 875 880
   Leu Val Glu Pro Glu Gly Leu Glu Lys Glu Thr Thr Phe Asn Ser Leu
   885 890 895
   Leu Cys Pro Ser Gly Gly Glu Val Ser Glu Glu Leu Ser Leu Lys Leu
   900 905 910
   Pro Pro Asn Val Val Glu Glu Ser Ala Arg Ala Ser Val Ser Val Leu
   915 920 925
   Gly Asp Ile Leu Gly Ser Ala Met Gln Asn Thr Gln Asn Leu Leu Gln
   930 935 940
   Met Pro Tyr Gly Cys Gly Glu Gln Asn Met Val Leu Phe Ala Pro Asn
   945 950 955 960
   Ile Tyr Val Leu Asp Tyr Leu Asn Glu Thr Gln Gln Leu Thr Pro Glu
   965 970 975
   Ile Lys Ser Lys Ala Ile Gly Tyr Leu Asn Thr Gly Tyr Gln Arg Gln
   980 985 990
   Leu Asn Tyr Lys His Tyr Asp Gly Ser Tyr Ser Thr Phe Gly Glu Arg
   995 1000 1005
   Tyr Gly Arg Asn Gln Gly Asn Thr Trp Leu Thr Ala Phe Val Leu
   1010 1015 1020
   Lys Thr Phe Ala Gln Ala Arg Ala Tyr Ile Phe Ile Asp Glu Ala
   1025 1030 1035
   His Ile Thr Gln Ala Leu Ile Trp Leu Ser Gln Arg Gln Lys Asp
   1040 1045 1050
   Asn Gly Cys Phe Arg Ser Ser Gly Ser Leu Leu Asn Asn Ala Ile
   1055 1060 1065
   Lys Gly Gly Val Glu Asp Glu Val Thr Leu Ser Ala Tyr Ile Thr
   1070 1075 1080
   Ile Ala Leu Leu Glu Ile Pro Leu Thr Val Thr His Pro Val Val
   1085 1090 1095
   Arg Asn Ala Leu Phe Cys Leu Glu Ser Ala Trp Lys Thr Ala Gln
   1100 1105 1110
   Glu Gly Asp His Gly Ser His Val Tyr Thr Lys Ala Leu Leu Ala
   1115 1120 1125
   Tyr Ala Phe Ala Leu Ala Gly Asn Gln Asp Lys Arg Lys Glu Val
   1130 1135 1140
   Leu Lys  Ser Leu Asn Glu Glu  Ala Val Lys Lys Asp  Asn Ser Val
       1145                 1150                 1155
   His Trp  Glu Arg Pro Gln Lys  Pro Lys Ala Pro Val  Gly His Phe
       1160                 1165                 1170
   Tyr Glu  Pro Gln Ala Pro Ser  Ala Glu Val Glu Met  Thr Ser Tyr
       1175                 1180                 1185
   Val Leu  Leu Ala Tyr Leu Thr  Ala Gln Pro Ala Pro  Thr Ser Glu
       1190                 1195                 1200
   Asp Leu  Thr Ser Ala Thr Asn  Ile Val Lys Trp Ile  Thr Lys Gln
       1205                 1210                 1215
   Gln Asn  Ala Gln Gly Gly Phe  Ser Ser Thr Gln Asp  Thr Val Val
       1220                 1225                 1230
   Ala Leu  His Ala Leu Ser Lys  Tyr Gly Ala Ala Thr  Phe Thr Arg
       1235                 1240                 1245
   Thr Gly  Lys Ala Ala Gln Val  Thr Ile Gln Ser Ser  Gly Thr Phe
       1250                 1255                 1260
   Ser Ser  Lys Phe Gln Val Asp  Asn Asn Asn Arg Leu  Leu Leu Gln
       1265                 1270                 1275
   Gln Val  Ser Leu Pro Glu Leu  Pro Gly Glu Tyr Ser  Met Lys Val
       1280                 1285                 1290
   Thr Gly  Glu Gly Cys Val Tyr  Leu Gln Thr Ser Leu  Lys Tyr Asn
       1295                 1300                 1305
   Ile Leu  Pro Glu Lys Glu Glu  Phe Pro Phe Ala Leu  Gly Val Gln
       1310                 1315                 1320
   Thr Leu  Pro Gln Thr Cys Asp  Glu Pro Lys Ala His  Thr Ser Phe
       1325                 1330                 1335
   Gln Ile  Ser Leu Ser Val Ser  Tyr Thr Gly Ser Arg  Ser Ala Ser
       1340                 1345                 1350
   Asn Met  Ala Ile Val Asp Val  Lys Met Val Ser Gly  Phe Ile Pro
       1355                 1360                 1365
   Leu Lys  Pro Thr Val Lys Met  Leu Glu Arg Ser Asn  His Val Ser
       1370                 1375                 1380
   Arg Thr Glu Val Ser Ser Asn His Val Leu Ile Tyr Leu Asp Lys
   1385 1390 1395
   Val Ser Asn Gln Thr Leu Ser Leu Phe Phe Thr Val Leu Gln Asp
   1400 1405 1410
   Val Pro Val Arg Asp Leu Lys Pro Ala Ile Val Lys Val Tyr Asp
   1415 1420 1425
   Tyr Tyr Glu Thr Asp Glu Phe Ala Ile Ala Glu Tyr Asn Ala Pro
   1430 1435 1440
   Cys Ser Lys Asp Leu Gly Asn Ala
   1445 1450
<210> 11
   <211> 814
   <212> PRT
   <213> Homo sapiens
<400> 11
   Thr Asn Glu Lys Asp Met Tyr Ser Phe Leu Glu Asp Met Gly Leu Lys
   1               5                   10                  15
   Ala Phe Thr Asn Ser Lys Ile Arg Lys Pro Lys Met Cys Pro Gln Leu
               20                  25                  30
   Gln Gln Tyr Glu Met His Gly Pro Glu Gly Leu Arg Val Gly Phe Tyr
           35                  40                  45
   Glu Ser Asp Val Met Gly Arg Gly His Ala Arg Leu Val His Val Glu
       50                  55                  60
   Glu Pro His Thr Glu Thr Val Arg Lys Tyr Phe Pro Glu Thr Trp Ile
   65                  70                  75                  80
   Trp Asp Leu Val Val Val Asn Ser Ala Gly Val Ala Glu Val Gly Val
                   85                  90                  95
   Thr Val Pro Asp Thr Ile Thr Glu Trp Lys Ala Gly Ala Phe Cys Leu
               100                 105                 110
   Ser Glu Asp Ala Gly Leu Gly Ile Ser Ser Thr Ala Ser Leu Arg Ala
           115                 120                 125
   Phe Gln Pro Phe Phe Val Glu Leu Thr Met Pro Tyr Ser Val Ile Arg
       130                 135                 140
   Gly Glu Ala Phe Thr Leu Lys Ala Thr Val Leu Asn Tyr Leu Pro Lys
   145                 150                 155                 160
   Cys Ile Arg Val Ser Val Gln Leu Glu Ala Ser Pro Ala Phe Leu Ala
                   165                 170                 175
   Val Pro Val Glu Lys Glu Gln Ala Pro His Cys Ile Cys Ala Asn Gly
               180                 185                 190
   Arg Gln Thr Val Ser Trp Ala Val Thr Pro Lys Ser Leu Gly Asn Val
           195                 200                 205
   Asn Phe Thr Val Ser Ala Glu Ala Leu Glu Ser Gln Glu Leu Cys Gly
       210                 215                 220
   Thr Glu Val Pro Ser Val Pro Glu His Gly Arg Lys Asp Thr Val Ile
   225                 230                 235                 240
   Lys Pro Leu Leu Val Glu Pro Glu Gly Leu Glu Lys Glu Thr Thr Phe
                   245                 250                 255
   Asn Ser Leu Leu Cys Pro Ser Gly Gly Glu Val Ser Glu Glu Leu Ser
               260                 265                 270
   Leu Lys Leu Pro Pro Asn Val Val Glu Glu Ser Ala Arg Ala Ser Val
           275                 280                 285
   Ser Val Leu Gly Asp Ile Leu Gly Ser Ala Met Gln Asn Thr Gln Asn
       290                 295                 300
   Leu Leu Gln Met Pro Tyr Gly Cys Gly Glu Gln Asn Met Val Leu Phe
   305                 310                 315                 320
   Ala Pro Asn Ile Tyr Val Leu Asp Tyr Leu Asn Glu Thr Gln Gln Leu
                   325                 330                 335
   Thr Pro Glu Ile Lys Ser Lys Ala Ile Gly Tyr Leu Asn Thr Gly Tyr
               340                 345                 350
   Gln Arg Gln Leu Asn Tyr Lys His Tyr Asp Gly Ser Tyr Ser Thr Phe
           355                 360                 365
   Gly Glu Arg Tyr Gly Arg Asn Gln Gly Asn Thr Trp Leu Thr Ala Phe
       370                 375                 380
   Val Leu Lys Thr Phe Ala Gln Ala Arg Ala Tyr Ile Phe Ile Asp Glu
   385                 390                 395                 400
   Ala His Ile Thr Gln Ala Leu Ile Trp Leu Ser Gln Arg Gln Lys Asp
                   405                 410                 415
   Asn Gly Cys Phe Arg Ser Ser Gly Ser Leu Leu Asn Asn Ala Ile Lys
   420 425 430
   Gly Gly Val Glu Asp Glu Val Thr Leu Ser Ala Tyr Ile Thr Ile Ala
   435 440 445
   Leu Leu Glu Ile Pro Leu Thr Val Thr His Pro Val Val Arg Asn Ala
   450 455 460
   Leu Phe Cys Leu Glu Ser Ala Trp Lys Thr Ala Gln Glu Gly Asp His
   465 470 475 480
   Gly Ser His Val Tyr Thr Lys Ala Leu Leu Ala Tyr Ala Phe Ala Leu
   485 490 495
   Ala Gly Asn Gln Asp Lys Arg Lys Glu Val Leu Lys Ser Leu Asn Glu
   500 505 510
   Glu Ala Val Lys Lys Asp Asn Ser Val His Trp Glu Arg Pro Gln Lys
   515 520 525
   Pro Lys Ala Pro Val Gly His Phe Tyr Glu Pro Gln Ala Pro Ser Ala
   530 535 540
   Glu Val Glu Met Thr Ser Tyr Val Leu Leu Ala Tyr Leu Thr Ala Gln
   545 550 555 560
   Pro Ala Pro Thr Ser Glu Asp Leu Thr Ser Ala Thr Asn Ile Val Lys
   565 570 575
   Trp Ile Thr Lys Gln Gln Asn Ala Gln Gly Gly Phe Ser Ser Thr Gln
   580 585 590
   Asp Thr Val Val Ala Leu His Ala Leu Ser Lys Tyr Gly Ala Ala Thr
   595 600 605
   Phe Thr Arg Thr Gly Lys Ala Ala Gln Val Thr Ile Gln Ser Ser Gly
   610 615 620
   Thr Phe Ser Ser Lys Phe Gln Val Asp Asn Asn Asn Arg Leu Leu Leu
   625 630 635 640
   Gln Gln Val Ser Leu Pro Glu Leu Pro Gly Glu Tyr Ser Met Lys Val
   645 650 655
   Thr Gly Glu Gly Cys Val Tyr Leu Gln Thr Ser Leu Lys Tyr Asn Ile
   660 665 670
   Leu Pro Glu Lys Glu Glu Phe Pro Phe Ala Leu Gly Val Gln Thr Leu
   675 680 685
   Pro Gln Thr Cys Asp Glu Pro Lys Ala His Thr Ser Phe Gln Ile Ser
   690 695 700
   Leu Ser Val Ser Tyr Thr Gly Ser Arg Ser Ala Ser Asn Met Ala Ile
   705 710 715 720
   Val Asp Val Lys Met Val Ser Gly Phe Ile Pro Leu Lys Pro Thr Val
   725 730 735
   Lys Met Leu Glu Arg Ser Asn His Val Ser Arg Thr Glu Val Ser Ser
   740 745 750
   Asn His Val Leu Ile Tyr Leu Asp Lys Val Ser Asn Gln Thr Leu Ser
   755 760 765
   Leu Phe Phe Thr Val Leu Gln Asp Val Pro Val Arg Asp Leu Lys Pro
   770 775 780
   Ala Ile Val Lys Val Tyr Asp Tyr Tyr Glu Thr Asp Glu Phe Ala Ile
   785 790 795 800
   Ala Glu Tyr Asn Ala Pro Cys Ser Lys Asp Leu Gly Asn Ala
   805 810
<210> 12
   <211> 377
   <212> PRT
   <213> Homo sapiens
<400> 12
   Glu Val Thr Leu Ser Ala Tyr Ile Thr Ile Ala Leu Leu Glu Ile Pro
   1               5                   10                  15
   Leu Thr Val Thr His Pro Val Val Arg Asn Ala Leu Phe Cys Leu Glu
               20                  25                  30
   Ser Ala Trp Lys Thr Ala Gln Glu Gly Asp His Gly Ser His Val Tyr
           35                  40                  45
   Thr Lys Ala Leu Leu Ala Tyr Ala Phe Ala Leu Ala Gly Asn Gln Asp
       50                  55                  60
   Lys Arg Lys Glu Val Leu Lys Ser Leu Asn Glu Glu Ala Val Lys Lys
   65                  70                  75                  80
   Asp Asn Ser Val His Trp Glu Arg Pro Gln Lys Pro Lys Ala Pro Val
                   85                  90                  95
   Gly His Phe Tyr Glu Pro Gln Ala Pro Ser Ala Glu Val Glu Met Thr
               100                 105                 110
   Ser Tyr Val Leu Leu Ala Tyr Leu Thr Ala Gln Pro Ala Pro Thr Ser
           115                 120                 125
   Glu Asp Leu Thr Ser Ala Thr Asn Ile Val Lys Trp Ile Thr Lys Gln
       130                 135                 140
   Gln Asn Ala Gln Gly Gly Phe Ser Ser Thr Gln Asp Thr Val Val Ala
   145                 150                 155                 160
   Leu His Ala Leu Ser Lys Tyr Gly Ala Ala Thr Phe Thr Arg Thr Gly
                   165                 170                 175
   Lys Ala Ala Gln Val Thr Ile Gln Ser Ser Gly Thr Phe Ser Ser Lys
               180                 185                 190
   Phe Gln Val Asp Asn Asn Asn Arg Leu Leu Leu Gln Gln Val Ser Leu
           195                 200                 205
   Pro Glu Leu Pro Gly Glu Tyr Ser Met Lys Val Thr Gly Glu Gly Cys
       210                 215                 220
   Val Tyr Leu Gln Thr Ser Leu Lys Tyr Asn Ile Leu Pro Glu Lys Glu
   225                 230                 235                 240
   Glu Phe Pro Phe Ala Leu Gly Val Gln Thr Leu Pro Gln Thr Cys Asp
                   245                 250                 255
   Glu Pro Lys Ala His Thr Ser Phe Gln Ile Ser Leu Ser Val Ser Tyr
               260                 265                 270
   Thr Gly Ser Arg Ser Ala Ser Asn Met Ala Ile Val Asp Val Lys Met
           275                 280                 285
   Val Ser Gly Phe Ile Pro Leu Lys Pro Thr Val Lys Met Leu Glu Arg
       290                 295                 300
   Ser Asn His Val Ser Arg Thr Glu Val Ser Ser Asn His Val Leu Ile
   305                 310                 315                 320
   Tyr Leu Asp Lys Val Ser Asn Gln Thr Leu Ser Leu Phe Phe Thr Val
                   325                 330                 335
   Leu Gln Asp Val Pro Val Arg Asp Leu Lys Pro Ala Ile Val Lys Val
               340                 345                 350
   Tyr Asp Tyr Tyr Glu Thr Asp Glu Phe Ala Ile Ala Glu Tyr Asn Ala
   355 360 365
   Pro Cys Ser Lys Asp Leu Gly Asn Ala
   370 375
<210> 13
   <211> 390
   <212> PRT
   <213> Homo sapiens
<400> 13
   Ser Leu Leu Asn Asn Ala Ile Lys Gly Gly Val Glu Asp Glu Val Thr
   1 5 10 15
   Leu Ser Ala Tyr Ile Thr Ile Ala Leu Leu Glu Ile Pro Leu Thr Val
   20 25 30
   Thr His Pro Val Val Arg Asn Ala Leu Phe Cys Leu Glu Ser Ala Trp
   35 40 45
   Lys Thr Ala Gln Glu Gly Asp His Gly Ser His Val Tyr Thr Lys Ala
   50 55 60
   Leu Leu Ala Tyr Ala Phe Ala Leu Ala Gly Asn Gln Asp Lys Arg Lys
   65 70 75 80
   Glu Val Leu Lys Ser Leu Asn Glu Glu Ala Val Lys Lys Asp Asn Ser
   85 90 95
   Val His Trp Glu Arg Pro Gln Lys Pro Lys Ala Pro Val Gly His Phe
   100 105 110
   Tyr Glu Pro Gln Ala Pro Ser Ala Glu Val Glu Met Thr Ser Tyr Val
   115 120 125
   Leu Leu Ala Tyr Leu Thr Ala Gln Pro Ala Pro Thr Ser Glu Asp Leu
   130 135 140
   Thr Ser Ala Thr Asn Ile Val Lys Trp Ile Thr Lys Gln Gln Asn Ala
   145 150 155 160
   Gln Gly Gly Phe Ser Ser Thr Gln Asp Thr Val Val Ala Leu His Ala
   165 170 175
   Leu Ser Lys Tyr Gly Ala Ala Thr Phe Thr Arg Thr Gly Lys Ala Ala
   180 185 190
   Gln Val Thr Ile Gln Ser Ser Gly Thr Phe Ser Ser Lys Phe Gln Val
           195                 200                 205
   Asp Asn Asn Asn Arg Leu Leu Leu Gln Gln Val Ser Leu Pro Glu Leu
       210                 215                 220
   Pro Gly Glu Tyr Ser Met Lys Val Thr Gly Glu Gly Cys Val Tyr Leu
   225                 230                 235                 240
   Gln Thr Ser Leu Lys Tyr Asn Ile Leu Pro Glu Lys Glu Glu Phe Pro
                   245                 250                 255
   Phe Ala Leu Gly Val Gln Thr Leu Pro Gln Thr Cys Asp Glu Pro Lys
               260                 265                 270
   Ala His Thr Ser Phe Gln Ile Ser Leu Ser Val Ser Tyr Thr Gly Ser
           275                 280                 285
   Arg Ser Ala Ser Asn Met Ala Ile Val Asp Val Lys Met Val Ser Gly
       290                 295                 300
   Phe Ile Pro Leu Lys Pro Thr Val Lys Met Leu Glu Arg Ser Asn His
   305                 310                 315                 320
   Val Ser Arg Thr Glu Val Ser Ser Asn His Val Leu Ile Tyr Leu Asp
                   325                 330                 335
   Lys Val Ser Asn Gln Thr Leu Ser Leu Phe Phe Thr Val Leu Gln Asp
               340                 345                 350
   Val Pro Val Arg Asp Leu Lys Pro Ala Ile Val Lys Val Tyr Asp Tyr
           355                 360                 365
   Tyr Glu Thr Asp Glu Phe Ala Ile Ala Glu Tyr Asn Ala Pro Cys Ser
       370                 375                 380
   Lys Asp Leu Gly Asn Ala
   385                 390
<210> 14
   <211> 391
   <212> PRT
   <213> Homo sapiens
<400> 14
   Gly Ser Leu Leu Asn Asn Ala Ile Lys Gly Gly Val Glu Asp Glu Val
   1               5                   10                  15
   Thr Leu Ser Ala Tyr Ile Thr Ile Ala Leu Leu Glu Ile Pro Leu Thr
               20                  25                  30
   Val Thr His Pro Val Val Arg Asn Ala Leu Phe Cys Leu Glu Ser Ala
           35                  40                  45
   Trp Lys Thr Ala Gln Glu Gly Asp His Gly Ser His Val Tyr Thr Lys
       50                  55                  60
   Ala Leu Leu Ala Tyr Ala Phe Ala Leu Ala Gly Asn Gln Asp Lys Arg
   65                  70                  75                      80
   Lys Glu Val Leu Lys Ser Leu Asn Glu Glu Ala Val Lys Lys Asp Asn
                   85                  90                      95
   Ser Val His Trp Glu Arg Pro Gln Lys Pro Lys Ala Pro Val Gly His
               100                 105                 110
   Phe Tyr Glu Pro Gln Ala Pro Ser Ala Glu Val Glu Met Thr Ser Tyr
           115                 120                 125
   Val Leu Leu Ala Tyr Leu Thr Ala Gln Pro Ala Pro Thr Ser Glu Asp
       130                 135                 140
   Leu Thr Ser Ala Thr Asn Ile Val Lys Trp Ile Thr Lys Gln Gln Asn
   145                 150                 155                     160
   Ala Gln Gly Gly Phe Ser Ser Thr Gln Asp Thr Val Val Ala Leu His
                   165                 170                     175
   Ala Leu Ser Lys Tyr Gly Ala Ala Thr Phe Thr Arg Thr Gly Lys Ala
               180                 185                 190
   Ala Gln Val Thr Ile Gln Ser Ser Gly Thr Phe Ser Ser Lys Phe Gln
           195                 200                 205
   Val Asp Asn Asn Asn Arg Leu Leu Leu Gln Gln Val Ser Leu Pro Glu
       210                 215                 220
   Leu Pro Gly Glu Tyr Ser Met Lys Val Thr Gly Glu Gly Cys Val Tyr
   225                 230                 235                     240
   Leu Gln Thr Ser Leu Lys Tyr Asn Ile Leu Pro Glu Lys Glu Glu Phe
                   245                 250                     255
   Pro Phe Ala Leu Gly Val Gln Thr Leu Pro Gln Thr Cys Asp Glu Pro
               260                 265                 270
   Lys Ala His Thr Ser Phe Gln Ile Ser Leu Ser Val Ser Tyr Thr Gly
           275                 280                 285
   Ser Arg Ser Ala Ser Asn Met Ala Ile Val Asp Val Lys Met Val Ser
       290                 295                 300
   Gly Phe Ile Pro Leu Lys Pro Thr Val Lys Met Leu Glu Arg Ser Asn
   305                 310                 315                 320
   His Val Ser Arg Thr Glu Val Ser Ser Asn His Val Leu Ile Tyr Leu
                   325                 330                 335
   Asp Lys Val Ser Asn Gln Thr Leu Ser Leu Phe Phe Thr Val Leu Gln
               340                 345                 350
   Asp Val Pro Val Arg Asp Leu Lys Pro Ala Ile Val Lys Val Tyr Asp
           355                 360                 365
   Tyr Tyr Glu Thr Asp Glu Phe Ala Ile Ala Glu Tyr Asn Ala Pro Cys
       370                 375                 380
   Ser Lys Asp Leu Gly Asn Ala
   385                 390
<210> 15
   <211> 392
   <212> PRT
   <213> Homo sapiens
<400> 15
   Ser Gly Ser Leu Leu Asn Asn Ala Ile Lys Gly Gly Val Glu Asp Glu
   1               5                   10                  15
   Val Thr Leu Ser Ala Tyr Ile Thr Ile Ala Leu Leu Glu Ile Pro Leu
               20                  25                  30
   Thr Val Thr His Pro Val Val Arg Asn Ala Leu Phe Cys Leu Glu Ser
           35                  40                  45
   Ala Trp Lys Thr Ala Gln Glu Gly Asp His Gly Ser His Val Tyr Thr
       50                  55                  60
   Lys Ala Leu Leu Ala Tyr Ala Phe Ala Leu Ala Gly Asn Gln Asp Lys
   65                  70                  75                  80
   Arg Lys Glu Val Leu Lys Ser Leu Asn Glu Glu Ala Val Lys Lys Asp
                   85                  90                  95
   Asn Ser Val His Trp Glu Arg Pro Gln Lys Pro Lys Ala Pro Val Gly
               100                 105                 110
   His Phe Tyr Glu Pro Gln Ala Pro Ser Ala Glu Val Glu Met Thr Ser
   115 120 125
   Tyr Val Leu Leu Ala Tyr Leu Thr Ala Gln Pro Ala Pro Thr Ser Glu
   130 135 140
   Asp Leu Thr Ser Ala Thr Asn Ile Val Lys Trp Ile Thr Lys Gln Gln
   145 150 155 160
   Asn Ala Gln Gly Gly Phe Ser Ser Thr Gln Asp Thr Val Val Ala Leu
   165 170 175
   His Ala Leu Ser Lys Tyr Gly Ala Ala Thr Phe Thr Arg Thr Gly Lys
   180 185 190
   Ala Ala Gln Val Thr Ile Gln Ser Ser Gly Thr Phe Ser Ser Lys Phe
   195 200 205
   Gln Val Asp Asn Asn Asn Arg Leu Leu Leu Gln Gln Val Ser Leu Pro
   210 215 220
   Glu Leu Pro Gly Glu Tyr Ser Met Lys Val Thr Gly Glu Gly Cys Val
   225 230 235 240
   Tyr Leu Gln Thr Ser Leu Lys Tyr Asn Ile Leu Pro Glu Lys Glu Glu
   245 250 255
   Phe Pro Phe Ala Leu Gly Val Gln Thr Leu Pro Gln Thr Cys Asp Glu
   260 265 270
   Pro Lys Ala His Thr Ser Phe Gln Ile Ser Leu Ser Val Ser Tyr Thr
   275 280 285
   Gly Ser Arg Ser Ala Ser Asn Met Ala Ile Val Asp Val Lys Met Val
   290 295 300
   Ser Gly Phe Ile Pro Leu Lys Pro Thr Val Lys Met Leu Glu Arg Ser
   305 310 315 320
   Asn His Val Ser Arg Thr Glu Val Ser Ser Asn His Val Leu Ile Tyr
   325 330 335
   Leu Asp Lys Val Ser Asn Gln Thr Leu Ser Leu Phe Phe Thr Val Leu
   340 345 350
   Gln Asp Val Pro Val Arg Asp Leu Lys Pro Ala Ile Val Lys Val Tyr
   355 360 365
   Asp Tyr Tyr Glu Thr Asp Glu Phe Ala Ile Ala Glu Tyr Asn Ala Pro
   370 375 380
   Cys Ser Lys Asp Leu Gly Asn Ala
   385 390
<210> 16
   <211> 10
   <212> PRT
   <213> Artificial
<220>
   <223> N-terminal sequencing
<220>
   <221> MISC_FEATURE
   <222> (9)..(9)
   <223> X = any amino acid
<400> 16
   Ser Leu Leu Asn Asn Ala Ile Lys Xaa Gly
   1 5 10
<210> 17
   <211> 7
   <212> PRT
   <213> Homo sapiens
<400> 17
   Glu Val Thr Leu Ser Ala Tyr
   1 5
<210> 18
   <211> 13
   <212> PRT
   <213> Artificial
<220>
   <223> epitope mapping control
<400> 18
   Ser Ser Gly Ser Leu Leu Asn Asn Ala Ile Lys Gly Gly
   1 5 10
<210> 19
   <211> 12
   <212> PRT
   <213> Artificial
<220>
   <223> epitope mapping control
<400> 19
   Ser Gly Ser Leu Leu Asn Asn Ala Ile Lys Gly Gly
   1 5 10
<210> 20
   <211> 11
   <212> PRT
   <213> Artificial
<220>
   <223> epitope mapping control
<400> 20
   Gly Ser Leu Leu Asn Asn Ala Ile Lys Gly Gly
   1 5 10
<210> 21
   <211> 10
   <212> PRT
   <213> Homo sapiens
<400> 21
   Ser Leu Leu Asn Asn Ala Ile Lys Gly Gly
   1 5 10
<210> 22
   <211> 9
   <212> PRT
   <213> Artificial
<220>
   <223> epitope mapping control
<400> 22
   Leu Leu Asn Asn Ala Ile Lys Gly Gly
   1 5
<210> 23
   <211> 10
   <212> PRT
   <213> Artificial
<220>
   <223> epitope mapping control
<400> 23
   Ala Leu Leu Asn Asn Ala Ile Lys Gly Gly
   1 5 10
<210> 24
   <211> 10
   <212> PRT
   <213> Artificial
<220>
   <223> epitope mapping control
<400> 24
   Ser Ala Leu Asn Asn Ala Ile Lys Gly Gly
   1               5                   10
<210> 25
   <211> 10
   <212> PRT
   <213> Artificial
<220>
   <223> epitope mapping control
<400> 25
   Ser Leu Ala Asn Asn Ala Ile Lys Gly Gly
   1               5                   10
<210> 26
   <211> 10
   <212> PRT
   <213> Artificial
<220>
   <223> epitope mapping control
<400> 26
   Ser Leu Leu Ala Asn Ala Ile Lys Gly Gly
   1               5                   10
<210> 27
   <211> 12
   <212> PRT
   <213> Artificial
<220>
   <223> epitope mapping control
<400> 27
   Glu Asp Glu Val Thr Leu Ser Ala Tyr Ile Thr Ile
   1               5                   10
<210> 28
   <211> 11
   <212> PRT
   <213> Artificial
<220>
   <223> epitope mapping control
<400> 28
   Asp Glu Val Thr Leu Ser Ala Tyr Ile Thr Ile
   1 5 10
<210> 29
   <211> 10
   <212> PRT
   <213> Homo sapiens
<400> 29
   Glu Val Thr Leu Ser Ala Tyr Ile Thr Ile
   1 5 10
<210> 30
   <211> 9
   <212> PRT
   <213> Artificial
<220>
   <223> epitope mapping control
<400> 30
   Val Thr Leu Ser Ala Tyr Ile Thr Ile
   1 5
<210> 31
   <211> 10
   <212> PRT
   <213> Artificial
<220>
   <223> epitope mapping control
<400> 31
   Ala Val Thr Leu Ser Ala Tyr Ile Thr Ile
   1 5 10
<210> 32
   <211> 10
   <212> PRT
   <213> Artificial
<220>
   <223> epitope mapping control
<400> 32
   Glu Ala Thr Leu Ser Ala Tyr Ile Thr Ile
   1 5 10
<210> 33
   <211> 10
   <212> PRT
   <213> Artificial
<220>
   <223> epitope mapping control
<400> 33
   Glu Val Ala Leu Ser Ala Tyr Ile Thr Ile
   1 5 10
<210> 34
   <211> 10
   <212> PRT
   <213> Artificial
<220>
   <223> epitope mapping control
<400> 34
   Glu Val Thr Ala Ser Ala Tyr Ile Thr Ile
   1 5 10
<210> 35
   <211> 10
   <212> PRT
   <213> Artificial
<220>
   <223> epitope mapping control
<400> 35
   Glu Val Thr Leu Ala Ala Tyr Ile Thr Ile
   1 5 10